(19) Europäisches Patentamt
European Patent Office
Office européen des brevets

(11) EP 4 446 435 B1

(12) EUROPEAN PATENT SPECIFICATION

(45) Date of publication and mention of the grant of the patent:
**29.07.2026 Bulletin 2026/31**

(21) Application number: **22904131.4**

(22) Date of filing: **01.12.2022**

(51) International Patent Classification (IPC):
***C12Q 1/6858*** *(2018.01)* ***C12Q 1/686*** *(2018.01)*
***C08F 220/06*** *(2006.01)* ***C08F 222/06*** *(2006.01)*
***C08F 222/38*** *(2006.01)* ***C08F 226/04*** *(2006.01)*
***C12Q 1/6827*** *(2018.01)* ***C08F 226/02*** *(2006.01)*

(52) Cooperative Patent Classification (CPC):
(C-Sets available)
**C12Q 1/6827; C08F 220/06; C08F 226/02; C08F 226/04** (Cont.)

(86) International application number:
**PCT/JP2022/044363**

(87) International publication number:
**WO 2023/106200 (15.06.2023 Gazette 2023/24)**

# (54) METHOD FOR DETECTING MUTATIONS IN TARGET BASE SEQUENCE OF NUCLEIC ACID, METHOD FOR SELECTIVELY INHIBITING AMPLIFICATION OF NUCLEIC ACID, AND KIT FOR IMPLEMENTING SAME

VERFAHREN ZUM NACHWEIS VON MUTATIONEN IN DER ZIELBASENSEQUENZ VON NUKLEINSÄURE, VERFAHREN ZUR SELEKTIVEN HEMMUNG DER AMPLIFIKATION VON NUKLEINSÄURE UND KIT ZUR IMPLEMENTIERUNG DAVON

PROCÉDÉ DESTINÉ À DÉTECTER LES MUTATIONS DANS UNE SÉQUENCE DE BASES OBJET DANS UN ACIDE NUCLÉIQUE, PROCÉDÉ D'INHIBITION SÉLECTIVE D'AMPLIFICATION D'ACIDE NUCLÉIQUE, ET KIT DESTINÉ À L'EXÉCUTION DE CES PROCÉDÉS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **10.12.2021 JP 2021201177**

(43) Date of publication of application:
**16.10.2024 Bulletin 2024/42**

(73) Proprietor: **Nitto Boseki Co., Ltd.**
**Fukushima-shi,**
**Fukushima 960-8161 (JP)**

(72) Inventors:
- **FUJIMURA, Nahohiro**
  **Kawasaki-shi, Kanagawa 210-0821 (JP)**
- **TACHIBANA, Ami**
  **Kawasaki-shi, Kanagawa 210-0821 (JP)**
- **UCHIKI, Tomoaki**
  **Kawasaki-shi, Kanagawa 210-0821 (JP)**
- **TAKEUCHI, Minoru**
  **Koriyama-shi, Fukushima 963-8061 (JP)**
- **TERUUCHI, Yoko**
  **Koriyama-shi, Fukushima 963-8061 (JP)**
- **WATANABE, Koji**
  **Koriyama-shi, Fukushima 963-8061 (JP)**

(74) Representative: **Hoffmann Eitle**
**Patent- und Rechtsanwälte PartmbB**
**Arabellastraße 30**
**81925 München (DE)**

(56) References cited:
**WO-A1-03/018841** **WO-A1-2012/033190**
**WO-A1-2014/051076** **WO-A1-2016/167320**
**WO-A1-2023/106189** **JP-A- 2004 537 263**
**JP-A- 2018 104 850** **JP-A- 2018 104 850**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **WANG BAOZHEN ET AL: "Use of Amphoteric Copolymer Films as Sacrificial Layers for Constructing Free-Standing Layer-by-Layer Films", MATER, vol. 6, no. 6, 6 June 2013 (2013-06-06), pages 2351 - 2359, XP093250116, ISSN: 1996-1944, DOI: 10.3390/ma6062351**
- **FARIN B. ET AL: "NiMoO4 preparation from polyampholytic hybrid precursors: Benefiting of the memory effect in the oxidative dehydrogenation of propane", CATALYSIS TODAY, vol. 203, 1 March 2013 (2013-03-01), AMSTERDAM, NL, pages 24 - 31, XP093250127, ISSN: 0920-5861, DOI: 10.1016/ j.cattod.2012.03.070**
- **RULLENS FRANÇOIS ET AL: "New Regular, Amphiphilic Poly(ampholyte)s: Synthesis and Characterization", MACROMOLECULAR CHEMISTRY AND PHYSICS, vol. 205, no. 9, 28 May 2004 (2004-05-28), DE, pages 1155 - 1166, XP093250130, ISSN: 1022-1352, DOI: 10.1002/ macp.200400081**
- **NAGAKUBO YUKI ET AL: "Accurate detection of KRAS, NRAS and BRAF mutations in metastatic colorectal cancers by bridged nucleic acid-clamp real-time PCR", vol. 12, no. 1, 1 December 2019 (2019-12-01), London UK, XP093117970, ISSN: 1755-8794, Retrieved from the Internet <URL:https://www.ncbi.nlm.nih.gov/pmc/ articles/PMC6849194/pdf/ 12920_2019_Article_610.pdf> DOI: 10.1186/ s12920-019-0610-8**
- **TACHIBANA AMI ET AL: "Cationic copolymers that enhance wild-type-specific suppression in BNA-clamp PCR and preferentially increase the T m of fully matched complementary DNA and BNA strands", vol. 7, no. 1, 10 January 2022 (2022-01-10), XP093068867, Retrieved from the Internet <URL:https://academic.oup.com/ biomethods/article-pdf/7/1/bpac009/43858911/ bpac009.pdf> DOI: 10.1093/biomethods/bpac009**
- **SAKAKI S, NAKABAYASHI N: "MPC polymer ni yoru 1 enki takata (SNPs) no kokateki na ninshiki / EFFECTIVE RECOGNITION OF SINGLE NUCLEOTIDE POLYMORPHISM (SNPs) BY USE OF MPC POLYMERS", POLYMER PREPRINTS. JAPAN, SOCIETY OF POLYMER SCIENCE., JP, vol. 50, no. 5, 1 May 2001 (2001-05-01), JP , pages 992, XP002960296**
- **JIALUN HAN, WU JINCAI, DU JIE: "Fluorescent DNA Biosensor for Single-Base Mismatch Detection Assisted by Cationic Comb-Type Copolymer", MOLECULES, SPRINGER VERLAG, BERLIN, DE, vol. 24, no. 3, DE , pages 575, XP055556584, ISSN: 1433-1373, DOI: 10.3390/molecules24030575**
- **TACHIBANA AMI, FUJIMURA NAHOHIRO, TAKEUCHI MINORU, WATANABE KOJI, TERUUCHI YOKO, UCHIKI TOMOAKI: "Cationic copolymers that enhance wild-type-specific suppression in BNA-clamp PCR and preferentially increase the T m of fully matched complementary DNA and BNA strands", BIOLOGY METHODS AND PROTOCOLS, vol. 7, no. 1, 10 January 2022 (2022-01-10), XP093068867, DOI: 10.1093/biomethods/bpac009**


Remarks:
The complete document including Reference Table(s) and the Sequence Listing(s) can be downloaded from the EPO website

(52) Cooperative Patent Classification (CPC): (Cont.)

C-Sets
**C08F 220/06, C08F 220/56, C08F 220/60;**
**C08F 220/06, C08F 220/56, C08F 226/04;**
**C08F 220/54, C08F 220/56;**
**C08F 226/02, C08F 222/02;**
**C08F 226/04, C08F 222/02;**
**C12Q 1/6827, C12Q 2531/113, C12Q 2561/113, C12Q 2525/117, C12Q 2527/125**

## Description

TECHNICAL FIELD

The invention is set out in the appended set of claims.

BACKGROUND ART

Mutations in genes may cause cancer, and early discovery of such mutations in genes is expected to lead to early treatment of cancer. A certain genetic mutation has been also found to be greatly involved in susceptibility to certain kinds of diseases, therapeutic effects of drugs, strength of side effects, and the like. For example, Gefitinib (trade name: Iressa), which is a tyrosine kinase inhibitor of epidermal growth factor receptor (EGFR), is known to show great efficacy in some lung cancer patients having specific genetic mutations, while causing severe interstitial pneumonia in about 0.5% of patients (Non-Patent Literature 1). In colorectal cancer, it is known that when there are mutations in the KRAS gene, the response rate of cetuximab (trade name: Erbitux), a molecularly targeted drug, is low, whereas the response rate is high when there is no mutation in that gene. Therefore, if the efficacy and side effects can be predicted for each patient, it is possible to perform effective treatment with fewer side effects while refraining from administration to a patient in whom efficacy cannot be expected, or a patient in whom serious side effects are expected.

From this perspective, it is recognized that clarifying gene mutations is important in terms of early discovery of cancer, improvement of treatment efficiency, and the like, and various technologies for detecting genetic mutation have been developed in order to meet such needs. Particularly, remarkable progress has been seen in gene amplification technologies such as PCR methods and comprehensive gene analysis technologies such as NGS.

However, genes extracted from a tumor specimen contains a large amount of wild-type gene and a trace amount of mutant gene, but many conventional methods for detecting a mutant gene still do not have sufficient sensitivity for detecting a trace amount of a mutant gene. For example, as one of methods for detecting gene mutations, there is a method comprising amplifying mutant genes and a wild-type gene without separating them, and then separating the mutant genes from the wild-type gene by an electrophoresis method, a hybridization method, or the like. However, this method has difficulty of detecting an extremely small amount of mutant gene in the wild-type genes with sufficient sensitivity and accuracy.

On the other hand, a so-called "clamp method" has been developed in which mutant genes are selectively amplified in the stage of gene amplification by inhibiting amplification of a wild-type gene using an artificial oligonucleotide having a base sequence complementary to a standard sequence of the wild-type gene. The artificial nucleic acid used in this method is referred to as a clamp nucleic acid, and has characteristics that the artificial nucleic acid (i) strongly hybridizes with a wild-type gene, but (ii) does not strongly hybridize with mutant genes, and (iii) is less likely to be degraded in a nucleic acid amplification process. When real-time PCR is performed using this nucleic acid, mutations are considered detectable until the frequency of mutant genes reaches 1%, and when a direct sequencing method is performed using the amplified product of real-time PCR as a template, mutations are considered detectable until the frequency of mutant genes reaches 0.1% (for example, Patent Literature 1, Non-Patent Literature 2, and Non-Patent Literature 3). As clamp nucleic acids, a peptide nucleic acid (PNA), a locked nucleic acid (LNA), and a bridged nucleic acid (BNA) have been developed, and amplification methods such as a PNA-LNA clamp PCR method and a BNA clamp PCR method have been developed (see, for example, Non-Patent Literatures 2, 3, and 6 and Patent Literatures 1 to 9.).

However, even with these clamp methods, when real-time PCR is performed in conditions where the ratio of mutant genes is very lower relative to the ratio of wild-type genes in a sample, the wild-type gene may be amplified, and the wild-type gene and the mutant genes may not be distinguished from each other. In this regard, when a direct sequencing method is performed using the amplified product of clamp PCR as a template, detection sensitivity of the mutant genes can be increased, but that sequencing is unsuitable for clinical fields where it is required to immediately process a large number of specimen.

Patent Literature 12 relates to a method of detecting a difference in the base sequences by using a clamp nucleic acid in nucleic acid amplification techniques, wherein the clamp nucleic acid is an oligonucleotide analog containing one or more of one or more kinds of unit structures of nucleoside analogs represented by formula (I), formula (II), formula (III) or formula (VI).

CITATION LIST

PATENT LITERATURE

PATENT LITERATURE 1: JP-B-6242336
PATENT LITERATURE 2: JP-B-5813263
PATENT LITERATURE 3: JP-B-4731324
PATENT LITERATURE 4: JP-B-4383178
PATENT LITERATURE 5: JP-B-5030998
PATENT LITERATURE 6: JP-B-4151751
PATENT LITERATURE 7: JP-A-2001-89496
PATENT LITERATURE 8: WO 2003/068795
PATENT LITERATURE 9: WO 2005/021570
PATENT LITERATURE 10: JP-B-3756313
PATENT LITERATURE 11: WO 2016/167320
PATENT LITERATURE 12: WO 2014/051076

NON-PATENT LITERATURE

NON-PATENT LITERATURE 1: Thomas J., et.al., 2004, The NEW ENGLAND JURNAL of MEDICINE, 350;21
NON-PATENT LITERATURE 2: Nagai K., et.al., 2005, Cancer Research, 65:7276-7282
NON-PATENT LITERATURE 3: Nishino K. et al., 2019, Guide for EGFR gene mutation testing in lung cancer patients, Edition 4.1
NON-PATENT LITERATURE 4: Luming Z. et al., 2011 BioTechniques, 50:311-318
NON-PATENT LITERATURE 5: Nagakubo Y. et al., 2019, BMC Medical Genomics, 12:162
NON-PATENT LITERATURE 6: Murina F.F. et al., 2020, Molecules, 25(4):786

SUMMARY OF INVENTION

TECHNICAL PROBLEM

As described above, there is a need for a method capable of detecting a mutant genes even in conditions where the ratio of mutant genes is very lower relative to the ratio of a wild-type gene in a sample in clinical or research sites, and research is in progress to address this need (Patent Literature 2, Non-Patent Literature 4, Non-Patent Literature 5). However, the conventional methods require expensive equipment and two or more complicated analysis steps to detect mutant genes with high sensitivity (Non-Patent Literature 2), and there is a demand for a method capable of detecting mutant genes in a sample with high sensitivity by a simple process in clinical and research sites.

An object of the present disclosure is thus to provide a simple method capable of detecting mutant genes at a mutant gene content lower than the detection limit of mutant genes by the conventional clamp-PCR methods; and a kit that implements the method.

It is another object of the present disclosure to provide a simple method capable of selectively inhibiting amplification of a target base sequence in a nucleic acid amplification reaction; and a kit for implementing the method.

SOLUTION TO PROBLEM

While repeatedly conducting studies for increasing the detection sensitivity for mutant genes by a clamping method, the present inventors conducted a nucleic acid amplification reaction in the presence of a clamp nucleic acid and a specific amphoteric copolymer, and as a result, the effect of selectively inhibiting amplification of a wild-type gene by the clamp nucleic acid was enhanced by the presence of the amphoteric copolymer, which allowed the inventors to successfully increase the detection sensitivity of mutant genes.

That is, the present invention provides a method for detecting mutation in a target base sequence of a nucleic acid in a sample relative to a standard base sequence, comprising the steps of:

subjecting the nucleic acid in the sample to a nucleic acid amplification reaction as a template with
a clamp nucleic acid having a base sequence complementary to the standard base sequence and containing at least one artificial nucleotide residue, and
an amphoteric copolymer that comprises a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in a range of 0.13 to 1.62, and wherein at least some of the cationic constituent units (1) are a constituent unit (1-1), (1-3) or (1-4) having a structure represented by general

formula (I-a), (I-b), (I-e) or (I-f), or a structure of an acid addition salt thereof:

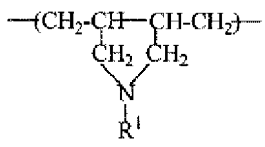
(I-a)

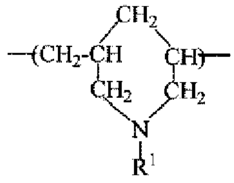
(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

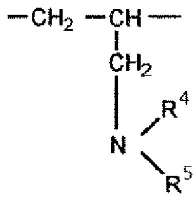
(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

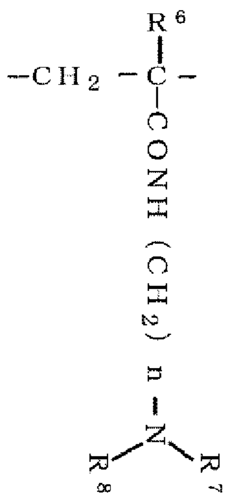
(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and
at least some of the anionic constituent units (2) are a constituent unit (2-1), (2-2) or (2-4) represented by general formula (II-a), (II-b) or (II-d):

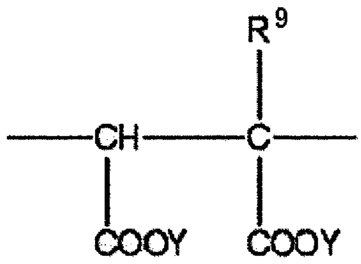

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

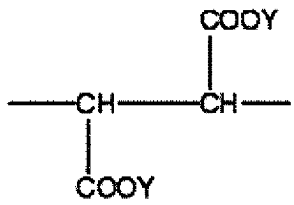

(II-b)

wherein Y represents a hydrogen, Na, K, $NH_4$, 1/2Ca, 1/2Mg, 1/2Fe, 1/3Al, or 1/3Fe independently for each carboxy group to be bonded, or

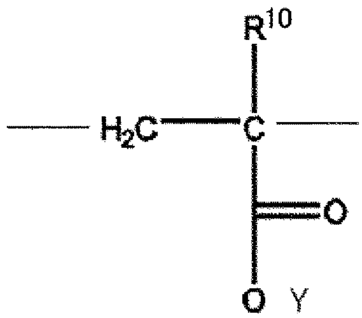

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded; and

determining the presence of the mutation based on the total amount of nucleic acid amplification products obtained by the nucleic acid amplification reaction, the number of amplification cycles of the nucleic acid amplification reaction required for the total amount of nucleic acid amplification products to reach a threshold value, the amount of nucleic acids with the mutation among nucleic acid amplification products, or the number of amplification cycles of the nucleic acid amplification reaction required for the amount of nucleic acids with the mutation among nucleic acid amplification products to reach a threshold value.

The present invention also provides a kit for detecting a mutation in a target base sequence of a nucleic acid in a sample relative to a standard base sequence, comprising:

a clamp nucleic acid having a base sequence complementary to the standard base sequence and containing at least one artificial nucleotide residue, and

an amphoteric copolymer comprising a cationic constituent unit (1) with an amino group in its structure and an anionic

constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

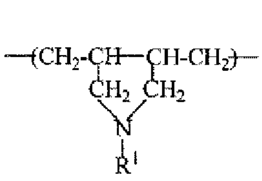

(I-a)

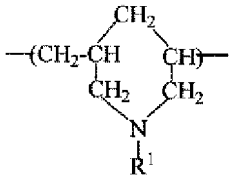

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

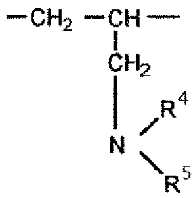

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

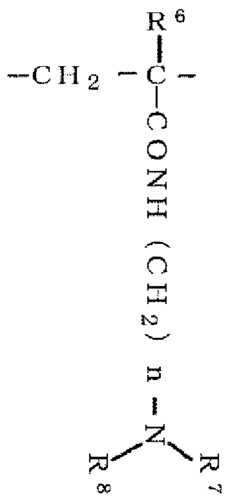

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and
at least some of the anionic constituent units (2) are a constituent unit (2-1), (2-2) or (2-4) represented by general formula (II-a), (II-b) or (II-d):

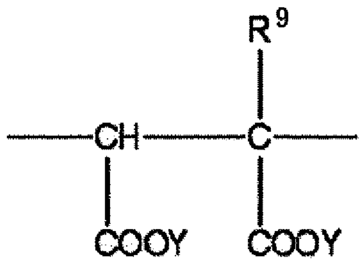

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

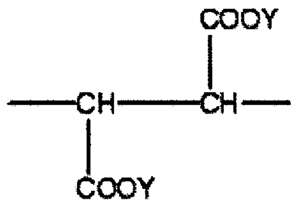

(II-b)

wherein Y represents a hydrogen, Na, K, $NH_4$, 1/2Ca, 1/2Mg, 1/2Fe, 1/3Al, or 1/3Fe independently for each carboxy group to be bonded, or

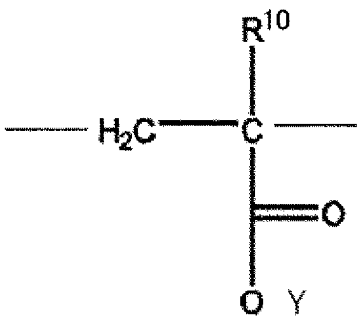

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

The present invention also provides a method for suppressing amplification of a nucleic acid in a sample having a predetermined target base sequence in a nucleic acid amplification reaction, comprising:

subjecting the nucleic acid in the sample to a nucleic acid amplification reaction as a template with
a clamp nucleic acid having a base sequence complementary to the target base sequence and containing at least one artificial nucleic acid residue, and
an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

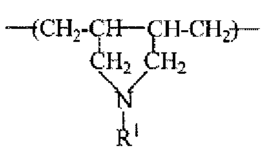 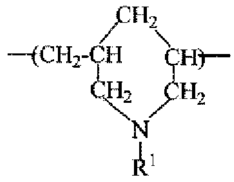

(I-a) (I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

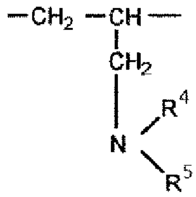

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

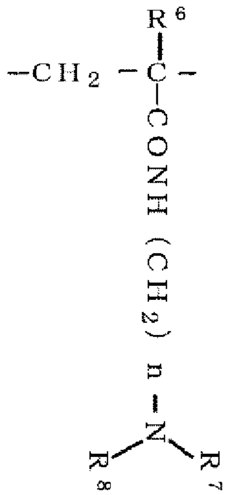

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and
at least some of the anionic constituent units (2) are a constituent unit (2-1), (2-2) or (2-4) represented by general formula (II-a), (II-b) or (II-d):

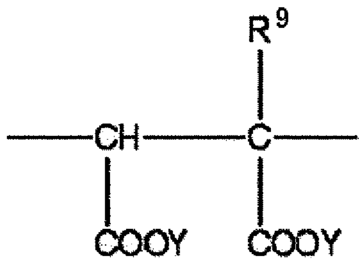

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

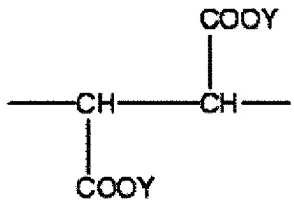

(II-b)

wherein Y represents a hydrogen, Na, K, $NH_4$, 1/2Ca, 1/2Mg, 1/2Fe, 1/3Al, or 1/3Fe independently for each carboxy group to be bonded, or

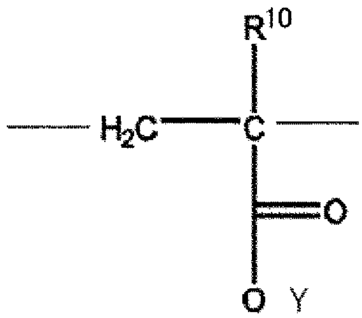

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

The present invention also provides a kit for suppressing amplification of a nucleic acid having a target base sequence in a nucleic acid amplification reaction, comprising:

a clamp nucleic acid having a base sequence complementary to the target base sequence and containing at least one artificial nucleotide residue, and

an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

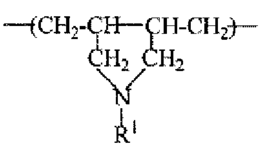

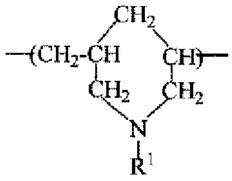

(I-a) (I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

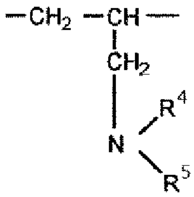

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

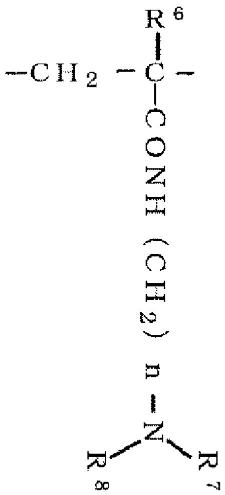

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and
at least some of the anionic constituent units (2) are a constituent unit (2-1), (2-2) or (2-4) represented by general formula (II-a), (II-b) or (II-d):

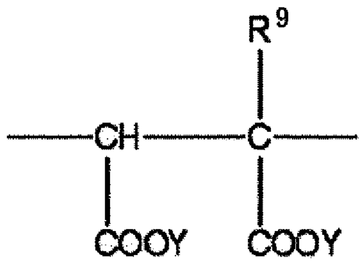

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

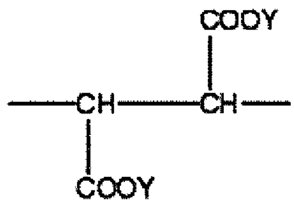

(II-b)

wherein Y represents a hydrogen, Na, K, $NH_4$, 1/2Ca, 1/2Mg, 1/2Fe, 1/3Al, or 1/3Fe independently for each carboxy group to be bonded, or

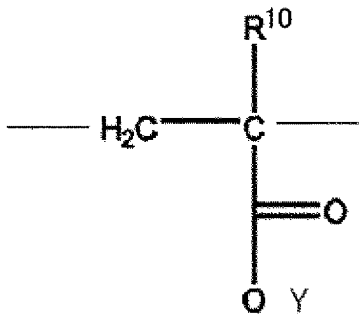

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

The present invention also provides the use of an agent for enhancing an effect of inhibiting nucleic acid amplification of a nucleic acid having a target base sequence by a clamp nucleic acid that has a base sequence complementary to the target base sequence and contains at least one artificial nucleotide residue, the agent comprising:

an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

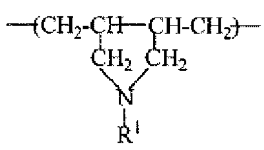

(I-a)

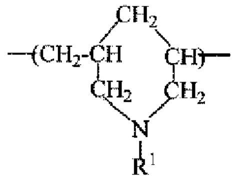

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

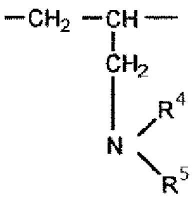

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

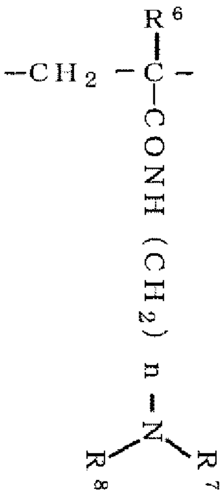

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and

at least some of the anionic constituent units (2) are a constituent unit (2-1), (2-2) or (2-4) represented by general formula (II-a), (II-b) or (II-d):

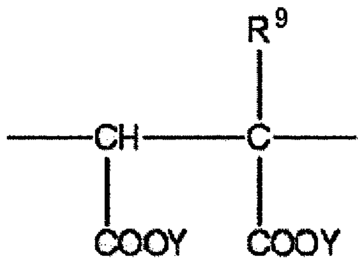

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

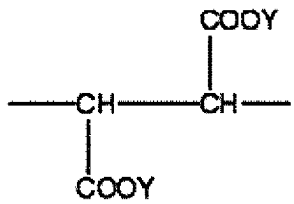

(II-b)

wherein Y represents a hydrogen, Na, K, $NH_4$, 1/2Ca, 1/2Mg, 1/2Fe, 1/3Al, or 1/3Fe independently for each carboxy group to be bonded, or

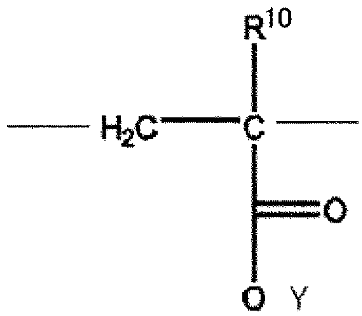

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

The amphoteric copolymer itself does not inhibit a nucleic acid amplification reaction, but can selectively enhance the effect of inhibiting amplification of a nucleic acid having a sequence complementary to a clamp nucleic acid by the clamp nucleic acid. Therefore, according to one embodiment of the present disclosure, even when the ratio of nucleic acids having mutations relative to a standard base sequence complementary to a clamp nucleic acid in a sample is very lower (for example, 0.1% or less) compared to the ratio of a nucleic acid having the standard base sequence, amplification of the nucleic acid having the standard base sequence is selectively inhibited by the clamp nucleic acid, while the nucleic acids having mutation relative to the standard base sequence are amplified as usual, so that the nucleic acids having mutation can be detected with very high sensitivity by a simple method.

BRIEF DESCRIPTION OF DRAWINGS

FIG. 1(a) shows a nucleic acid amplification curve in the case of using the polymer of Example 2 (copolymer of diallylmethylamine/maleic acid 1 : 1), as an example of a nucleic acid amplification curve in a case where a PCR reaction was not inhibited in Test 1. FIG. 1(b) shows a nucleic acid amplification curve in the case of using the polymer of Comparative Example 7 (copolymer of diallyldimethylammonium chloride/acrylamide 8 : 1), as an example of a nucleic acid amplification curve in a case where a PCR reaction was inhibited in Test 1.

FIG. 2 shows, as an example of a nucleic acid amplification curve in the case of enhancing an effect of selectively inhibiting amplification of a wild-type gene by a clamp nucleic acid containing BNA (BNA clamp) without inhibiting amplification of mutant genes, nucleic acid amplification curves in the cases where real-time PCR reactions were implemented using a wild-type gene or a mutant gene as a template in the presence or absence of the BNA clamp, with or without addition of the polymer of Example 4 (copolymer of dimethylaminopropyl methacrylamide hydrochloride/acrylamide/acrylic acid 1 : 1 : 2).

FIG. 3 shows nucleic acid amplification curves in the cases where real-time PCR reactions were implemented using, as a template, KRAS gene with a mutant form content rate of 10%, 1%, 0.10%, 0.01%, or 0% (WT) in the presence of a BNA clamp, without addition of a polymer, or with addition of the polymer of Example 1, 2, 4, or 6 (copolymer of diallylamine hydrochloride/maleic acid 1 : 1, copolymer of diallylmethylamine/maleic acid 1 : 1, copolymer of dimethylaminopropylmethacrylamide hydrochloride/acrylamide/acrylic acid 1 : 1 : 2, or copolymer of diallylamine hydrochloride/acrylamide/acrylic acid 1 : 1 : 2).

FIG. 4 shows the structure of KRAS WT/Mutant plasmid DNA used in Test 5.

FIG. 5 shows nucleic acid amplification curves in the cases where real-time PCR reactions were performed using, as a template, plasmid DNAs of FIG. 4 having KRAS wild-type gene or KRAS mutant gene at a mutant rate of 0.01% in the presence of a BNA clamp, without or with addition of the polymer of Example 1, 2, 4, or 6 (copolymer of diallylamine hydrochloride/maleic acid 1 : 1, copolymer of diallylmethylamine/maleic acid 1 : 1, copolymer of dimethylaminopropylmethacrylamide hydrochloride/acrylamide/acrylic acid 1 : 1 : 2, or copolymer of diallylamine hydrochloride/acrylamide/acrylic acid 1 : 1 : 2).

FIG. 6 shows a result obtained by sequencing a PCR product obtained by implementing a real-time PCR reaction using plasmid DNAs of FIG. 4 having KRAS wild-type gene or KRAS mutant gene at a mutant rate of 0.01% as a template in the presence of a BNA clamp without or with addition of the polymer of Example 1, 2, 4, or 6 (copolymer of diallylamine hydrochloride/maleic acid 1 : 1, copolymer of diallylmethylamine/maleic acid 1 : 1, copolymer of dimethylaminopropylmethacrylamide hydrochloride/acrylamide/acrylic acid 1 : 1 : 2, or copolymer of diallylamine hydrochloride/acrylamide/acrylic acid 1 : 1 : 2).

DESCRIPTION OF EMBODIMENTS

The invention is set out in the appended set of claims. Embodiments of the invention will be described in detail below.

1. Method for Detecting Mutation in Target Base Sequence Relative to Standard Base Sequence

One embodiment of the present invention relates to a method for detecting mutation in a target base sequence of a nucleic acid in a sample relative to a standard base sequence, comprising the steps of:

subjecting the nucleic acid in the sample to a nucleic acid amplification reaction as a template with
a clamp nucleic acid having a base sequence complementary to the standard base sequence and containing at least one artificial nucleotide residue, and
an amphoteric copolymer that comprises a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in a range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

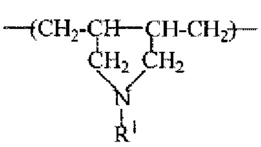
(I-a)

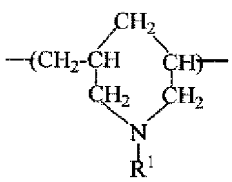
(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

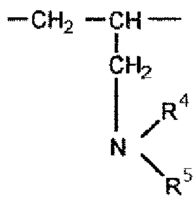

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

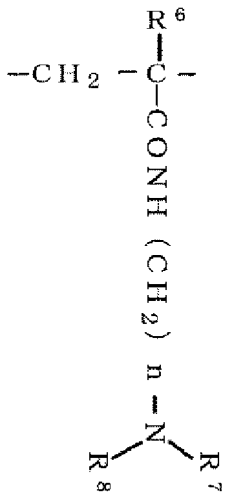

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and
at least some of the anionic constituent units (2) are a constituent unit (2-1), (2-2) or (2-4) represented by general formula (II-a), (II-b) or (II-d):

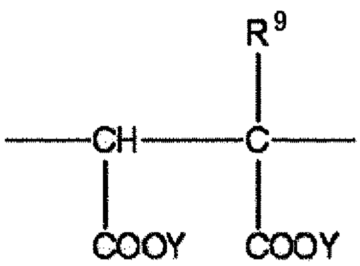

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

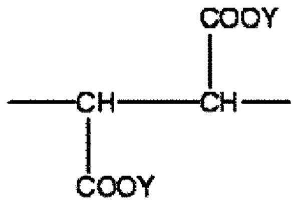

(II-b)

wherein Y represents a hydrogen, Na, K, $NH_4$, 1/2Ca, 1/2Mg, 1/2Fe, 1/3Al, or 1/3Fe independently for each carboxy group to be bonded, or

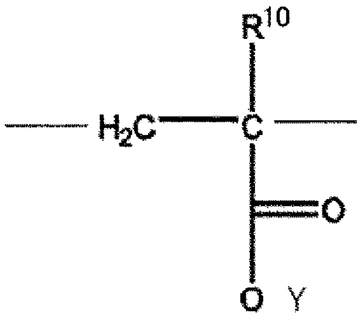

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded; and
determining the presence of the mutation based on the total amount of nucleic acid amplification products obtained by the nucleic acid amplification reaction, the number of amplification cycles of the nucleic acid amplification reaction required for the total amount of nucleic acid amplification products to reach a threshold value, the amount of nucleic acids with the mutation among nucleic acid amplification products, or the number of amplification cycles of the nucleic acid amplification reaction required for the amount of nucleic acids with the mutation among nucleic acid amplification products to reach a threshold value.

Sample

The samples provided for the methods are assumed to contain a nucleic acid for which the detection of mutation is desired or a nucleic acid that may be subject to amplification inhibition, depending on the embodiments. In the present embodiment, a sample is assumed to contain a nucleic acid for which detection of mutation is desired. The sample is, for example, prepared from a specimen collected from a mammal, typically a human. Examples thereof include liquid samples such as blood, pleural effusion, bronchial lavage fluid, bone marrow fluid, and lymph fluid, and samples prepared from solid samples such as lymph nodes, blood vessels, bone marrow, brain, spleen, and skin. Since the method of the present embodiment can detect mutation in a nucleic acid with very high sensitivity, for example, not only a sample from a specimen collected from a lesion site such as cancer but also a sample from a specimen containing only a trace amount of a mutant gene such as blood can be used.

Target Base Sequence

As used herein, the "target base sequence" means a base sequence that is a target for detection of mutation or inhibition of amplification, depending on the methods according to the embodiments of the present disclosure. Further, the "nucleic acid in a sample" is a nucleic acid that may contain a base sequence to be subjected to detection of mutation or inhibition of amplification. However, the "target base sequence" is not meant to actually have mutation or actually contain a base sequence of which amplification is inhibited, and the "nucleic acid in a sample" is not meant to actually contain a base sequence that is a target for detection of mutation or inhibition of amplification.
In the method according to the present embodiment, the "target base sequence" is a base sequence that is a

target for detecting the presence or absence of mutation, and the "nucleic acid in a sample" is a nucleic acid containing a base sequence that is a target for detecting presence or absence of mutation. The "region containing a target base sequence" is a region to be amplified by the methods of the present disclosure.

Nucleic Acid in Sample

The nucleic acid may be DNA or RNA and may include any natural or artificial nucleic acids such as genomic DNA, cDNA, plasmid DNA, cell-free DNA, circulating DNA, RNA, miRNA, and mRNA. In the case of using a sample from a specimen collected from a mammal, typically a human, the nucleic acid to be subjected to detection of mutation is often DNA, typically genomic DNA having great clinical significance. Further, even from the viewpoint that the effect of enhancing the inhibitory effect on nucleic acid amplification by a clamp nucleic acid is increased, a nucleic acid in a sample is preferably a genomic DNA.

Examples of the genomic DNA include genomic DNA in which mutation (inherited or acquired mutation) is associated with development of a specific disease and/or therapeutic sensitivity. A large number of such diseases are known, and representative examples thereof include various cancers. Examples of the gene in which mutation is known to be associated with development of cancer and/or therapeutic sensitivity, include ABL/BCR fusion gene (chronic myelogenous leukemia), HER2 gene (breast cancer), EGFR gene (non-small cell lung cancer), c-KIT gene (gastrointestinal stromal tumor), KRAS gene (colon cancer, pancreatic cancer), BRAF gene (melanoma, colorectal cancer), PI3KCA gene (lung cancer, colorectal cancer), FLT3 gene (acute myelogenous leukemia), MYC gene (various cancers), MYCN gene (neuroblastoma), MET gene (Lung cancer, gastric cancer, melanoma), BCL2 gene (follicular B-lymphoma), and EML4/ALK fusion gene (lung cancer).

Standard Base Sequence and Mutation

As used herein, the "standard base sequence" means a base sequence that serves as a reference when determining whether a "target base sequence" has mutation in a method for detecting mutation in the "target base sequence", and the term "mutation", "a mutation", and "mutations" mean a state in which the "target base sequence" has some difference in base sequence due to substitution, insertion, deletion, inversion, duplication, translocation, or a combination of two or more thereof, relative to the "standard base sequence". In the specification, such a "mutation", "a mutation", or "mutations" may be a difference in base sequence of 20% or less, a difference in base sequence of 10% or less, a difference in base sequence of 5% or less, or a difference in base sequence of 1% or less.

A "standard base sequence" can be selected from various base sequences according to the purpose of a test, but is typically a base sequence derived from a wild-type nucleic acid, and is preferably a base sequence derived from a wild-type gene of which mutation is known to be associated with development of a specific disease and/or therapeutic sensitivity. Therefore, a "mutant nucleic acid" is typically a nucleic acid having some difference in base sequence due to substitution, insertion, deletion, inversion, duplication, translocation, or a combination of two or more thereof relative to a base sequence derived from a wild-type nucleic acid, and is preferably a nucleic acid having such a difference in base sequence relative to a wild-type gene of which mutation is known to be associated with development of a specific disease and/or therapeutic sensitivity. For example, it is known that mutations at the 12th codon or the 13th codon in the second exon of the KRAS gene are associated with the response rate of cetuximab, a molecularly targeted drug. Further, in the BRAF gene, it is known that mutations at the 600th codon in exon 15 are associated with the response rate of molecularly targeted drugs, cetuximab and panitumumab.

Since the specific amphoteric copolymer particularly enhances the effect of inhibiting nucleic acid amplification by a clamp nucleic acid, an expected ratio of a target base sequence containing mutation with respect to the total of a sequence not containing the mutation (i.e., standard base sequence) and the target sequence containing the mutation, which are contained in nucleic acids in the above-mentioned sample, may be less than 0.1%. The ratio may be 0.05% or less in a preferred embodiment, and may be 0.01% or less in a more preferred embodiment. Meanwhile, an expected proportion of a target base sequence containing mutation could be clinically determined for each mutation.

Clamp Nucleic Acid

As used herein, a "clamp nucleic acid" is an artificial nucleic acid composed of an oligonucleotide that has a base sequence complementary to a "base sequence" which is a target for inhibiting nucleic acid amplification and contains at least one artificial nucleotide. It is to be noted that the "base sequence" as the target may be a "standard base sequence" or may be a "target base sequence", depending on the embodiments. The "clamp nucleic acid" hybridizes with a "base sequence" as a target for inhibiting nucleic acid amplification to inhibit nucleic acid amplification, but does not strongly hybridize with a nucleic acid of a base sequence that is not complementary to the targeted base sequence and does not inhibit nucleic acid amplification.

In a method for detecting mutation in a "target base sequence", a "base sequence" of which nucleic acid amplification is inhibited by a "clamp nucleic acid" is a "standard base sequence", and the "clamp nucleic acid" hybridizes with the "standard base sequence" to inhibit nucleic acid amplification thereof.

Examples of the artificial nucleotide include a peptide nucleic acid (PNA), a bridged nucleic acid (BNA), a peptide nucleic acid having a phosphate group (PHONA), and a morpholino nucleic acid. The first generation BNA is also referred to as locked nucleic acid (LNA) (see, for example, Non-Patent Literatures 2 and 3 and Patent Literatures 1 and 2.).

A clamp nucleic acid containing PNA (PNA clamp) is a fully modified artificial nucleic acid composed of an oligonucleotide containing one or more nucleotides forming a backbone with a peptide bond instead of a phosphate bond of nucleic acid. A modified nucleotide typically has a structure in which the sugar-phosphate backbone of the nucleotide is replaced with a backbone having N-(2-aminoethyl)glycine as a unit, and bases are bonded through a methylenecarbonyl bond. The details of the PNA are as described in Non-Patent Literature 6. Since a PNA clamp has stronger ability to hybridize with a DNA strand having a complementary structure thereto than a natural DNA has and does not undergo degradation by a nucleolytic enzyme, the PNA clamp has desirable characteristics as a clamp nucleic acid.

A clamp nucleic acid containing BNA (BNA clamp) is an artificial nucleic acid composed of an oligonucleotide containing one or more artificial nucleotides having a structure in which an oxygen atom at the 2'-position and a carbon atom at the 4'-position of ribose are crosslinked, and the first generation BNA is also referred to as locked nucleic acid (LNA). The BNA clamp is such that a helical structure is fixed, and when hybridizing with a nucleic acid having a complementary strand, the BNA clamp can form a very stable double strand. The bonding to a nucleic acid having a complementary strand is strengthened in proportion to the number of modified nucleotides, and thus appropriate hybridizing ability can be acquired by adjusting the length of the nucleic acid and the number of modified nucleotides, and it is possible to exhibit a clamping effect with a relatively short oligonucleotide chain.

Representative examples of the BNA clamp are oligonucleotides containing modified nucleotides having the following structures:

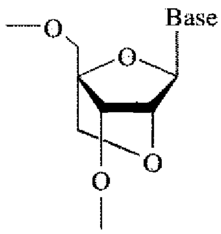

(I)

First generation BNA: 2',4'-BNA (LNA) (for example, Patent Literature 10)

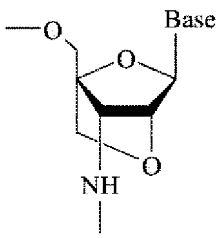

(I I)

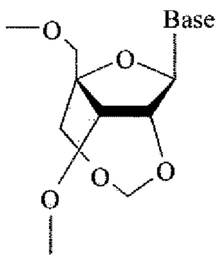

(I I I)

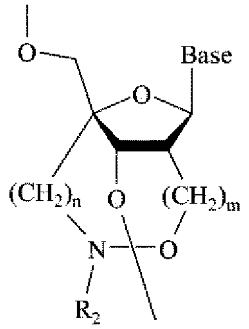

(I V)

wherein

Base represents a pyrimidine or purine nucleic acid base, or a pyrimidine or purine nucleic acid base substituted with a substituent selected from the group consisting of a hydroxyl group, a hydroxyl group protected with a protective group for nucleic acid synthesis, an alkoxy group having 1 to 5 carbon atoms, a mercapto group, a mercapto group protected with a protective group for nucleic acid synthesis, an alkylthio group having 1 to 5 carbon atoms, an amino group, an amino group protected with a protective group for nucleic acid synthesis, an amino group substituted with an alkyl group having 1 to 5 carbon atoms, an alkyl group having 1 to 5 carbon atoms, and a halogen atom (for example, a fluorine atom, a chlorine atom, a bromine atom, or an iodine atom);

$R_2$ represents a hydrogen atom, a linear or branched alkyl group having 1 to 20 carbon atoms, a linear or branched alkenyl group having 2 to 20 carbon atoms, a cycloalkyl group having 3 to 10 carbon atoms, an aryl group having 6 to 14 carbon atoms, an aralkyl group in which an alkyl group having 1 to 6 carbon atoms is substituted with an aryl group having 6 to 14 carbon atoms, an acyl group, a sulfonyl group, a silyl group, or a labeling molecule,

m is an integer of 0 to 2; and

n is an integer of 1 to 3.

A modified nucleotide of formula (IV) is preferably such that the Base is selected from the group consisting of benzoylaminopurin-9-yl, adeninyl, 2-isobutyrylamino-6 hydroxypurin-9-yl, guaninyl, 2-oxo-4 benzoylamino-1,2-dihydropyrimidin-1-yl, cytosinyl, 2-oxo-5-methyl-4-benzoylamino-1,2-dihydropyrimidin-1-yl, 5-methylcytosinyl, uracinyl, and thyminyl groups, $R_1$ is a methyl group, m is 0, and n is 1.

However, the BNA used in the present disclosure is not limited to the above-described ones, and other BNAs (for example, 5'-amino-2',4'-BNA, 2',4'-BNACOC, and 2',4'-BNANC) can also be used. Further, in the BNA clamp, the bond between a modified nucleotide and a nucleotide or the bond between modified nucleotides is usually a phosphodiester bond, but may include a phosphorothioate bond. The details of the BNA are described in Patent Literatures 1 to 10, and those skilled in the art to which the present disclosure is pertained can easily understand what artificial nucleic acid corresponds to the BNA clamp from known technical matters such as these documents.

As the clamp nucleic acid, a BNA clamp is preferable, and a BNA clamp containing a modified nucleotide having a structure of formula (IV) is particularly preferable, from the viewpoint that a relatively small number of nucleotides allow a stable double strand to be formed with a nucleic acid having a complementary sequence thereto to selectively inhibit nucleic acid amplification of the nucleic acid (ΔΔΔCt value is large with a method for detecting mutation based on the number of amplification cycles of a wild-type gene, such as an intercalator method that will be described later).

The length of a clamp nucleic acid and the number of artificial nucleotides thereof are factors that determine the binding force to a nucleic acid that is subjected to inhibition of nucleic acid amplification. Therefore, it is preferable that these factors are appropriately set so that a clamp nucleic acid can strongly bind to a nucleic acid that is subjected to inhibition of nucleic acid amplification (in the method for detecting mutation, a nucleic acid having the standard base sequence) and selectively inhibit amplification of the nucleic acid. From this point of view, the length of a clamp nucleic acid is usually 5- to 50-mer, preferably 6- to 30-mer, more preferably 7- to 25-mer, even more preferably 8- to 20-mer, and particularly preferably 9- to 15-mer.

From the same point of view, the proportion of the artificial nucleic acids in a clamp nucleic acid is usually 10 to 100%, preferably 30 to 95%, more preferably 40 to 90%, even more preferably 50 to 85%, and particularly preferably 60 to 80%.

The synthesis of a clamp nucleic acid can be performed according to the disclosure in literatures such as Patent Literatures 7 to 10. Further, the synthesis of a clamp nucleic acid may be outsourced to a contractor who synthesizes the clamp nucleic acid.

Amphoteric Copolymer

In a method of the present disclosure, when subjecting a nucleic acid in a sample to a nucleic acid amplification reaction as a template, the nucleic acid amplification reaction is performed in a condition where a specific amphoteric copolymer is present together with the above-described blocker nucleic acid. The amphoteric copolymer used in the present disclosure enhances a selective nucleic acid amplification inhibitory effect by a blocker nucleic acid, and comprises a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

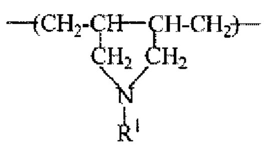

(I-a)

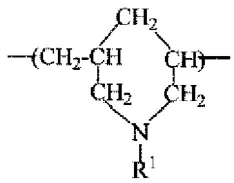

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

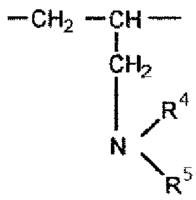

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

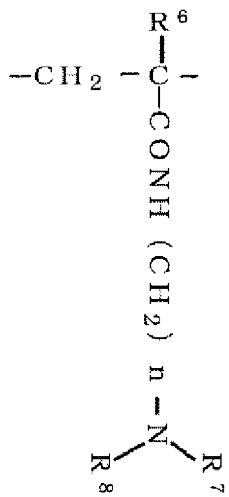

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and

at least some of the anionic constituent units (2) are a constituent unit (2-1), (2-2) or (2-4) represented by general formula (II-a), (II-b) or (II-d):

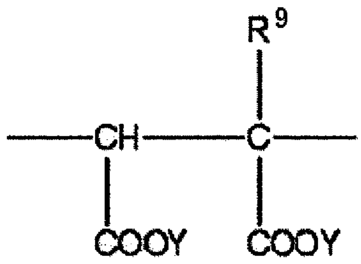

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

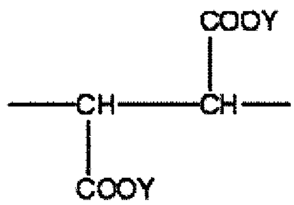

(II-b)

wherein Y represents a hydrogen, Na, K, $NH_4$, 1/2Ca, 1/2Mg, 1/2Fe, 1/3Al, or 1/3Fe independently for each carboxy group to be bonded, or

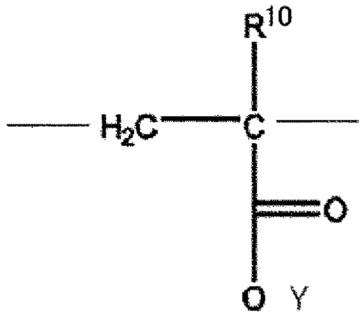

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

Constituent Unit (1-1)

A constituent unit (1-1) is a constituent unit having a structure represented by the following general formula (I-a) or (I-b), or a structure of an acid addition salt thereof. From the viewpoint of increasing the effect of enhancing the inhibitory effect on nucleic acid amplification by a clamp nucleic acid, a constituent unit (1-1) is a particularly preferable constituent unit as a cationic constituent unit (1). That is, if a constituent unit (1-1) constitutes entirely or partially the cationic constituent unit(s) (1), the inhibitory effect on nucleic acid amplification by a clamp nucleic acid can be further enhanced.

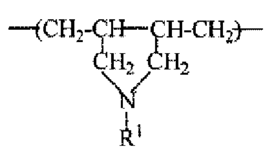
(I-a)

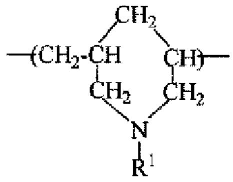
(I-b)

In the formula, $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms. $R^1$ represents preferably a hydrogen atom, a methyl group, an ethyl group, or a benzyl group, and particularly preferably a hydrogen atom or a methyl group.

A constituent unit (1-1) may be an inorganic acid salt, an organic acid salt, or the like of the structure represented by the above structural formula (1-a) or (1-b), that is, an acid addition salt thereof.

In a case where the amphoteric copolymer has a constituent unit (1-1), from the viewpoint of production costs, and the like, a diallylamine monomer having an addition salt is preferably used in producing the amphoteric copolymer. Since a process of removing the addition salt such as HCl from the polymer is complicated and causes an increase in cost, it is a preferable embodiment also from the viewpoint of costs and the like to use an addition salt-type constituent unit (1-1), with which the specific amphoteric copolymer can be produced without such a process.

From the viewpoint of the ease of availability, the controllability of reaction, and the like, an inorganic acid salt or organic acid salt of a constituent unit (1-1) of this embodiment is preferably a hydrochloride, a carboxylate, a sulfonate, or an alkyl sulfate, and particularly preferably a hydrochloride.

Constituent Unit (1-2)

A constituent unit (1-2) is a constituent unit having a structure represented by the following general formula (I-c) or (I-d):

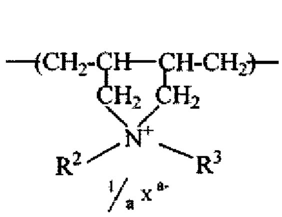
(I-c)

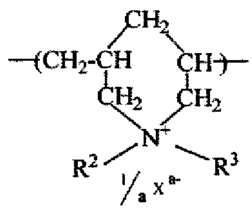
(I-d)

In the formula, $R^2$ and $R^3$ each independently represent a hydrogen atom, an alkyl group having 1 to 10 carbon atoms that may have a hydroxyl group, a cycloalkyl group having 5 to 10 carbon atoms, or an aralkyl group having 7 to 10 carbon atoms, $X^{a-}$ represents a counter ion, and a represents the valency number of the counter ion.

$R^2$ and $R^3$ each independently represent preferably a hydrogen atom, a methyl group, an ethyl group, or a benzyl group, and particularly preferably a hydrogen atom, or a methyl group.

There is no particular limitation on the counter ion $X^{a-}$, but from the viewpoint of the ease of availability, the controllability of reaction, and the like, the counter ion $X^{a-}$ is preferably a chloride ion, a carboxylic acid ion, a sulfonic acid ion, or an alkyl sulfate ion, and particularly preferably a chloride ion, or an ethyl sulfate ion.

From the viewpoint of production costs, and the like, a diallylamine monomer having a counter ion is preferably used in producing the amphoteric copolymer. Since a process of removing a counter ion from the polymer is complicated and causes an increase in cost, it is a preferable embodiment also from the viewpoint of costs and the like to use a specific amphoteric copolymer having a counter ion-type constituent unit (1-2) with which the amphoteric copolymer can be produced without such a process.

Constituent Unit (1-3)

A constituent unit (1-3) is a constituent unit having a structure represented by the following general formula (I-e), or a structure of an acid addition salt thereof.

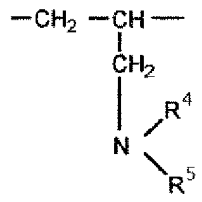

(1-e)

In the formula, $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms.

The alkyl group having 1 to 12 carbon atoms or the aralkyl group, which is preferable as $R^4$ and $R^5$, may be linear or branched. Examples of the group include a methyl group, an ethyl group, an n-propyl group, an isopropyl group, a n-butyl group, an isobutyl group, a sec-butyl group, a tert-butyl group, a pentyl group, a hexyl group, an octyl group, a decyl group, a dodecyl group, and a benzyl group. Examples of the cycloalkyl group having 5 to 6 carbon atoms, which is preferable as $R^4$ and $R^5$, include, but are not limited to, a cyclopentyl group, and a cyclohexyl group.

$R^4$ and $R^5$ each independently represent preferably a hydrogen atom, a methyl group, an ethyl group, or a benzyl group, and particularly preferably a hydrogen atom, or a methyl group.

There is no particular limitation on the type of an addition salt in a case where the constituent unit (1-3) is an acid addition salt having a structure represented by general formula (I-e), but from the viewpoint of the ease of availability, controllability of reaction, and the like, for example, a hydrochloride, a sulfate, a phosphate, a nitrate, a sulfite, a phosphite, a nitrite, a hydrobromide, an acetate, an amide sulfate, a methanesulfonate, a trifluoroacetate, a p-toluenesulfonate, or the like can be used.

Among them, a hydrochloride, a sulfate, a phosphate, and an amide sulfate are preferable, and a hydrochloride, a sulfate, a phosphate, and an amide sulfate which have a structure derived from monoallylamine, are particularly preferable.

Constituent Unit (1-4)

A constituent unit (1-4) is a constituent unit having a structure represented by the following general formula (I-f), or a structure of an acid addition salt thereof:

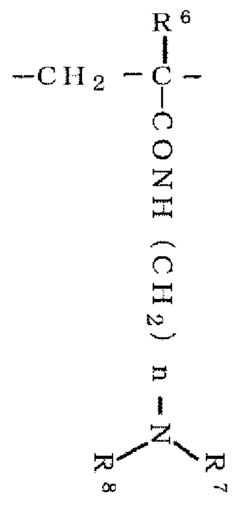

(1-f)

In the formula, $R^6$ represents a hydrogen atom or a methyl group, $R^7$ and $R^8$ each independently represent a hydrogen atom or an alkyl group having 1 to 4 carbon atoms, and n is an integer of 2 to 4.

$R^6$ is preferably a methyl group, n is preferably 2 to 3, and $R^7$ and $R^8$ are each independently preferably a hydrogen atom or a methyl group.

There is no particular limitation on the type of an addition salt in a case where the constituent unit (1-4) is an acid addition salt having a structure represented by general formula (I-f), but from the viewpoint of the ease of availability,

controllability of reaction, and the like, for example, a hydrochloride, a sulfate, a phosphate, a nitrate, a sulfite, a phosphite, a nitrite, a hydrobromide, an acetate, an amide sulfate, a methanesulfonate, a trifluoroacetate, a p-toluenesulfonate, or the like can be used.

Among them, a hydrochloride, a sulfate, a phosphate, and an amide sulfate are preferable, and a hydrochloride, a sulfate, a phosphate and an amide sulfate which have a structure derived from monoallylamine, are particularly preferable.

The proportion of a cationic constituent unit (1) to the whole constituent units of the amphoteric copolymer is usually 10 to 70 mol%, preferably 15 to 60 mol%, and particularly preferably 20 to 55 mol%. In a case where two or more types of cationic constituent units (1) are contained, the proportion of the unit is defined based on the total amount of two or more types of cationic constituent units (1).

Constituent Unit (2-1)

A constituent unit (2-1) is a constituent unit having a structure represented by the following general formula (II-a).

A constituent unit (2-1) constituting entirely or partially anionic constituent unit(s) (2), can further enhance the inhibitory effect on nucleic acid amplification by a clamp nucleic acid.

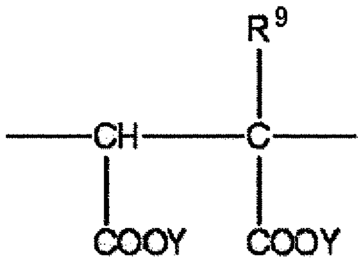

(II-a)

In the formula, $R^9$ represents a hydrogen or a methyl group, Y represents a hydrogen, Na, K, $NH_4$, 1/2Ca, 1/2Mg, 1/2Fe, 1/3Al, or 1/3Fe independently for each carboxy group to be bonded.

$R^9$ represents preferably a hydrogen, and Y represents preferably a hydrogen or Na. The constituent unit (2-1) is particularly preferably derived from maleic acid.

Constituent Unit (2-2)

A constituent unit (2-2) is a constituent unit having a structure represented by the following general formula (II-b):

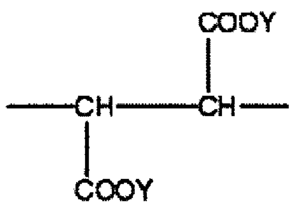

(II-b)

In the formula, Y represents a hydrogen, Na, K, $NH_4$, 1/2Ca, 1/2Mg, 1/2Fe, 1/3Al, or 1/3Fe independently for each carboxy group to be bonded.

Y represents preferably a hydrogen or Na.

Constituent Unit (2-3)

A constituent unit (2-3) is a constituent unit having a structure represented by the following general formula (II-c).

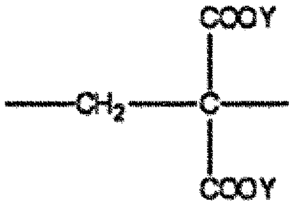

(II-c)

In the formula, Y is a hydrogen, Na, K, $NH_4$, 1/2Ca, 1/2Mg, 1/2Fe, 1/3Al, or 1/3Fe independently for each carboxy group to be bonded.

Y represents preferably a hydrogen or Na.

Constituent Unit (2-4)

A constituent unit (2-4) is a constituent unit having a structure represented by the following general formula (II-d):

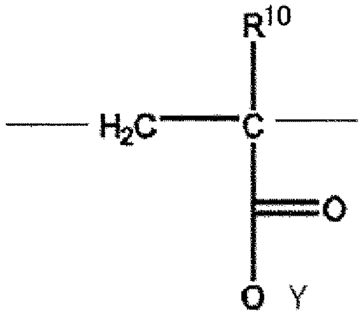

(II-d)

In the formula, $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded. $R^{10}$ represents preferably a hydrogen, and Y represents preferably a hydrogen or Na.

The constituent unit (2-4) is preferably derived from (meth)acrylic acid, and particularly preferably derived from acrylic acid.

The amphoteric copolymer, which comprises the above-described cationic constituent unit (1) having a specific structure and the above-described anionic constituent unit (2) and has a molar ratio of cationic constituent unit (1)/anionic constituent unit (2) in the above-described specific range, brings about remarkable effects such as enhancing the inhibitory effect on nucleic acid amplification by a clamp nucleic acid. Although not adhering to a theory, it is inferred that the mechanism for bringing about the remarkable effect may be due to as follows: A cationic constituent unit (1) having a specific structure stabilizes the binding between a clamp nucleic acid and a DNA by a strong positive charge thereof, but an excessively strong positive charge may inhibit the formation of a double strand of DNA and the denaturation of DNA into a single strand. With regard to this event, the presence of a constituent unit (2) having an anion charge at a specific molar ratio with respect to the cationic constituent unit (1) could moderately modify the positive charge of the cationic constituent unit (1), suppress inhibition of the formation of a double strand of DNA and the denaturation of DNA into a single strand, and allow the copolymer to express a function of stabilizing the binding between the clamp nucleic acid and DNA.

As described above, a cationic constituent unit (1) is preferably a constituent unit (1-1), and an anionic constituent unit (2) is preferably a constituent unit (2-1), and the amphoteric copolymer therefore particularly preferably comprises a constituent unit (1-1) as a cationic constituent unit (1) and a constituent unit (2-1) as an anionic constituent unit (2). That amphoteric copolymer can sufficiently enhance the inhibitory effect on nucleic acid amplification by a clamp nucleic acid even when the content rate of a target base sequence having mutation relative to a standard base sequence in a sample is low.

Other Constituent Units (3)

The amphoteric copolymer may comprise other constituent units in addition to a cationic constituent unit (1) and an anionic constituent unit (2), described above.

Examples of the other constituent units include a nonionic constituent unit (3-1) that will be described later and a cationic constituent unit (3-2) other than a cationic constituent unit (1), that is, having no amino group.

From the viewpoint of just adjusting the distance between a cationic constituent unit (1) and an anionic constituent unit (2), described above, it is preferable that the amphoteric copolymer further comprises a nonionic constituent unit (3-1).

Nonionic Constituent Unit (3-1)

A nonionic constituent unit (3-1) in the present embodiment may be derived from a nonionic monomer that can be copolymerized with a cationic constituent unit (1), described above, and an anionic constituent unit (2), described above, and is not otherwise particularly limited. A nonionic constituent unit (3-1) may be preferably a constituent unit derived from a methacrylic acid ester-based monomer, an acrylic ester-based monomer, a methacrylamide-based monomer, an acrylamide-based monomer, sulfur dioxide, or the like. More specific examples of those nonionic constituent units include constituent units derived from methyl methacrylate, ethyl methacrylate, butyl methacrylate, methyl acrylate, ethyl acrylate, butyl acrylate, methacrylamide, N-methyl methacrylamide, dimethyl methacrylamide, N-(3-dimethylaminopropyl) methacrylamide, acrylamide, dimethyl acrylamide, hydroxyethyl acrylamide, dimethylaminopropyl acrylamide, a dimethylaminopropyl acrylamide methyl chloride quaternary salt, acryloylmorpholine, isopropyl acrylamide, 4-t-butylcyclohexyl acrylate, and sulfur dioxide. A constituent unit derived from acrylamide or methacrylamide is particularly preferable.

A nonionic constituent unit (3-1) can usually be introduced into the amphoteric copolymer by using a nonionic monomer as a monomer.

There is no particular limitation on the content of a nonionic constituent unit (3-1) in a case where the amphoteric copolymer comprises the nonionic constituent unit (3-1), and a suitable amount thereof varies depending on the type of a nonionic constituent unit (3-1), but the molar ratio with an anionic constituent unit (2), as described above, that is, nonionic constituent unit (3-1)/anionic constituent unit (2) , as described above, is preferably 0.1/1 to 1/1, more preferably 0.2/1 to 0.8/1, still more preferably, 0.3/1 to 0.7/1, and particularly preferably 0.4/1 to 0.6/1.

In a case where a nonionic constituent unit (3-1) is derived from a methacrylic acid ester-based monomer, an acrylic ester-based monomer, a methacrylamide-based monomer, or an acrylamide-based monomer, the above-described molar ratio is preferably 0.1/1 to 1/1, more preferably 0.2/1 to 0.8/1, still more preferably, 0.3/1 to 0.7/1, and particularly preferably 0.4/1 to 0.6/1.

In a case where a nonionic constituent unit (3-1) is derived from sulfur dioxide, the above-described molar ratio is preferably 0.1/1 to 1/1, and particularly preferably 0.2/1 to 1/1.

The amphoteric copolymer comprises a cationic constituent unit (1), as described above, and an anionic constituent unit (2), as described above, and the amphoteric copolymer may therefore consist of a cationic constituent unit (1), as described above, and an anionic constituent unit (2), as described above, or may comprise another constituent unit in addition to a cationic constituent unit (1), as described above, and an anionic constituent unit (2), as described above. Examples of the other constituent units include a nonionic constituent unit (3-1) that will be described later and a cationic constituent unit (3-2) other than a cationic constituent unit (1) as described above.

The proportion of the total of a cationic constituent unit (1), as described above, and an anionic constituent unit (2), as described above, to the whole constituent units of the amphoteric copolymer is not particularly limited, but is usually 67 mol% or more, preferably 75 to 100 mol%, more preferably 80 to 100 mol%, and particularly preferably 90 to 100 mol%.

The molar ratio of a cationic constituent unit (1), as described above, to an anionic constituent unit (2), as described above, (cationic constituent unit (1)/anionic constituent unit (2)) in the amphoteric copolymer is in the range of 0.13 to 1.62.

The molar ratio of cationic constituent unit (1)/anionic constituent unit (2) in the amphoteric copolymer is preferably in the range of 0.25 to 1.25, and particularly preferably in the range of 0.50 to 1.00.

In a case where the structures of a cationic constituent unit (1), as described above, and a anionic constituent unit (2), as described above, are known, the molar ratio of the cationic constituent unit (1), as described above, and anionic constituent unit (2), as described above, in the amphoteric copolymer can be specified by reprecipitating the amphoteric copolymer with an organic solvent such as isopropyl alcohol, or acetone, and by analyzing the reprecipitation in appropriate modes according to the structures of the constituent units, using Perkin Elmer 2400II CHNS/O fully automatic elemental analyzer or a device with a performance equivalent thereto. In addition, the measurement can be performed by using helium gas as a carrier gas, weighing a solid sample into a tin capsule, dropping the sample into a combustion tube to burn the sample at a combustion temperature of 1800°C or higher in pure oxygen gas, detecting each component of interest by a frontal chromatography system with a separation column and a thermal conductivity detector, and quantifying the content of each element using a calibration factor. In a case where the structures of a cationic constituent unit (1), as

described above, and an anionic constituent unit (2), as described above, in the amphoteric copolymer are unknown, the structures of the respective constituent units are specified by a known method with 1H-NMR or 13C-NMR prior to the above-mentioned measurement by the elemental analyzer. The molar ratio can also be calculated from the amount of each monomer supplied in the production (copolymerization) of the amphoteric copolymer, and the amount of each monomer remaining without being incorporated into the amphoteric copolymer. In this regard, the proportion (molar ratio) of a constituent unit derived from each monomer in the amphoteric copolymer is considered almost corresponding to the monomer composition (molar ratio) for preparation of the constituent units, the mixing ratio of a monomer may be treated as the proportion (molar ratio) of a corresponding constituent unit for convenience herein.

The molar ratio of cationic constituent unit (1), as described above,/anionic constituent unit (2), as described above, in the amphoteric copolymer can be appropriately adjusted by selecting or adjusting the type and amount of each of the monomers supplied in the production (copolymerization) of the amphoteric copolymer, particularly, a monomer that results in a cationic constituent unit (1), as described above, and a monomer that results in an anionic constituent unit (2), as described above, and the conditions for the production (copolymerization), such as the type and amount of a catalyst, a copolymerization temperature and time.

Method for Producing Amphoteric Copolymer

There is no particular limitation on the method for producing the amphoteric copolymer, and the amphoteric copolymer can be produced by a method conventionally known in the art. For example, the amphoteric copolymer can be produced by copolymerizing a cationic monomer having a structure corresponding to a cationic constituent unit (1), as described above, an anionic monomer having a structure corresponding to an anionic constituent unit (2), as described above, and optionally a monomer such as a nonionic monomer having a structure corresponding to a nonionic constituent unit (3).

In a case of copolymerizing a cationic monomer having a structure corresponding to a cationic constituent unit (1), as described above, an anionic monomer having a structure corresponding to an anionic constituent unit (2), as described above, and others, a solvent is not particularly limited, and may be a water-based solvent, or an organic solvent such as alcohol, ether, sulfoxide, or amide, but is preferably a water-based solvent.

In the case of copolymerizing a cationic monomer having a structure corresponding to a cationic constituent unit (1), as described above, an anionic monomer having a structure corresponding to an anionic constituent unit (2), as described above, and others, the concentration of the monomers varies depending on the types of monomers and the type of a solvent used for copolymerization, but is usually 10 to 75% by mass in a water-based solvent. This copolymerization reaction is usually a radical polymerization reaction, and is performed in the presence of a radical polymerization catalyst. The type of the radical polymerization catalyst is not particularly limited, and preferred examples of the radical polymerization catalyst include a peroxide such as t-butyl hydroperoxide, a persulfate such as ammonium persulfate, sodium persulfate, or potassium persulfate, and a water-soluble azo compound such as an azobis-based compound, or a diazo-based compound.

The amount of a radical polymerization catalyst to be added is commonly 0.1 to 20 mol%, and preferably 1.0 to 10 mol% in relation to the whole monomers. The polymerization temperature is commonly 0 to 100°C and preferably 5 to 80°C, and the polymerization time is commonly 1 to 150 hours and preferably 5 to 100 hours. As to the polymerization atmosphere, even in the air, there is no major problem in polymerization, and the polymerization can be performed in an atmosphere of an inert gas such as nitrogen.

Nucleic Acid Amplification Reaction

In a method according to the present disclosure, a nucleic acid amplification reaction with a nucleic acid in a sample as a template is performed with a clamp nucleic acid and an amphoteric copolymer, as described above.

The method for nucleic acid amplification is not particularly limited, and examples thereof include PCR methods, such as a real-time PCR method, a digital PCR method, a traditional PCR method, and an RT-PCR method, a LAMP method, an ICAN method, a NASBA method, an LCR method, an SDA method, a TRC method, and a TMA method. The method for nucleic acid amplification is preferably a PCR method from the viewpoint of versatility and convenience of the technique, and is more preferably a real-time PCR method from the viewpoint of easy quantification of nucleic acid amplification products.

The composition of a reaction mixture for nucleic acid amplification varies somewhat depending on the method for nucleic acid amplification used, but usually, the composition may comprises a primer set, deoxynucleoside triphosphate (for example, dATP, dTTP, dCTP, and dGTP; hereinafter collectively referred to as dNTPs.) as substrates, and a nucleic acid synthase (for example, DNA polymerase) in a buffer in addition to the clamp nucleic acid and the amphoteric copolymer described above, and may also comprise, in some cases, magnesium ions (for example, contained at a concentration of 1 to 6mM in the reaction mixture) as a cofactor of the nucleic acid synthase.

Further, in the case of a method for nucleic acid amplification that simultaneously performs nucleic acid amplification and detection of amplified nucleic acid, such as a real-time PCR method, an appropriate detection reagent such as an intercalator, a fluorescent-labeled probe, or a cycling probe is also contained in a reaction mixture for nucleic acid amplification.

The primer set is only required to be able to amplify a region containing a target base sequence by a method for nucleic acid amplification, and is usually designed to hybridize to a predetermined region adjacent to a region to be amplified, but the primers may contain a sequence that hybridizes to a part of the target base sequence.

The primers can be composed of a nucleic acid such as DNA or RNA. Regarding the concentration of the primers, an appropriate concentration may be usually selected in the range of 20nM to 2μM in a reaction mixture. The length of the primer is usually 5- to 40-mer, preferably 12- to 35-mer, more preferably 14- to 30-mer, and even more preferably 15- to 25-mer. The distance between primers, that is, the region to be amplified, is usually 50 to 5000 bases, and preferably 100 to 2000 bases. The primers may be designed manually, or with an appropriate primer design software. Examples of such software include Primer3 software (http://frodo.wi.mit.edu).

The concentration of dNTPs may be selected from concentrations at which the concentration of each of dATP, dTTP, dCTP, and dGTP in a reaction mixture is usually in the range of 100 to 400 μM. Examples of the nucleic acid synthase include DNA polymerase, RNA polymerase, and reverse transcriptase, and an enzyme having properties suitable for the method for nucleic acid amplification may be used. For example, in the case of a PCR method, TaqDNA polymerase, PfuDNA polymerase, and other various heat-resistant DNA polymerases developed by bioscience-related companies are preferably used.

It is preferable to select a buffer having an optimum pH and an optimum salt concentration for a DNA polymerase to be used. Depending on the method for nucleic acid amplification, ribonuclease H (RNaseH), reverse transcriptase (RT), or the like may be further contained. Since various kits are commercially available for each type of the methods for nucleic acid amplification, a reaction mixture for nucleic acid amplification may be prepared with such a commercially available kit.

The concentration of a nucleic acid that serves as a template for a nucleic acid amplification reaction, that is, "nucleic acid in sample", in a reaction mixture may be usually 0.3 ng to 3 μg per 100 μl of a reaction mixture, but when the amount of a nucleic acid as a template is too large, the frequency of non-specific amplification increases, and therefore, it is preferable to suppress the concentration to 0.5 μg or less per 100 μl of a reaction mixture.

Regarding the amount of a clamp nucleic acid in a reaction mixture, it is preferable to select such an amount that, in a case where a target base sequence has mutation, amplification of a nucleic acid having a standard base sequence is sufficiently inhibited and a nucleic acid containing the target base sequence having mutation is preferentially amplified, depending on the amount of the nucleic acid having the target base sequence assumed to have mutation with respect to the amount of the nucleic acid having the standard base sequence (for example, a wild-type nucleic acid) in a sample. Since the majority of a wild type nucleic acid is usually found in a sample relative to mutant nucleic acids, the amount is preferably set to 10nM to 1μM, more preferably 20nM to 500nM, and particularly preferably 50nM to 200nM.

Regarding the concentration of the amphoteric copolymer, as described above, in a reaction mixture, it is preferable to select an appropriate concentration so that the effect of selectively inhibiting a nucleic acid amplification reaction by a clamp nucleic acid can be enhanced without adversely affecting the nucleic acid amplification reaction. The concentration of the amphoteric copolymer in a reaction mixture varies depending on the type of the polymer, but the concentration may be usually 0.001 to 0.500% by mass, and is preferably 0.010 to 0.300% by mass, more preferably 0.020 to 0.150% by mass, and particularly preferably 0.025 to 0.100% by mass.

When a nucleic acid amplification reaction with a nucleic acid in a sample as a template is performed by using a clamp nucleic acid and the amphoteric copolymer described above, the clamp nucleic acid strongly binds to a nucleic acid having a standard base sequence and inhibits annealing of a primer and/or extension of the primer during the amplification reaction, and the coexisting amphoteric copolymer enhances this inhibitory effect. On the other hand, the binding force of a clamp nucleic acid is greatly reduced for a base sequence having mutation relative to the standard base sequence, annealing and primer extension are hardly inhibited, and a normal nucleic acid amplification reaction occurs. The amphoteric copolymer itself does not inhibit the nucleic acid amplification reaction. As a result, according to a method of the present disclosure, even in the event where nucleic acids having mutation among the nucleic acids in a sample is in a trace amount (for example, in the case of 0.1% or less or 0.05% or less, particularly 0.02% or less), and the nucleic acid having the standard base sequence is dominant, amplification of the nucleic acid having the standard base sequence is selectively inhibited, and the nucleic acids having mutation are preferentially amplified, which enables detection nucleic acids with mutation contained in a trace amount.

According to the method of the present disclosure, the presence of mutation is detected employing the above-mentioned characteristics of the nucleic acid amplification reaction. That is, amplification of a nucleic acid having a standard base sequence is selectively inhibited, whereas in the case that a target base sequence has mutation relative to the standard base sequence, a region containing the target base sequence is preferentially amplified, and as a result, differences occur in the progress rate of a nucleic acid amplification reaction, the amount of a reaction product, and the like depending on the presence or absence of mutation. Therefore, these differences are used for the detection of mutation.

In the present disclosure, the detection of mutation can be done by a method of detecting the final product of an amplification reaction and a method of monitoring amplification over time during an amplification reaction. For example, mutation can be detected based on the total amount of the obtained nucleic acid amplification products, the number of amplification cycles of a nucleic acid amplification reaction required for the total amount of the nucleic acid amplification products to reach a threshold value, the amount of nucleic acids having mutation among nucleic acid amplification products, or the number of amplification cycles of a nucleic acid amplification reaction required for the amount of nucleic acids having mutation among nucleic acid amplification products to reach a threshold value. Particularly, from the viewpoint of simplicity of implementation and a wide adaptation range, preferable is a method of detecting mutation based on the number of amplification cycles of a nucleic acid amplification reaction required for the total amount of nucleic acid amplification products to reach a threshold value.

Further, accurate and highly sensitive detection can be achieved by examining the base sequences of amplification products by a direct sequencing method or the like. However, as demonstrated in Examples described below, according to the method of the present disclosure, it is possible to accurately detect mutation with high sensitivity without depending on the sequence determination.

Examples of a method for detecting mutation based on the total amount of nucleic acid amplification products include: a method which comprises subjecting a solution after an amplification reaction to deproteinization treatment with a phenol/chloroform (1 : 1) solution, subsequently purifying the aqueous layer directly or after ethanol precipitation using an appropriate purification kit, and measuring the absorbance of the purified solution at a wavelength of 260 nm using a spectrophotometer; a method which comprises subjecting the amplification products to electrophoresis with an agarose gel or a polyacrylamide gel and then detecting mutation with an appropriate probe by a Southern hybridization method ; a method of detecting mutation by a chromatography hybridization method using gold nanoparticles; a method of measuring turbidity of a solution containing amplified nucleic acids; and a method which comprises fluorescently labeling an amplification primer at the 5'-end in advance, performing amplification reaction followed by electrophoresis with an agarose gel or a polyacrylamide gel, subsequently introducing the fluorescence in an imaging plate, and performing detection using an appropriate detection apparatus.

Examples of a method for detecting mutation based on the number of amplification cycles of a nucleic acid amplification reaction required for the total amount of nucleic acid amplification products to reach a threshold value, include real-time PCR methods such as an intercalator method, a TaqManTM probe method, and a cycling probe method. The threshold value can be automatically detected by a real-time PCR apparatus (for example, Step One Plus real-time PCR system, manufactured by Thermo Fisher Scientific, Inc.).

The intercalator method is a method with an intercalating fluorescent dye (also referred to as an intercalator). An intercalator is a reagent that specifically binds between a pair of bases of double-stranded nucleic acids to emit fluorescence, and it emits fluorescence when irradiated with excitation light. Based on detection of the fluorescence intensity originating from an intercalator, the amount of primer extension products can be found. According to the present disclosure, monitoring this fluorescence intensity in real time makes it possible to know the number of amplification cycles of a nucleic acid amplification reaction required for the total amount of nucleic acid amplification products to reach a threshold value. For the present disclosure, any intercalator usually used in this field can be used, and examples thereof include SYBRTM Green I (Molecular Probes, Inc.), ethidium bromide, and fluorene.

The TaqManTM probe method is a method in which a nucleic acid of interest can be quantitatively detected in a trace amount with high sensitivity by a real-time PCR method using an oligonucleotide probe that is labeled with a fluorescent group (reporter) at one end (usually at the 5'-end) and a quenching group at the other end (usually at the 3'-end) and hybridizes to a specific region of a target nucleic acid. With the probe, fluorescence of the reporter is inhibited by the quenching group in a normal state. In a condition in which this fluorescent probe is completely hybridized with a detection region, PCR starts from the outside of the region using DNA polymerase. As an extension reaction by the DNA polymerase proceeds, the fluorescent probe is hydrolyzed by the exonuclease activity of the DNA polymerase, the reporter dye is released, and fluorescence is emitted. According to the present disclosure, monitoring this fluorescence intensity in real time make it possible to know the number of amplification cycles of a nucleic acid amplification reaction required for the total amount of nucleic acid amplification products to reach a threshold value can be known.

The cycling probe method is a highly sensitive detection method with a combination of RNase H and a chimeric probe composed of RNA and DNA. The probe is labeled with a fluorescent substance (reporter) on one side and a substance that quenches fluorescence (quencher) on the other side across the RNA portion. This probe does not emit fluorescence due to quenching in an intact state, but once the sequence forms a hybrid with a complementary amplification product, the RNA portion is cleaved by RNase H and the probe emits strong fluorescence. According to the present disclosure, the total amount of nucleic acid amplification products can be known by measuring this fluorescence intensity, and monitoring the fluorescence intensity in real time makes it possible to know the number of amplification cycles of a nucleic acid amplification reaction required for the total amount of nucleic acid amplification products reaches a threshold value.

In this method, when there is a mismatch in the vicinity of RNA of the cycling probe, cleavage by RNase H does not

occur, and this method therefore makes possible highly specific detection capable of recognizing even a difference in a single base. The cycling probe can also be acquired by entrusting design and synthesis to an appropriate company, or the like.

Examples of a method for detecting mutation based on the amount of nucleic acid having base mutation among nucleic acid amplification products include a Sanger sequencing method and a melting curve analysis method.

Examples of a method for detecting mutation based on the number of amplification cycles of a nucleic acid amplification reaction required for the amount of nucleic acids with base mutation among nucleic acid amplification products reach to a threshold value, include a real-time PCR method with a probe that specifically binds to a nucleic acid with base mutation.

2. Kit for Detecting Mutation in Target Base Sequence

In another embodiment, the present invention provides a kit for detecting a mutation in a target base sequence of a nucleic acid in a sample relative to a standard base sequence comprising:

a clamp nucleic acid having a base sequence complementary to the standard base sequence and containing at least one artificial nucleic acid residue;
an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

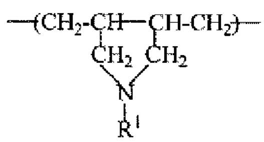
(I-a)

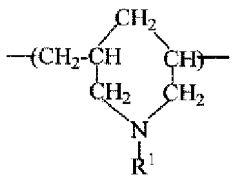
(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

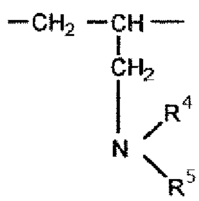
(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

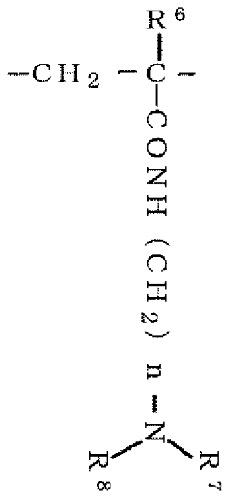

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and
at least some of the anionic constituent units (2) are a constituent unit (2-1), (2-2) or (2-4) represented by general formula (II-a), (II-b) or (II-d):

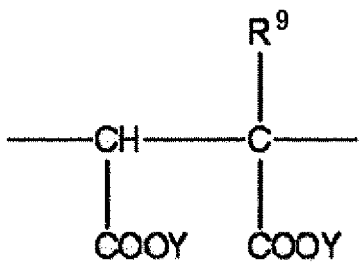

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

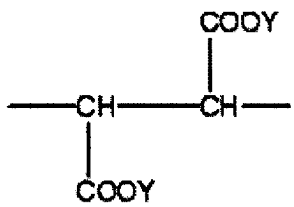

(II-b)

wherein Y represents a hydrogen, Na, K, $NH_4$, 1/2Ca, 1/2Mg, 1/2Fe, 1/3Al, or 1/3Fe independently for each carboxy group to be bonded, or

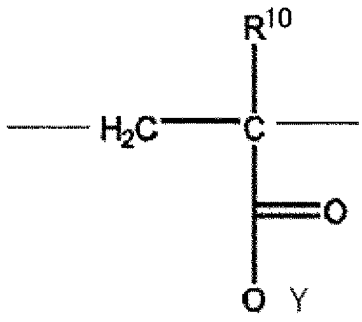

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded;

optionally a primer set capable of amplifying a region containing a target base sequence by a nucleic acid amplification method; and

optionally other reagents for implementing a nucleic acid amplification reaction.

The details of the clamp nucleic acid, the amphoteric copolymer, and the primer set are as described in the method for detecting mutation in a target base sequence, and preferred embodiments and specific examples thereof are the same as those of the method.

Other reagents for implementing a nucleic acid amplification reaction include nucleoside triphosphates, nucleic acid synthases, and buffer solutions for amplification reaction. The nucleoside triphosphates may include substrates according to nucleic acid synthases (dNTP, rNTP, and the like). The nucleic acid synthases are enzymes according to the nucleic acid amplification method conducted with the kit, and examples thereof include DNA polymerase, RNA polymerase, and reverse transcriptase. Examples of the buffer solutions for amplification reaction include buffer solutions used in implementing a conventional nucleic acid amplification reaction or hybridization reaction, such as a Tris buffer solution, a phosphate buffer solution, a veronal buffer solution, a borate buffer solution, and a Good's buffer solution, and the pH thereof is not particularly limited, but a pH in the range of 5 to 9 is usually preferable.

A kit according to the present embodiment may also comprise a reagent for detecting the above-described nucleic acid amplification reaction products. Examples thereof include an intercalator, a fluorescent-labeled probe, and a cycling probe, which are used for real-time PCR.

Further, other components generally used as nucleic acid amplification reaction reagents, such as a stabilizer and a preservative, may also be comprised.

A primer set and other reagents for implementing a nucleic acid amplification reaction are necessary for implementing a nucleic acid amplification reaction, but may not be comprised in the kit. Other components such as a reagent for detecting a nucleic acid amplification reaction product, a stabilizer, and a preservative are optional components for the nucleic acid amplification reactions, and may not be comprised in the kit.

3. Method for Suppressing Amplification of Nucleic Acid Having Predetermined Target Base Sequence in Nucleic Acid Amplification Reaction

In still another embodiment, the present invention provides a method for suppressing amplification of a nucleic acid in a sample having a predetermined target base sequence in a nucleic acid amplification reaction, comprising:

subjecting the nucleic acid in the sample to a nucleic acid amplification reaction as a template with

a clamp nucleic acid having a base sequence complementary to the target base sequence and containing at least one artificial nucleic acid residue, and

an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

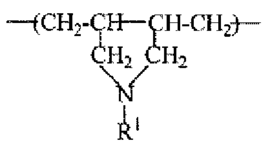

(I-a)

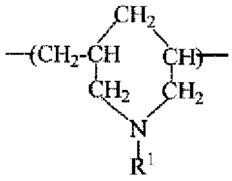

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

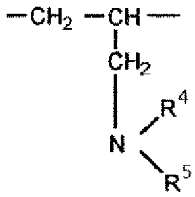

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

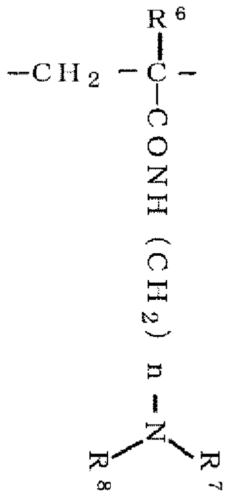

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and
at least some of the anionic constituent units (2) are a constituent unit (2-1), (2-2) or (2-4) represented by general formula (II-a), (II-b) or (II-d):

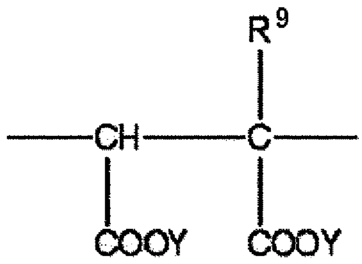

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

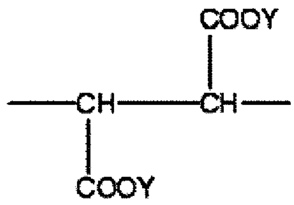

(II-b)

wherein Y represents a hydrogen, Na, K, $NH_4$, 1/2Ca, 1/2Mg, 1/2Fe, 1/3Al, or 1/3Fe independently for each carboxy group to be bonded, or

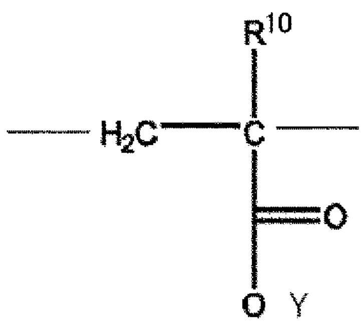

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

In a method according to the present embodiment, a "sample" is assumed to contain a nucleic acid to be subjected to inhibition of amplification, a "target base sequence" is a base sequence to be a subject for inhibition of amplification, and a "nucleic acid in a sample" is a nucleic acid that may contain a base sequence to be a subject for inhibition of amplification. However, these terms do not mean that a "nucleic acid in the sample" actually contains a base sequence to be a subject for inhibition of amplification.

A "clamp nucleic acid" according to the present embodiment has a base sequence complementary to a "target base sequence", and amplification of a nucleic acid having the "target base sequence" is inhibited by the presence of such a "clamp nucleic acid".

According to the present embodiment, in a nucleic acid amplification reaction, the specific amphoteric copolymer coexists with a clamp nucleic acid having a base sequence complementary to a target base sequence, which enhances the effect of inhibiting amplification of a nucleic acid having the target base sequence by the clamp nucleic acid, and selectively inhibit amplification of the nucleic acid even when a large amount of the nucleic acid having the target base sequence is

present in the sample.

Other matters as to a sample and a nucleic acid in the sample are as described in the method for detecting mutation in a target base sequence, and preferred embodiments and specific examples thereof are the same as those of the method. Further, the details of the clamp nucleic acid and the amphoteric copolymer are also as described in the method for detecting mutation in a target base sequence, and preferred embodiments and specific examples thereof are the same as those of the method. Further, the details of implementation of a nucleic acid amplification reaction and reagents for implementing a nucleic acid amplification reaction are also as described in the method and kit for detecting mutation in a target base sequence, and preferred embodiments and specific examples are the same as those of the method and kit. However, in the method of the present embodiment, it is not essential to detect inhibition of amplification of a nucleic acid having a predetermined target base sequence, and steps and reagents necessary for such detection may be implemented or used as necessary.

4. Kit for Suppressing Amplification of Nucleic Acid Having Predetermined Target Base Sequence in Nucleic Acid Amplification Reaction

In still another embodiment, the present invention provides a kit for suppressing amplification of a nucleic acid having a target base sequence in a nucleic acid amplification reaction comprising:

a clamp nucleic acid having a base sequence complementary to the target base sequence and containing at least one artificial nucleotide residue;
an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

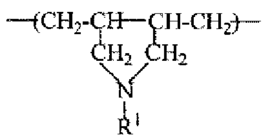
(I-a)

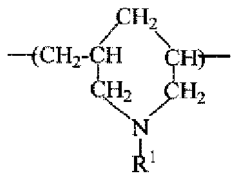
(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

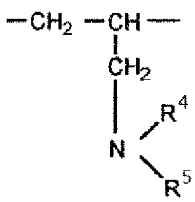
(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

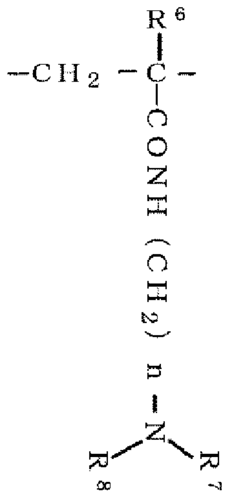

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and

at least some of the anionic constituent units (2) are a constituent unit (2-1), (2-2) or (2-4) represented by general formula (II-a), (II-b) or (II-d):

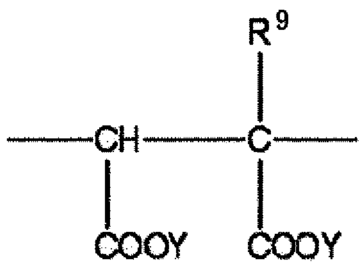

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

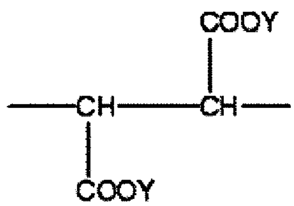

(II-b)

wherein Y represents a hydrogen, Na, K, $NH_4$, 1/2Ca, 1/2Mg, 1/2Fe, 1/3Al, or 1/3Fe independently for each carboxy group to be bonded, or

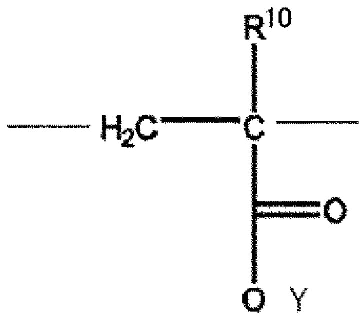

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded;

optionally a primer set capable of amplifying a region containing a target base sequence by a nucleic acid amplification method; and

optionally other reagents for implementing a nucleic acid amplification reaction.

The meanings of the terms "sample", "target base sequence", "nucleic acid in a sample", and "clamp nucleic acid" in the present embodiment are the same as those described in the method for inhibiting amplification of a nucleic acid having a target base sequence in a nucleic acid amplification reaction.

Further, other matters as to a sample and a nucleic acid in the sample are as described in the method for detecting mutation in a target base sequence, and preferred embodiments and specific examples thereof are the same as those of the method. Further, the details of a clamp nucleic acid and the amphoteric copolymer are also as described in the method for detecting mutation in a target base sequence, and preferred embodiments and specific examples thereof are the same as those of the method. Further, the details of implementation of a nucleic acid amplification reaction and the reagents for implementing a nucleic acid amplification reaction are also as described in the method and kit for detecting mutation in a target base sequence, and preferred embodiments and specific examples are the same as those of the method.

5. Use of a Nucleic Acid Amplification Inhibition Enhancing Agent for Enhancing Inhibition of Nucleic Acid Amplification by Clamp Nucleic Acid

In still another embodiment, the present invention provides the use of an agent for enhancing an effect of inhibiting nucleic acid amplification of a nucleic acid having a target base sequence by a clamp nucleic acid that has a base sequence complementary to the target base sequence and contains at least one artificial nucleotide residue, the agent comprising:

an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

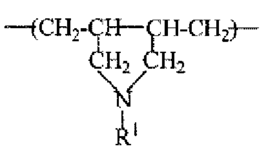

(I-a)

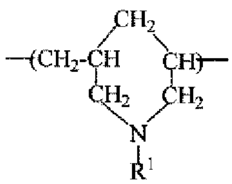

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

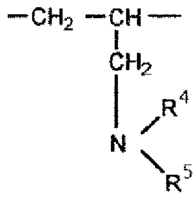

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

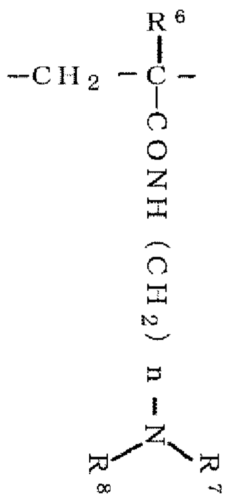

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and

at least some of the anionic constituent units (2) are a constituent unit (2-1), (2-2) or (2-4) represented by general formula (II-a), (II-b) or (II-d):

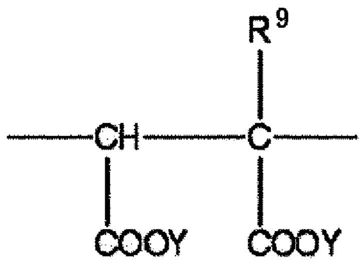

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

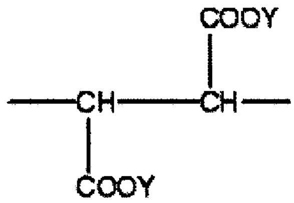

(II-b)

wherein Y represents a hydrogen, Na, K, $NH_4$, 1/2Ca, 1/2Mg, 1/2Fe, 1/3Al, or 1/3Fe independently for each carboxy group to be bonded, or

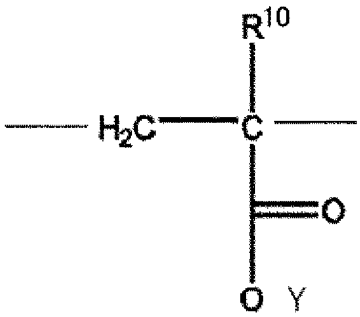

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

The "nucleic acid amplification" implemented with this enhancing agent is performed with "a clamp nucleic acid having a base sequence complementary to a target base sequence and containing at least one artificial nucleotide residue". The meanings of the terms "sample", "target base sequence", "nucleic acid in a sample", and "clamp nucleic acid" according to the present embodiment are the same as those described in the method for inhibiting amplification of a nucleic acid having a target base sequence in a nucleic acid amplification reaction.

Further, the "enhancing agent for an effect of inhibiting by a clamp nucleic acid" according to the present embodiment comprises the amphoteric copolymer. This is also as described in the method for detecting mutation in a target base sequence, and preferred embodiments and specific examples are the same as those of the method.

EXAMPLES

Hereinafter, the present disclosure will be more specifically described by way of Examples, but the present disclosure is not intended to be limited to these.

1. Influence of Addition of Polymer on PCR Reaction

A specific amphoteric copolymer that can be used for the method of the present disclosure (Example product), or a comparative polymer or monomer was added to a PCR reaction mixture, and the influence on PCR reactions was verified.

1-1. Polymer Aqueous Solution Used

Each of the polymers or monomers shown in the following tables was dissolved in water, and a polymer aqueous solution having a pH of 7.0 and a concentration of 10% by mass was prepared. The product names in the table are all those of Nittobo Medical Co., Ltd., and the polymerization conditions for polymers 1 to 18 are as follows.

Polymer 1: Into a 500-mL four-neck flask equipped with a thermometer, a stirrer, and a cooling pipe, 160.07 g (0.80 mol) of 66.78% by mass diallylamine hydrochloride, 78.45 g (0.80 mol) of maleic anhydride, and 91.86 g of distilled

water were charged, and the internal temperature was raised to 50°C. 28.5% by mass of ammonium persulfate aqueous solution was added only in an amount such that the amount of ammonium persulfate in the aqueous solution was 0.5% by mass to the whole amount of monomers, and the polymerization was started. The ammonium persulfate aqueous solution was added in an amount such that the amount of ammonium persulfate was 0.5% by mass with respect to the whole amount of monomers after 4 hours, the amount of ammonium persulfate was 1.0% by mass with respect to the whole amount of monomers after 20 and 26 hours, and the amount of ammonium persulfate was 1.5% by mass with respect to the whole amount of monomers after 45 and 51 hours, and the mixture was reacted for 68 hours.

Polymer 2: Into a 20-L four-neck flask equipped with a thermometer, a stirrer, and a cooling pipe, 1.86 kg (19.0 mol) of maleic anhydride and 0.34 kg of distilled water were charged, and 2.11 kg (19.0 mol) of diallylmethylamine was added dropwise under cooling. After that, the internal temperature was raised to 50°C. A 28.5% by mass ammonium persulfate aqueous solution was added only in an amount such that the amount of ammonium persulfate was 0.5% by mass with respect to the whole amount of monomers, and polymerization was started. After 3, 21, and 25 hours, the ammonium persulfate aqueous solution was added in an amount such that the amount of ammonium persulfate was 1.0 mass% with respect to the whole amount of monomers, and the reaction was further carried out overnight.

Polymer 3: Into a 3-L four-neck flask equipped with a thermometer, a stirrer, and a cooling pipe, 842.93 g of distilled water and 488.34 g (4.98 mol) of maleic anhydride were charged. Thereafter, 342.60 g (6.00 mol) of allylamine was added dropwise while being cooled, and the temperature was raised to 65°C. A 28.5% by mass ammonium persulfate aqueous solution was added in an amount such that the amount of ammonium persulfate was 1 mol% with respect to the whole amount of monomers, and polymerization was started. The ammonium persulfate aqueous solution was added in an amount such that the amount of ammonium persulfate was 1 mol% with respect to the whole amount of monomers after 3 hours, and the amount of ammonium persulfate was 2 mol% with respect to the whole amount of monomers after 24 and 28 hours, and the mixture was further reacted overnight.

Polymer 4: Into a 300-mL four-neck flask equipped with a thermometer, a stirrer, and a cooling pipe, 16.15 (equivalent to 0.04 mol) of dimethylaminopropyl methacrylamide hydrochloride (solid content concentration 51.2% by mass), 2.93 g (equivalent to 0.04 mol) of acrylamide (solid content concentration 97% by mass), 5.82 g (equivalent to 0.08 mol) of acrylic acid (solid content concentration 99% by mass), and 143.86 g of distilled water were charged, and the temperature was raised to 60°C. A 28.5% by mass ammonium persulfate aqueous solution was added every 2 hours in an amount such that the amount of ammonium persulfate was 0.25, 0.5, 0.5, and 0.75 mol%, respectively, with respect to the whole amount of monomers, and the reaction was continued overnight.

Polymer 5: Into a 300-mL four-neck flask equipped with a thermometer, a stirrer, and a cooling pipe, 20.17 g (equivalent to 0.05 mol) of dimethylaminopropyl methacrylamide hydrochloride (solid content concentration 51.2% by mass), 3.66 g (equivalent to 0.05 mol) of acrylamide (solid content concentration 97% by mass), 8.69 g (equivalent to 0.10 mol) of methacrylic acid (solid content concentration 99% by mass), and 192.43 g of distilled water were charged, and the temperature was raised to 60°C. A 28.5% by mass ammonium persulfate aqueous solution was added every 2 hours in an amount such that the amount of ammonium persulfate was 0.25, 0.5, 0.5, and 0.75 mol%, respectively, with respect to the whole amount of monomers, and the reaction was continued overnight.

Polymer 6: Into a 300-mL four-neck flask equipped with a thermometer, a stirrer, and a cooling pipe, 20.43 g (equivalent to 0.1 mol) of diallylamine hydrochloride (solid content concentration 65.40% by mass) and 110.9 g of distilled water were charged, and the temperature was raised to 65°C. A 28.5% by mass ammonium persulfate aqueous solution was added in an amount such that the amount of ammonium persulfate was 2.0 mol% with respect to the whole amount of monomers, subsequently after a lapse of 30 minutes, a solution obtained by mixing 7.11 g (equivalent to 0.1 mol) of acrylamide (solid content concentration 97% by mass), 14.56 g (equivalent to 0.2 mol) of acrylic acid (solid content concentration 99% by mass), and 21.15 g of distilled water was added dropwise over 3 hours, and the reaction was continued overnight.

Polymer 7: Into a 300-mL four-neck flask equipped with a thermometer, a stirrer, and a cooling pipe, 29.15 g (corresponding to 0.30 mol) of diallylamine (concentration 100 mass%), 27.86 g (corresponding to 0.24 mol) of fumaric acid (concentration 100 mass%), and 227.45 g of distilled water were charged, and the temperature was raised to 50°C. 0.57 g (corresponding to 0.0054 mol) of sodium hypophosphite (concentration 100 mass%) was added thereto, and a 28.5% by mass ammonium persulfate aqueous solution was added only in an amount such that the amount of ammonium persulfate was 10% by mass with respect to the whole amount of monomers, the amount being added in 8 divided portions, and polymerization was performed at 50°C.

Polymer 8: Into a 300-mL four-neck flask equipped with a thermometer, a stirrer, and a cooling pipe, 16.15 g (0.04 mol) of 51.2% N-dimethylaminopropyl methacrylamide hydrochloride, 5.86 g (0.08 mol) of 97.0% acrylamide, 5.82 g (0.08 mol) of 99.0% acrylic acid, and 169.36 g of distilled water were charged, and the internal temperature was raised to 60°C. A 28.5% by mass ammonium persulfate aqueous solution was added in an amount such that the amount of ammonium persulfate was 0.25 mol% with respect to the whole amount of monomers, and polymerization was started. The ammonium persulfate aqueous solution was added in an amount such that the amount of ammonium persulfate was 0.5 mol% with respect to the whole amount of monomers after 2 and 4 hours, and the amount of ammonium persulfate was 0.75 mol%

with respect to the whole amount of monomers after 6 hours, and the mixture was reacted for 24 hours.

Polymer 9: Into a 300-mL four-neck flask equipped with a thermometer, a stirrer, and a cooling pipe, 14.13 g (0.035 mol) of 51.2% N-dimethylaminopropyl methacrylamide hydrochloride, 2.56 g (0.035 mol) of 97.0% acrylamide, 10.19 g (0.140 mol) of 99.0% acrylic acid, and 171.23 g of distilled water were charged, and the internal temperature was raised to 60°C. A 28.5% by mass ammonium persulfate aqueous solution was added only in an amount such that the amount of ammonium persulfate was 0.25 mol% with respect to the whole amount of monomers, and polymerization was started. The ammonium persulfate aqueous solution was added in an amount such that the amount of ammonium persulfate was 0.5 mol% with respect to the whole amount of monomers after 2 and 4 hours, and the amount of ammonium persulfate was 0.75 mol% with respect to the whole amount of monomers after 6 hours, and the mixture was reacted for 24 hours.

Polymer 10: Into a 300-mL four-neck flask equipped with a thermometer, a stirrer, and a cooling pipe, 18.17 g (equivalent to 0.05 mol) of dimethylaminopropyl methacrylamide hydrochloride (solid content concentration 51.2% by mass) and 74.86 g of distilled water were charged, and the temperature was raised to 60°C. A 28.5% by mass ammonium persulfate aqueous solution was added every 1 hour only in an amount such that the amount of ammonium persulfate was 0.25, 0.5, 0.5, and 0.75 mol%, respectively, with respect to the whole amount of monomers, and the reaction was continued overnight at 60°C.

Polymer 11: Into a 300-mL four-neck flask equipped with a thermometer, a stirrer, and a cooling pipe, 189.16 g (amount to have a monomer concentration of 10% by mass) of distilled water was charged, and the temperature was raised to 60°C. A 28.5% by mass ammonium persulfate aqueous solution was added only in an amount such that the amount of ammonium persulfate was 3.0% by mass with respect to the whole amount of monomers, subsequently 63.06 g of a 40% by mass acrylic acid aqueous solution was added dropwise over 3 hours, and the reaction was continued overnight at 60°C.

Polymer 12: Into a 1-L four-neck flask equipped with a thermometer, a stirrer, and a cooling pipe, 10.63 g (0.065 mol) of 57.22% by mass allylamine hydrochloride, 253.02 g (1.235 mol) of 65.22% by mass diallylamine hydrochloride, and 31.35 g of distilled water were charged, and the temperature was raised to 60°C. A 28.5% by mass ammonium persulfate aqueous solution was added only in an amount such that the amount of ammonium persulfate was 0.25% by mass with respect to the whole amount of monomers, and polymerization was started. The ammonium persulfate aqueous solution was added in an amount such that the amount of ammonium persulfate was 0.25% by mass with respect to the whole amount of monomers after 3, 5, and 21 hours, and the amount of ammonium persulfate was 0.50% by mass with respect to the whole amount of monomers after 23, 25, 27, and 29 hours, and the mixture was further reacted overnight.

Polymer 13: Into a 300-mL four-neck flask equipped with a thermometer, a stirrer, and a cooling pipe, 124.37 g (0.50 mol) of 65.0% diallyldimethylammonium chloride, 4.44 g (0.06 mol) of acrylamide, 0.85 g of sodium hypophosphite, and 84.39 g of distilled water were charged, and the internal temperature was raised to 50°C. A28.5% by mass ammonium persulfate aqueous solution was added only in an amount such that the amount of ammonium persulfate was 0.2% by mass with respect to the whole amount of monomers, and polymerization was started. The ammonium persulfate aqueous solution was added in an amount such that the amount of ammonium persulfate was 0.3% by mass with respect to the whole amount of monomers after 4 hours, the amount of ammonium persulfate was 0.5% by mass with respect to the whole amount of monomers after 23 hours, and the amount of ammonium persulfate was 1.0% by mass with respect to the whole amount of monomers after 28 hours, and the mixture was reacted for 48 hours.

Polymer 14: Into a 1-L four-neck flask equipped with a thermometer, a stirrer, and a cooling pipe, 209.67 g (1.3 mol) of 58.01% by mass of allylamine hydrochloride and 248.51 g (1.3 mol) of 63.61% by mass of dimethylallylamine hydrochloride were charged, and the temperature was raised to 60°C. Initiator V-50 (2,2'-azobis(2-methylpropionamidine) dihydrochloride) was added in an amount such that the amount of V-50 was 12 mol% with respect to the whole amount of monomers, the amount being added in 3 divided portions, and polymerization was performed for 72 hours. Thereafter, thermal decomposition treatment was performed at 60°C for 48 hours. Thereafter, 449.28 g (2.81 mol) of sodium hydroxide having a concentration of 25% by mass was added under cooling at 30°C or lower, and the mixture was reacted at 40°C for 24 hours. Thereafter, demonomerization (50°C, 3 hours) by an evaporator was performed. After the demonomerization, the concentration was adjusted to 15%, and desalination by electrodialysis (ended in about 3 hours, 1 hour after conductivity completely decreased) was performed.

Polymer 15: Into a 100-ml three-neck flask equipped with a stirrer, a thermometer, and a glass stopper, 25.95 g of distilled water, 0.088 mol of a diallylmethylamine hydrochloride aqueous solution, and 0.011 mol of acrylamide were charged, and the temperature was raised to 55°C. A 28.5% by mass ammonium persulfate aqueous solution was added only in an amount such that the amount of ammonium persulfate was 4.0 mol% with respect to the whole amount of monomers, the amount being added in 7 divided portions, and polymerization was performed at 55°C.

Polymer 16: Into a 100-ml three-neck flask equipped with a stirrer, a thermometer, and a glass stopper, 24.71 g of distilled water, 0.06 mol of a diallylmethylamine hydrochloride aqueous solution, and 0.06 mol of N-isopropylacrylamide were charged, and the temperature was raised to 55°C. A 28.5% by mass ammonium persulfate aqueous solution was added only in an amount such that the amount of ammonium persulfate was 3.25 mol% with respect to the whole amount of

monomers, the amount being added in 7 divided portions, and polymerization was performed at 55°C.

Polymer 17: Into a 300-mL four-neck flask equipped with a thermometer, a stirrer, and a cooling pipe, 32.30 g (equivalent to 0.08 mol) of dimethylaminopropyl methacrylamide hydrochloride (solid content concentration 51.2% by mass), 2.93 g (equivalent to 0.04 mol) of acrylamide (solid content concentration 97% by mass), 2.91 g (equivalent to 0.04 mol) of acrylic acid (solid content concentration 99% by mass), and 184.49 g of distilled water were charged, and the temperature was raised to 60°C. A 28.5% by mass ammonium persulfate aqueous solution was added every 2 hours in an amount such that the amount of ammonium persulfate was 0.25, 0.5, 0.5, and 0.75 mol%, respectively, with respect to the whole amount of monomers, and the reaction was continued overnight.

Polymer 18: Into a 300-mL four-neck flask equipped with a thermometer, a stirrer, and a cooling pipe, 32.30 g (equivalent to 0.08 mol) of dimethylaminopropyl methacrylamide hydrochloride (solid content concentration 51.2% by mass), 2.93 g (equivalent to 0.04 mol) of acrylamide (solid content concentration 97% by mass), 3.44 g (equivalent to 0.04 mol) of methacrylic acid (solid content concentration 99% by mass), and 189.55 g of distilled water were charged, and the temperature was raised to 60°C. A 28.5% by mass ammonium persulfate aqueous solution was added every 2 hours in an amount such that the amount of ammonium persulfate was 0.25, 0.5, 0.5, and 0.75 mol%, respectively, with respect to the whole amount of monomers, and the reaction was continued overnight.

[Table 1-1]

| | Compound name (polymer product name or polymer number) | Cation : anion : nonion | Cation/ anion | Class of cationic amine |
|---|---|---|---|---|
| Example 1 | Copolymer of diallylamine hydrochloride/maleic acid 1 : 1 (polymer 1) | 1:1:0 | 1 | 2 |
| Example 2 | Copolymer of diallylmethylamine/maleic acid 1 : 1 (polymer 2) | 1:1:0 | 1 | 3 |
| Example 3 | Copolymer of allylamine/maleic acid 1.2 : 1 (polymer 3) | 1.2:1:0 | 1.2 | 1 |
| Example 4 | Copolymer of dimethylaminopropyl methacrylamide hydrochloride/acrylamide/acrylic acid 1 : 1 : 2 (polymer 4) | 1:2:1 | 0.5 | 3 |
| Example 5 | Copolymer of dimethylaminopropyl methacrylamide hydrochloride/acrylamide/ methacrylic acid 1 : 1 : 2 (polymer 5) | 1:2:1 | 0.5 | 3 |
| Example 6 | Copolymer of diallylamine hydrochloride/ acrylamide/acrylic acid 1 : 1 : 2 (polymer 6) | 1:2:1 | 0.5 | 2 |
| Example 7 | Copolymer of diallylamine/fumaric acid 1 : 0.8 added with 1 mol% sodium hypophosphite (polymer 7) | 1:0.8 | 1.25 | 2 |
| Example 8 | Copolymer of dimethylaminopropyl methacrylamide hydrochloride/acrylamide/acrylic acid 1 : 2 : 2 (polymer 8) | 1:2:2 | 0.5 | 3 |
| Example 9 | Copolymer of dimethylaminopropyl methacrylamide hydrochloride/acrylamide/acrylic acid 1 : 1 : 4 (polymer 9) | 1:4:1 | 0.25 | 3 |
| Comparative Example 1 | Polydimethylaminopropyl methacrylamide hydrochloride (polymer 10) | 1:0:0: | | 3 |
| Comparative Example 2 | Polydiallylamine (PAS-21) | 1:0:0 | | 2 |
| Comparative Example 3 | Polydiallylmethylamine (PAS-M-1) | 1:0:0 | | 3 |
| Comparative Example 4 | Polydiallyldimethylammonium chloride (PAS-H-5L) | 1:0:0 | | 4 |
| Comparative Example 5 | Polyacrylic acid (polymer 11) | 0:1:0 | | |
| Comparative Example 6 | Copolymer of allylamine hydrochloride/ diallylamine hydrochloride 1 : 19 (polymer 12) | (1:19):0:0 | | 2 |

[Table 1-2]

| | | | | |
|---|---|---|---|---|
| Comparative Example 7 | Copolymer of diallyldimethylammonium chloride/ acrylamide 8 : 1 (polymer 13) | 8:0:1 | | 4 |
| Comparative Example 8 | Copolymer of allylamine/dimethylallylamine 1 : 1 (polymer 14) | (1:1):0:0 | | |
| Comparative Example 9 | Copolymer of diallylmethylamine hydrochloride/ acrylamide 8 : 1 (polymer 15) | 8:0:1 | | 3 |
| Comparative Example 10 | Copolymer of diallylmethylamine hydrochloride/ N-isopropylacrylamide 1 : 1 (polymer 16) | 1:0:1 | | 3 |
| Comparative Example 11 | Copolymer of dimethylaminopropyl methacrylamide hydrochloride/acrylamide/acrylic acid 2 : 1 : 1 (polymer 17) | 2:1:1 | 2 | 3 |
| Comparative Example 12 | Copolymer of dimethylaminopropyl methacrylamide hydrochloride/acrylamide/ methacrylic acid 2 : 1 : 1 (polymer 18) | 2:1:1 | 2 | 3 |
| Comparative Example 13 | Diallylmethylamine hydrochloride | | | |
| Comparative Example 14 | Diallylamine hydrochloride | | | |

1-2. PCR Reaction Mixture

A PCR reaction mixture having the composition shown in the following table was prepared.

[Table 2]

| Component | Content |
|---|---|
| 2×PCR Buffer TB Green Premix Dimer Eraser*1 (manufactured by Takara Bio, Inc.) | 10.0μL |
| 10 μM Forward primer (synthesis entrusted to Fasmac Co., Ltd.) | 0.6μL |
| 10 μM Reverse primer (synthesis entrusted to Fasmac Co., Ltd.) | 0.6μL |
| 50×ROX dye (manufactured by Takara Bio, Inc.) | 0.4μL |
| 50 ng/μL template DNA | 1.0μL |
| Polymer aqueous solution | 2.0μL |
| Nuclease-free water (manufactured by Thermo Fisher Scientific, Inc.) | 5.4μL |
| *1 contains Taq polymerase (TaKaRa Ex Taq HS), dNTP Mixture, $Mg^{2+}$, and a fluorescent dye (TB Green). The final concentration of each polymer aqueous solution in the PCR reaction mixture was 0.01% by mass. | |

1-3. Primers and Template DNA

The sequences of a forward primer and a reverse primer used in the PCR reaction are as shown in the following table.

[Table 3]

| | |
|---|---|
| Forward primer | GCCTGCTGAAAATGACTGAATATA (SEQ ID NO:1) |
| Reverse primer | CAAGATTTACCTCTATTGTTGGA (SEQ ID NO:2) |

Each primer was designed using Primer3 (obtained from http://frodo.wi.mit.edu) to target the KRAS gene (the base sequence of the gene can be found at, for example, NCBI (DB name)).

As a template DNA, a genome extracted from HCC70 cells containing the following base sequence (sequence to be amplified) was used.

GCCTGCTGAAAATGACTGAATATAAACTTGTGGTAGTTGGAGCTGGTGGCGTAGGCAAGAGTGCCTTGACGATAC
AGCTAATTCAGAATCATTTTGTGGACGAATATGATCCAACAATAGAGGTAAATCTTG (SEQ ID NO:3)

The genome of HCC70 cells which contains the above-described base sequence, and a base sequence adjacent thereto is as follows.

TGAGTTTGTATTAAAAGGTACTGGTGGAGTATTTGATAGTGTATTAACCTTATGTGTGACATGTTCTAATATAGT
CACATTTTCATTATTTTTATTATAAGGCCTGCTGAAAATGACTGAATATAAACTTGTGGTAGTTGGAGCTGGTGG
CGTAGGCAAGAGTGCCTTGACGATACAGCTAATTCAGAATCATTTTGTGGACGAATATGATCCAACAATAGAGGT
AAATCTTGTTTTAATATGCATATTACTGGTGCAGGACCATTCTTTGATACACGATAAAGGTTTCTCTGACCATTT
(SEQ ID NO:4)

1-4. Real-Time PCR System

A Step One Plus real-time PCR system (Thermo Fisher Scientific, Inc.) was used.

1-5. PCR Reaction

20 μL of the prepared PCR reaction mixture was loaded in a real-time PCR system, and after performing step (i) described in the following table, 40 cycles of steps (ii) to (iv) were repeated.

[Table 4]

| Step | Temperature | Time |
|---|---|---|
| i | 95°C | 30 seconds |
| ii | 95°C | 5 seconds |
| iii | 55°C | 30 seconds |
| iv | 72°C | 30 seconds |
| v | 95°C | 15 seconds |
| vi | 60°C | 60 seconds |
| vii | 95°C | 15 seconds |

After completion of 40 cycles, steps (v) to (vii) were performed, and the reaction was terminated. The amount of DNA amplification was monitored by measuring the fluorescence intensity of the reaction mixture for each cycle. Further, after completion of the reaction, whether the PCR reaction was inhibited was determined based on whether the fluorescence intensity reached the Threshold Line. The Threshold Line was automatically calculated by the above-described apparatus. In the case where the Threshold Line was unavoidably set to too low in some experiments, the ΔRn value was manually set in the range between 0.7 and 1.2 in order to ensure a comparison with other experiments on the same level.

1-5. Results

The success or failure of the PCR reaction is shown in the following table.

[Table 5-1]

| | Polymer or monomer | Cation : anion : nonion | Cation/ anion | Success or failure of PCR reaction (○/×) |
|---|---|---|---|---|
| Example 1 | Copolymer of diallylamine hydrochloride/maleic acid 1 : 1 (polymer 1) | 1:1:0 | 1 | ○ |
| Example 2 | Copolymer of diallylmethylamine/maleic acid 1 : 1 (polymer 2) | 1:1:0 | 1 | ○ |
| Example 3 | Copolymer of allylamine/malein 1.2 : 1 (polymer 3) | 1.2:1:0 | 1.2 | ○ |

(continued)

| | Polymer or monomer | Cation : anion : nonion | Cation/ anion | Success or failure of PCR reaction (○/×) |
|---|---|---|---|---|
| Example 4 | Copolymer of dimethylaminopropyl methacrylamide hydrochloride/ acrylamide/acrylic acid 1 : 1 : 2 (polymer 4) | 1:2:1 | 0.5 | ○ |
| Example 5 | Copolymer of dimethylaminopropyl methacrylamide hydrochloride/acrylamide/ methacrylic acid 1 : 1 : 2 (polymer 5) | 1:2:1 | 0.5 | ○ |
| Example 6 | Copolymer of diallylamine hydrochloride/ acrylamide/acrylic acid 1 : 1 : 2 (polymer 6) | 1:2:1 | 0.5 | ○ |
| Example 7 | Copolymer of diallylamine/fumaric acid 1 : 0.8 added with 1 mol% sodium hypophosphite (polymer 7) | 1:0.8 | 1.25 | ○ |
| Example 8 | Copolymer of dimethylaminopropyl methacrylamide hydrochloride/acrylamide/ acrylic acid 1 : 2 : 2 (polymer 8) | 1:2:2 | 0.5 | ○ |
| Example 9 | Copolymer of dimethylaminopropyl methacrylamide hydrochloride/acrylamide/ acrylic acid 1 : 1 : 4 (polymer 9) | 1:4:1 | 0.25 | ○ |
| Comparative Example 1 | Polydimethylaminopropyl methacrylamide hydrochloride (polymer 10) | 1:0:0: | | × |
| Comparative Example 2 | Polydiallylamine (PAS-21) | 1:0:0 | | × |
| Comparative Example 3 | Polydiallylmethylamine (PAS-M-1) | 1:0:0 | | × |
| Comparative Example 4 | Polydiallyldimethylammonium chloride (PAS-H-5L) | 1:0:0 | | × |
| Comparative Example 5 | Polyacrylic acid (polymer 11) | 0:1:0 | | × |

[Table 5-2]

| | | | | |
|---|---|---|---|---|
| Comparative Example 6 | Copolymer of allylamine hydrochloride/ diallylamine hydrochloride 1 : 19 (polymer 12) | (1:9):0:0 | | × |
| Comparative Example 7 | Copolymer of diallyldimethylammonium chloride/acrylamide 8 : 1 (polymer 13) | 8:0:1 | | × |
| Comparative Example 8 | Copolymer of allylamine/ N,N-dimethylallylamine 1 : 1 (polymer 14) | (1:1):0:0 | | × |
| Comparative Example 9 | Copolymer of diallylmethylamine hydrochloride/acrylamide 8 : 1 (polymer 15) | 8:0:1 | | × |
| Comparative Example 10 | Copolymer of diallylmethylamine hydrochloride/ N-isopropylacrylamide 1 : 1 (polymer 16) | 1:0:1 | | × |
| Comparative Example 11 | Copolymer of dimethylaminopropyl methacrylamide hydrochloride/acrylamide/ acrylic acid 2 : 1 : 1 (polymer 17) | 2:1:1 | 2 | × |
| Comparative Example 12 | Copolymer of dimethylaminopropyl methacrylamide hydrochloride/acrylamide/ methacrylic acid 2 : 1 : 1 (polymer 18) | 2:1:1 | 2 | × |
| Comparative Example 13 | Diallylmethylamine hydrochloride | | | ○ |

(continued)

| | | |
|---|---|---|
| Comparative Example 14 | Diallylamine hydrochloride | ○ |
| O: Did not inhibit PCR reaction. ×: Inhibited PCR reaction. | | |

An example of a nucleic acid amplification curve in the case of not inhibiting the PCR reaction (copolymer of diallylmethylamine/maleic acid 1 : 1 of Example 2) and an example of the nucleic acid amplification curve in the case of inhibiting the PCR reaction (copolymer of diallyldimethylammonium chloride/acrylamide 8 : 1 of Comparative Example 7) are respectively shown in FIG. 1.

As described above, when each of the polymers of Example 1 to 9, which is an amphoteric copolymers having a cationic constituent unit containing amine in the structure and an anionic constituent unit and having a cationic constituent unit/anionic constituent unit in the range of 0.25 to 1.25, was added, or when the monomer of Comparative Example 13 or 14 was added, the PCR reaction was not inhibited.

2. Polymer-Induced Enhancement of Effect of Selectively Inhibiting Amplification of Wild-Type Gene by BNA Clamp

The polymers of Examples 1 to 9 and the monomers of Comparative Examples 13 and 14, which were verified not to inhibit the PCR reaction in Test 1, were evaluated for the enhancement of the effect of inhibiting amplification of the wild-type KRAS gene in BNA clamp PCR.

2-1. Polymer or Monomer Used

The polymers of Examples 1 to 9 and the monomers of Comparative Examples 13 and 14 were used. Each polymer or monomer was dissolved in nuclease-free water, and an aqueous solution of the polymer or monomer having a concentration of 10% at pH of 7.0 was prepared and used.

Each of the obtained polymer aqueous solutions or monomer aqueous solutions having a concentration of 10% was diluted to a concentration in a range that would not inhibit the PCR reaction from the results of Test 1. The following table shows the concentration of each polymer aqueous solution or monomer aqueous solution used in the present test and the concentration of the polymer or monomer in the reaction mixture.

[Table 6]

| Polymer or monomer | Concentration of solution of polymer or monomer (% by mass) | Concentration of polymer or monomer in reaction mixture (% by mass) |
|---|---|---|
| Example 1 (copolymer of diallylamine hydrochloride/maleic acid 1 : 1) | 0.25 | 0.025 |
| Example 2 (copolymer of diallylmethylamine/maleic acid 1 : 1) | 0.50 | 0.050 |
| Example 3 (copolymer of allylamine/ maleic acid 1.2 : 1) | 0.10 | 0.010 |
| Example 4 (copolymer of dimethylaminopropyl methacrylamide hydrochloride/acrylamide/acrylic acid 1 : 1 :2) | 1.00 | 0.100 |
| Example 5 (copolymer of dimethylaminopropyl methacrylamide hydrochloride/acrylamide/-methacrylic acid 1 : 1 : 2) | 0.10 | 0.010 |
| Example 6 (copolymer of diallylamine hydrochloride/acrylamide/acrylic acid 1 : 1 : 2) | 0.50 | 0.050 |
| Example 7 (copolymer of diallylamine/ fumaric acid 1 : 0.8 added with 1 mol% sodium hypophosphite) | 1.00 | 0.100 |
| Example 8 (copolymer of dimethylaminopropyl methacrylamide hydrochloride/acrylamide/acrylic acid 1 : 2 : 2) | 1.00 | 0.100 |

(continued)

| Polymer or monomer | Concentration of solution of polymer or monomer (% by mass) | Concentration of polymer or monomer in reaction mixture (% by mass) |
|---|---|---|
| Example 9 (copolymer of dimethylaminopropyl methacrylamide hydrochloride/acrylamide/acrylic acid 1 : 1 : 4) | 1.00 | 0.100 |
| Comparative Example 13 (diallylmethylamine hydrochloride) | 1.00 | 0.100 |
| Comparative Example 14 (diallylamine hydrochloride) | 1.00 | 0.100 |

2-2. Kit Used

Appendages of BNA Clamp KRAS Enrichment Kit (Riken Genesis Co., Ltd.), which is a kit for detecting a mutant-type KRAS gene, were used. The details will be described below, but the kit appendages were used as the primer set and the BNA clamp. The reaction conditions for real-time PCR were in accordance with the kit protocol.

2-3. Primers, BNA Clamp, and Template DNA

The forward primer, reverse primer and BNA clamp shown in the following table were used.

[Table 7]

| | |
|---|---|
| Forward primer | CTGAATATAAACTTGTGGTAGTTGG (SEQ ID NO:5) |
| Reverse primer | GTCCTGCACCAGTAATATGC (SEQ ID NO:6) |
| BNA clamp | C*C*T*AC*GC*C*AC*C* (SEQ ID NO:7) |
| *: Indicates a base of artificial nucleotide. | |

Further, as a template DNA having a wild-type KRAS gene containing a standard base sequence, a genome extracted from HCC70 cells containing the following base sequence (sequence to be amplified; bases different from those of the base sequence of SEQ ID NO: 10 that will be described below are shown in underlined bold letters) was used.

CTGAATATAAACTTGTGGTAGTTGGAGCTGGTG**G**CGTAGGCAAGAGTGCCTTGACGATACAGCTAATTCAGAATC
ATTTTGTGGACGAATATGATCCAACAATAGAGGTAAATCTTGTTTTAATATGCATATTACTGGTGCAGGAC
(SEQ ID NO:8)

In the genome of HCC70 cells, the above-described base sequence and a base sequence adjacent thereto are as follows (bases different from those of the base sequence of SEQ ID NO:11 that will be described later are shown in underlined bold letters).

TGAGTTTGTATTAAAAGGTACTGGTGGAGTATTTGATAGTGTATTAACCTTATGTGTGACATGTTCTAATATAGT
CACATTTTCATTATTTTTATTATAAGGCCTGCTGAAAATGACTGAATATAAACTTGTGGTAGTTGGAGCTGGTG**G**
CGTAGGCAAGAGTGCCTTGACGATACAGCTAATTCAGAATCATTTTGTGGACGAATATGATCCAACAATAGAGGT
AAATCTTGTTTTAATATGCATATTACTGGTGCAGGACCATTCTTTGATACACGATAAAGGTTTCTCTGACCATTT
(SEQ ID NO:9)

On the other hand, as a template DNA having a mutation relative to a standard base sequence, a genome of MDA-MB-231 cells having a mutant-type KRAS gene, which contains the following base sequence (sequence to be amplified; bases different from those of the base sequence of SEQ ID NO:8 described above are shown in underlined bold letters), was used.

CTGAATATAAACTTGTGGTAGTTGGAGCTGGTG**A**CGTAGGCAAGAGTGCCTTGACGATACAGCTAATTCAGAATC
ATTTTGTGGACGAATATGATCCAACAATAGAGGTAAATCTTGTTTTAATATGCATATTACTGGTGCAGGAC
(SEQ ID NO:10)

In the genome of MDA-MB-231 cell, the above-described base sequence and a base sequence adjacent thereto are as follows (bases different from those of the base sequence of SEQ ID NO:9 are shown in underlined bold letters).

TGAGTTTGTATTAAAAGGTACTGGTGGAGTATTTGATAGTGTATTAACCTTATGTGTGACATGTTCTAATATAGT
CACATTTTCATTATTTTTATTATAAGGCCTGCTGAAAATGACTGAATATAAACTTGTGGTAGTTGGAGCTGGTG**A**
CGTAGGCAAGAGTGCCTTGACGATACAGCTAATTCAGAATCATTTTGTGGACGAATATGATCCAACAATAGAGGT
AAATCTTGTTTTAATATGCATATTACTGGTGCAGGACCATTCTTTGATACACGATAAAGGTTTCTCTGACCATTT
(SEQ ID NO:11)

2-4. BNA Clamp PCR Reaction Mixture

Reaction mixtures 1 to 4 having the compositions shown in the following table were prepared. In the reaction mixture 1 or the reaction mixture 2, the concentration of the polymer or the monomer was 0.025% by mass. In the reaction mixture 1 or the reaction mixture 2, the concentration of the polymer or the monomer was as shown in the above Table 6.

[Table 8]

| Reaction mixture 1 (polymer or monomer +, BNA clamp +) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0.2μL |
| Template DNA (50 ng/μL genomic DNA of HCC70 cells, or 50 ng/μL genomic DNA of MDA-MB-231 cells) | 1.0μL |
| 0.25% by mass polymer aqueous solution or monomer aqueous solution | 2.0μL |
| Nuclease-free water (manufactured by Thermo Fisher Scientific, Inc.) | 5.2μL |
| *1 contains Taq polymerase (TaKaRa Ex Taq HS), dNTP Mixture, $Mg^{2-}$, and a fluorescent dye (TB Green). | |

[Table 9]

| Reaction mixture 2 (polymer or monomer +, BNA clamp -) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0μL |
| Template DNA (50 ng/μL genomic DNA of HCC70 cells, or 50 ng/μL genomic DNA of MDA-MB-231 cells) | 1.0μL |
| 0.25% by mass polymer aqueous solution or monomer aqueous solution | 2.0μL |

(continued)

| Reaction mixture 2 (polymer or monomer +, BNA clamp -) | |
|---|---|
| Component | Content |
| Nuclease-free water (manufactured by Thermo Fisher Scientific, Inc.) | 5.4μL |
| *1 contains Taq polymerase (TaKaRa Ex Taq HS), dNTP Mixture, $Mg^{2+}$, and a fluorescent dye (TB Green). | |

[Table 10]

| Reaction mixture 3 (polymer or monomer -, BNA clamp +) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0.2μL |
| Template DNA (50 ng/μL genomic DNA of HCC70 cells, or 50 ng/μL genomic DNA of MDA-MB-231 cells) | 1.0μL |
| 0.25% by mass polymer aqueous solution or monomer aqueous solution | 0μL |
| Nuclease-free water (manufactured by Thermo Fisher Scientific, Inc.) | 7.2μL |
| *1 contains Taq polymerase (TaKaRa Ex Taq HS), dNTP Mixture, $Mg^{2+}$, and a fluorescent dye (TB Green). | |

[Table 11]

| Reaction mixture 4 (polymer or monomer -, BNA clamp -) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0μL |
| Template DNA (50 ng/μL genomic DNA of HCC70 cells, or 50 ng/μL genomic DNA of MDA-MB-231 cells) | 1.0μL |
| 0.25% by mass polymer aqueous solution or monomer aqueous solution | 0μL |
| Nuclease-free water (manufactured by Thermo Fisher Scientific, Inc.) | 7.4μL |
| *1 contains Taq polymerase (TaKaRa Ex Taq HS), dNTP Mixture, $Mg^{2+}$, and a fluorescent dye (TB Green). | |

2-5. Real-Time PCR System

A Step One Plus real-time PCR system (Thermo Fisher Scientific, Inc.) was used.

2-6. PCR Reaction

20 μL of each of the prepared reaction mixtures was weighed in a PCR tube, the PCR tube was set in a real-time PCR system, step (i) described in the following table was performed, and then 50 cycles of steps (ii) to (iv) were repeated. After completion of 50 cycles, steps (v) to (vii) were performed, and the reaction was terminated. The amount of DNA amplification was monitored by measuring the fluorescence intensity of the reaction mixture for each cycle, and a growth curve of each reaction mixture was obtained.

[Table 12]

| Step | Temperature | Time |
|---|---|---|
| i | 95°C | 30 seconds |
| ii | 95°C | 5 seconds |
| iii | 58°C | 30 seconds |
| iv | 72°C | 30 seconds |
| v | 95°C | 15 seconds |
| vi | 60°C | 60 seconds |
| vii | 95°C | 15 seconds |

2-7. Calculation of ΔCt (WT) and ΔCt (Mutant) with or without addition of polymer, calculation of ΔΔCt with or without addition of polymer, and calculation of ΔΔΔCt

Reaction mixture 1 (with addition of polymer, with addition of BNA clamp), reaction mixture 2 (with addition of polymer, without addition of BNA clamp), reaction mixture 3 (without addition of polymer, with addition of BNA clamp), and reaction mixture 4 (without addition of polymer, without addition of BNA clamp) were subjected to the above-described PCR reaction using the wild-type KRAS gene or a mutant-type KRAS gene as a template DNA, to obtain amplification curves, and from the amplification curve under each condition, each Ct value was obtained from the set Threshold Line (in most experimental sections, the Threshold Line was automatically calculated by the apparatus). In the case where the Threshold Line was unavoidably set to too low in some experiments, the ΔRn value was manually set in the range between 0.7 and 1.2 in order to ensure a comparison with other experiments on the same level.

Next, ΔCt (WT) and ΔCt (Mutant) in the case of no addition of polymer were calculated by the following formulas.

ΔCt (WT) in case of no addition of polymer = Ct (WT) in case of reaction mixture 3 (without addition of polymer, with addition of BNA clamp) - Ct (WT) in case of reaction mixture 4 (without addition of polymer, without addition of BNA clamp)

ΔCt (Mutant) in case of no addition of polymer = Ct (Mutant) in case of reaction mixture 3 (without addition of polymer, with addition of BNA clamp) - Ct (Mutant) in case of reaction mixture 4 (without addition of polymer, without addition of BNA clamp)

Further, in order to verify that there is no non-specific amplification in the intercalator method, ΔΔCt in the case of no addition of polymer was calculated by the following formula. ΔΔCt in case of no addition of polymer = ΔCt (WT) in case of no addition of polymer - ΔCt (Mutant) in case of no addition of polymer

It is understood that there is no non-specific amplification in the intercalator method when ΔΔCt > 0 in the case of no addition of polymer.

Next, ΔCt (WT) and ΔCt (Mutant) in the case of addition of polymer were calculated by the following formulas, and further, ΔΔCt in the case of addition of polymer was calculated in order to verify that there is no non-specific amplification in the intercalator method.

ΔCt (WT) in case of addition of polymer = Ct (WT) in case of reaction mixture 1 (with addition of polymer, with addition of BNA clamp) - Ct (WT) in case of reaction mixture 2 (with addition of polymer, without addition of BNA clamp)

ΔCt (Mutant) in case of addition of polymer = Ct (Mutant) in case of reaction mixture 1 (with addition of polymer, with addition of BNA clamp) - Ct (Mutant) in case of reaction mixture 2 (with addition of polymer, without addition of BNA clamp)

ΔΔCt in case of addition of polymer = ΔCt (WT) in case of addition of polymer - ΔCt (Mutant) in case of addition of polymer

It is understood that when ΔΔCt > 0 in the case of addition of polymer, there is no non-specific amplification in the intercalator method.

Finally, as an index for evaluating the enhancement of the effect of selectively inhibiting amplification of a wild-type gene by the BNA clamp brought by addition of polymer, ΔΔΔCt was calculated by the following formula.

$$\Delta\Delta\Delta Ct = \Delta\Delta Ct \text{ in case of addition of polymer - } \Delta\Delta Ct \text{ in case of no addition of polymer}$$

Even in consideration of fluctuations in the results that can occur in the test, when ΔΔΔCt ≥ 1.00 in the case of using a mutation detection method based on the number of amplification cycles of a wild-type gene, such as an intercalator method, it can be evaluated that the effect of selectively inhibiting amplification of the wild-type gene by a BNA clamp was sufficiently enhanced. Further, when ΔΔΔCt ≥ 3.20 in the case of using a mutation detection method based on the number of amplification cycles of a wild-type gene, such as an intercalator method, it can be evaluated that the effect of selectively inhibiting amplification of the wild-type gene by the BNA clamp was remarkably enhanced. ΔΔΔCt = 3.20 is an indication that the sensitivity is improved by about 10 times.

2-8. Results

The test results are summarized below.

[Table 13]

| Polymer or monomer | Evaluation of ΔΔΔCt |
|---|---|
| Example 1 (copolymer of diallylamine hydrochloride/maleic acid 1 : 1) | A |
| Example 2 (copolymer0 of diallylmethylamine/maleic acid 1 : 1) | A |
| Example 3 (copolymer of allylamine/maleic acid 1.2 : 1) | B |
| Example 4 (copolymer of dimethylaminopropyl methacrylamide hydrochloride/acrylamide/acrylic acid 1 : 1 : 2) | A |
| Example 5 (copolymer of dimethylaminopropyl methacrylamide hydrochloride/acrylamide/-methacrylic acid 1 : 1 : 2) | A |
| Example 6 (copolymer of diallylamine hydrochloride/acrylamide/ acrylic acid 1 : 1 : 2) | A |
| Example 7 (copolymer of diallylamine/fumaric acid 1 : 0.8 added with 1 mol% sodium hypophosphite) | A |
| Example 8 (copolymer of dimethylaminopropyl methacrylamide hydrochloride/acrylamide/acrylic acid 1 : 2 : 2) | A |
| Example 9 (copolymer of dimethylaminopropyl methacrylamide hydrochloride/acrylamide/acrylic acid 1 : 1 : 4) | A |
| Comparative Example 13 (diallylmethylamine hydrochloride) | C |
| Comparative Example 14 (diallylamine hydrochloride) | C |
| A: ΔΔΔCt is 3.20 or more, B: ΔΔΔCt is 1.00 or more and less than 3.20, C: ΔΔΔ is less than 1.00. | |

From the above-described test results, it became clear that when the polymers of Examples 1 to 9 were added, the effect of selectively inhibiting amplification of the wild-type gene by the BNA clamp was enhanced without inhibiting the amplification of the mutant gene. Further, it became clear that the polymers of Examples 1, 2, and 4 to 9 (amphoteric copolymers in which the cationic constituent unit has a secondary amino group or a tertiary amino group) have a greater effect of enhancing the inhibitory effect on nucleic acid amplification by the clamp nucleic acid than the polymer of Example 3 (amphoteric copolymer in which the cationic constituent unit has a primary amino group). FIG. 2 shows a graph of an example of enhancing the effect of selectively inhibiting amplification of the wild-type gene in this way.

3. Sensitivity Enhancement of BNA Clamp PCR by Addition of Polymer

An attempt was made to increase the sensitivity of BNA clamp PCR by using the polymers of Examples 1, 2, 4, and 6, in which an effect of enhancing the effect of selectively inhibiting amplification of the wild-type gene by a BNA clamp had been verified in the above-described test.

3-1. Polymer Used

The polymers of Examples 1, 2, 4, and 6 were used. The concentration of each polymer solution and the final concentration in the PCR reaction mixture are shown in the following table.

[Table 14]

| Polymer | Concentration of polymer solution (% by mass) | Concentration of polymer in reaction mixture (% by mass) |
|---|---|---|
| Example 1 (copolymer of diallylamine hydrochloride/maleic acid 1 : 1) | 0.25 | 0.025 |
| Example 2 (copolymer of diallylmethylamine/ maleic acid 1 : 1) | 0.50 | 0.050 |
| Example 4 (copolymer of dimethylaminopropyl methacrylamide hydrochloride/acrylamide/acrylic acid 1 : 1 : 2) | 1.00 | 0.100 |
| Example 6 (copolymer of diallylamine hydrochloride/acrylamide/acrylic acid 1 : 1 : 2) | 0.50 | 0.050 |

3-2. Kit Used

The appendages of the same kit as that in Test 2 were used.

3-3. Template DNA

As a template DNA having a standard base sequence, a genome of HCC70 cells having a wild-type KRAS gene containing the base sequence of SEQ ID NO:8, was used. Further, as a template DNA having a mutation relative to the standard base sequence, a genome of MDA-MB-231 cells having a mutant-type KRAS gene containing the base sequence of SEQ ID NO:10, was used.

In the present test, the template DNA of the wild-type gene and the template DNA of the mutant gene were mixed such that the % by mass of the template DNA of the mutant gene in the whole template DNAs was as shown in the following table while the whole amount of the template DNAs was adjusted to 50 ng in 2.0 μL, and each of the obtained template DNA mixtures and the template DNA of the wild-type gene were used in the PCR reaction.

[Table 15]

| Mutant ratio | Number of Mutant copies |
|---|---|
| 10% Mutant(5ng) | $1.65 \times 10^3$ copies |
| 1% Mutant(0.5ng) | $1.65 \times 10^2$ copies |
| 0.1% Mutant(0.05ng) | $1.65 \times 10$ copies |
| 0.01% Mutant(0.005ng) | 1.65 copies |

3-4. Primers and BNA Clamp

The same primers and BNA clamp as those used in Test 2 were used.

3-5. BNA Clamp PCR Reaction Mixture

Reaction mixtures 1 and 2 having compositions as shown in the following table were prepared. In the reaction mixture 1, the concentration of the polymer was as shown in the Table 14.

[Table 16]

| Reaction mixture 1 (polymer +, BNA clamp +) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0.2μL |
| 50 ng of template DNA (WT*2 or mixture*4 of WT and Mutant*3) | 2.0μL |
| 0.1% by mass polymer aqueous solution | 2.0μL |
| Nuclease-free water (manufactured by Thermo Fisher Scientific, Inc.) | 4.2μL |
| *1 contains Taq polymerase (TaKaRa Ex Taq HS), dNTP Mixture, $Mg^{2-}$, and a fluorescent dye (TB Green).<br>*2 Genomic DNA of HCC70 cells<br>*3 Genomic DNA of MDA-MB-231 cells<br>*4 contains 50 ng of the template DNA in 2.0 μL of whole mixture. | |

[Table 17]

| Reaction mixture 2 (polymer -, BNA clamp +) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0.2μL |
| 50 ng of template DNA (WT*2 or mixture*4 of WT and Mutant*3) | 2.0μL |
| Polymer aqueous solution | 0μL |
| Nuclease-free water (manufactured by Thermo Fisher Scientific, Inc.) | 6.2μL*2 |
| *1 contains Taq polymerase (TaKaRa Ex Taq HS), dNTP Mixture, $Mg^{2-}$, and a fluorescent dye (TB Green).<br>*2 Genomic DNA of HCC70 cells<br>*3 Genomic DNA of MDA-MB-231 cells<br>*4 contains 50 ng of the template DNA in 2.0 μL of whole mixture. | |

3-6. Real-Time PCR System and PCR Reaction

PCR reactions were carried out under the same conditions as those in Test 2, using the same real-time PCR system as that in Test 2.

3-7. Determination of δCt (Mutant 10% to 0.01%) with or without Addition of Polymer and Calculation of δδCt (Mutant 10% to 0.01%) with or without Addition of Polymer

The reaction mixture 1 (with addition of polymer, with addition of BNA clamp) and the reaction mixture 2 (without addition of polymer, with addition of BNA clamp) were subjected to the above-described PCR reaction using the template DNA or each template DNA mixture to obtain amplification curves, Ct (Mutant 10% to 0%) was determined in the same manner as in Test 2 from the amplification curve under each condition, and as shown in the following table, δCt (Mutant 10% to 0.01%) with or without addition of each polymer was calculated by subtracting Ct (Mutant 10% to 0.01%) at each mutant gene template concentration from Ct (Mutant 0%, WT).

[Table 18]

| Calculation formula of δCt (Mutant 10% to 0.01%) in case of no addition of polymer | | | | | |
|---|---|---|---|---|---|
| Mutant | 10% | 1% | 0.10% | 0.01% | 0% |
| Ct | ① | ② | ③ | ④ | ⑤ |
| δCt | ⑤-① | ⑤-② | ⑤-③ | ⑤-④ | |

[Table 19]

| Calculation formula of δCt (Mutant 10% to 0.01%) in case of addition of polymer | | | | | |
|---|---|---|---|---|---|
| Mutant | 10% | 1% | 0.10% | 0.01% | 0% |
| Ct | A | B | C | D | E |
| δCt | E-A | E-B | E-C | E-D | |

Finally, each δδCt (Mutant 10% to 0.01%) was calculated by subtracting δCt (Mutant 10% to 0.01%) in the case of no addition of polymer from δCt (Mutant 10% to 0.01%), in the case of addition of polymer.

In a case where a mutation detection method based on the number of amplification cycles of a wild-type gene, such as an intercalator method, is used, when δδCt > 0, it means that a mutant gene is specifically detected by adding a polymer, and the amplification curve of the mutant gene is distinguished from the amplification curve of the wild-type gene (citation: Patent Literature 11).

3-8. Results

The test results are summarized in the following table and FIG. 3. In the case of a Mutant ratio of 0.01%, δCt without addition of polymer was -0.68.

[Table 20]

| Polymer | Evaluation of δδCt at each Mutant ratio | | | |
|---|---|---|---|---|
| | 10% | 1% | 0.1% | 0.01% |
| Example 1 (copolymer of diallylamine hydrochloride/maleic acid 1 : 1) | A | A | B | B |
| Example 2 (copolymer of diallylmethylamine/ maleic acid 1 : 1) | A | A | A | A |
| Example 4 (copolymer of dimethylaminopropyl methacrylamide hydrochloride/acrylamide/ acrylic acid 1 : 1 : 2) | B | A | B | B |
| Example 6 (copolymer of diallylamine hydrochloride/acrylamide/acrylic acid 1 : 1 : 2) | B | B | B | B |
| A: δδCt is 3.20 or more, B: δδCt is 1.00 or more and less than 3.20 | | | | |

As a result of the present test, it became clear that in the case of no addition of polymer, the Mutant ratio was 0.01% by mass, δCt was less than 0, and the amplification curve of the mutant gene could not be distinguished from the amplification curve of the wild-type gene; however, in the case where the polymer of Example 1, 2, 4, or 6 was added, the amplification curve of the mutant gene could be distinguished from the amplification curve of the wild-type gene even at a Mutant ratio of 0.01% by mass. Further, particularly when the polymer of Example 2 (amphoteric copolymer in which the cationic constituent unit is a constituent unit having a structure represented by the general formula (I-a) or the general formula (I-b), and the anionic constituent unit is a constituent unit represented by the general formula (II-a)) was added, it was revealed that the effect of enhancing the inhibitory effect on nucleic acid amplification by the clamp nucleic acid is especially large under the condition in which the Mutant ratio is 0.1% or less. Although a reaction mixture without addition of BNA clamp was not tested in the present experiment, it is clear from the results of Test 2 that there is no non-specific amplification even in a reaction mixture without addition of BNA clamp, regardless of the presence or absence of the addition of polymer.

<u>4. Adaptability 1 to Mutant Gene Other Than KRAS (Polymer-Induced Enhancement of Effect of Selectively Inhibiting Amplification of Wild-Type Gene by BNA Clamp)</u>

4-1. Polymer Used

Among the polymers used in Test 2, the polymers of Examples 1, 2, 4, 6, and 7 were used. The polymer solution concentration and the polymer concentration in the PCR reaction mixture in each case were as shown in the following table.

[Table 21]

| Polymer | Concentration of polymer solution (% by mass) | Concentration of polymer in reaction mixture (% by mass) |
|---|---|---|
| Example 1 (copolymer of diallylamine hydrochloride/maleic acid 1 : 1) | 0.25 | 0.025 |
| Example 2 (copolymer of diallylmethylamine/ maleic acid 1 : 1) | 0.50 | 0.050 |
| Example 4 (copolymer of dimethylaminopropyl methacrylamide hydrochloride/acrylamide/acrylic acid 1 : 1 : 2) | 1.00 | 0.100 |
| Example 6 (copolymer of diallylamine hydrochloride/acrylamide/acrylic acid 1 : 1 : 2) | 0.50 | 0.050 |
| Example 7 (copolymer of diallylamine/fumaric acid 1 : 0.8 added with 1 mol% sodium hypophosphite) | 1.00 | 0.100 |

4-2. Kit Used

Appendages of BNA Clamp BRAF Enrichment Kit (Riken Genesis Co., Ltd.), which is a kit for detecting a mutant-type BRAF gene, were used. The details will be described below, but the kit appendages were used as the primer set and the BNA clamp. The reaction conditions for real-time PCR were in accordance with the kit protocol.

4-3. Template DNA

As a template DNA having a standard base sequence, a genome of HCC70 cells having a wild-type BRAF gene, the genome containing the following base sequence (bases different from those of the base sequence of SEQ ID NO:13 that will be described below are shown in underlined bold letters), was used.

ATAGAAATTAGATCTCTTACCTAAACTCTTCATAATGCTTGCTCTGATAGGAAAATGAGATCTACTGTTTTCCTTA
CTTACTACACCTCAGATATATTTCTTCATGAAGACCTCACAGTAAAAATAGGTGATTTTGGTCTAGCTACAG**<u>T</u>**GAAA
TCTCGATGGAGTGGGTCCCATCAGTTTGAACAGTTGTCTGGATCCATTTTGTGGATGGTAAGAATTGAGGCTATTTT
TCCACTGATTAAATTTTTGGCCCTGAGATGCTGCTGAGTTACTAGAAAGTCATTGAAGGTCTCAACTAT (SEQ ID
NO:12)

It should be noted that the above-described sequence is understood to contain a base sequence other than the sequence to be amplified.

On the other hand, as a template DNA having a mutation relative to the standard base sequence, a genome of DU4475 cells having a mutant-type BRAF gene, the genome containing the following base sequence (bases different from those of the base sequence of SEQ ID NO: 12 described above are shown in underlined bold letters), was used.

ATAGAAATTAGATCTCTTACCTAAACTCTTCATAATGCTTGCTCTGATAGGAAAATGAGATCTACTGTTTTCCTT
TACTTACTACACCTCAGATATATTTCTTCATGAAGACCTCACAGTAAAAATAGGTGATTTTGGTCTAGCTACAG**<u>A</u>**
GAAATCTCGATGGAGTGGGTCCCATCAGTTTGAACAGTTGTCTGGATCCATTTTGTGGATGGTAAGAATTGAGGC
TATTTTTCCACTGATTAAATTTTTGGCCCTGAGATGCTGCTGAGTTACTAGAAAGTCATTGAAGGTCTCAACTAT
(SEQ ID NO:13)

It should be noted that the above-described sequence is understood to contain a base sequence other than the

sequence to be amplified.

4-4. BNA Clamp PCR Reaction Mixture

Reaction mixtures 1 to 4 having the compositions shown in the following table were prepared. In the reaction mixture 1 or the reaction mixture 2, the concentration of the polymer was as shown in Table 21 described above.

[Table 22]

| Reaction mixture 1 (polymer +, BNA clamp +) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0.2μL |
| Template DNA (50 ng/μL genomic DNA of HCC70 cells, or 50 ng/μL genomic DNA of DU4475 cells) | 1.0μL |
| 0.1% by mass polymer aqueous solution | 2.0μL |
| Nuclease-free water (manufactured by Thermo Fisher Scientific, Inc.) | 5.2μL |
| *1 contains Taq polymerase (TaKaRa Ex Taq HS), dNTP Mixture, $Mg^{2+}$, and a fluorescent dye (TB Green). | |

[Table 23]

| Reaction mixture 2 (polymer +, BNA clamp -) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0μL |
| Template DNA (50 ng/μL genomic DNA of HCC70 cells, or 50 ng/μL genomic DNA of DU4475 cells) | 1.0μL |
| 0.1% by mass polymer aqueous solution | 2.0μL |
| Nuclease-free water (manufactured by Thermo Fisher Scientific, Inc.) | 5.4μL |
| *1 contains Taq polymerase (TaKaRa Ex Taq HS), dNTP Mixture, $Mg^{2+}$, and a fluorescent dye (TB Green). | |

[Table 24]

| Reaction mixture 3 (polymer -, BNA clamp +) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0.2μL |
| Template DNA (50 ng/μL genomic DNA of HCC70 cells, or 50 ng/μL genomic DNA of DU4475 cells) | 1.0μL |
| Polymer aqueous solution | 0μL |

(continued)

| Reaction mixture 3 (polymer -, BNA clamp +) | |
|---|---|
| Component | Content |
| Nuclease-free water (manufactured by Thermo Fisher Scientific, Inc.) | 7.2μL |
| *1 contains Taq polymerase (TaKaRa Ex Taq HS), dNTP Mixture, $Mg^{2+}$, and a fluorescent dye (TB Green). | |

[Table 25]

| Reaction mixture 4 (polymer -, BNA clamp -) | |
|---|---|
| Component | Content |
| PCR Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0μL |
| Template DNA (50 ng/μL genomic DNA of HCC70 cells, or 50 ng/μL genomic DNA of MDU4475 cells) | 1.0μL |
| Polymer aqueous solution | 0μL |
| Nuclease-free water (manufactured by Thermo Fisher Scientific, Inc.) | 7.4μL |
| *1 contains Taq polymerase (TaKaRa Ex Taq HS), dNTP Mixture, $Mg^{2+}$, and a fluorescent dye (TB Green). | |

4-5. Real-Time PCR System and PCR Reaction

A PCR reaction was carried out in the same manner as in Test 2, using the same system as that used in Test 2.

4-6. Calculation of ΔCt (WT) and ΔCt (Mutant) with or without Addition of Polymer, Calculation of ΔΔCt with or without Addition of Polymer, and Calculation of ΔΔΔCt

The same procedure as that used in Test 2 was carried out.

4-7. Results

The test results are summarized below.

[Table 26]

| Polymer or monomer | ΔΔΔCt evaluation |
|---|---|
| Example 1 (copolymer of diallylamine hydrochloride/maleic acid 1 : 1) | A |
| Example 2 (copolymer0 of diallylmethylamine/maleic acid 1 : 1) | A |
| Example 4 (copolymer of dimethylaminopropyl methacrylamide hydrochloride/acrylamide/acrylic acid 1 : 1 : 2) | A |
| Example 6 (copolymer of diallylamine hydrochloride/acrylamide/ acrylic acid 1 : 1 : 2) | A |
| Example 7 (copolymer of diallylamine/fumaric acid 1 : 0.8 added with 1 mol% sodium hypophosphite) | A |

As described above, both the polymers enhanced the effect of selectively inhibiting amplification of the wild-type gene by the BNA clamp, similarly to the case of KRAS gene. It was found that the amplification inhibitory effect in both cases was larger than ΔΔΔCt = 3.2, which is an indication of a 10-fold increase in sensitivity.

## 5. Verification of Sensitivity Enhancement in BNA Clamp PCR Using Plasmid DNA and Sequence Analysis of PCR Products

### 5-1. Polymer Used

The same polymers as those used in Test 3 (polymers of Examples 1, 2, 4, and 6) were used. The concentration of each polymer solution and the concentration in the PCR reaction mixture are shown in the following table.

[Table 27]

| Polymer | Concentration of polymer solution (% by mass) | Polymer concentration in reaction mixture (% by mass) |
|---|---|---|
| Example 1 (copolymer of diallylamine hydrochloride/maleic acid 1 : 1) | 0.50 | 0.050 |
| Example 2 (copolymer of diallylmethylamine/ maleic acid 1 : 1) | 1.00 | 0.100 |
| Example 4 (copolymer of dimethylaminopropyl methacrylamide hydrochloride/acrylamide/acrylic acid 1 : 1 : 2) | 2.50 | 0.250 |
| Example 6 (copolymer of diallylamine hydrochloride/acrylamide/acrylic acid 1 : 1 : 2) | 1.00 | 0.100 |

### 5-2. Kit Used

The appendages of the same kit as that in Test 2 were used.

### 5-3. Template DNA

As the template DNA, KRAS WT/Mutant plasmid DNA shown in FIG. 4 was used. The content rate of Mutant DNA (Mutant ratio) in the template DNA was set to 0.01%. In FIG. 4, in the case of KRAS WT plasmid DNA, the inserted sequence was a sequence set forth in the following SEQ ID NO:14, and in the case of KRAS Mutant plasmid DNA, the inserted sequence was a sequence set forth in the following SEQ ID NO:15. In all the sequences, the last 6 bases (AAGCTT) are the recognition sequence of Hind III.

CATTATTTTTATTATAAGGCCTGCTGAAAATGACTGAATATAAACTTGTGGTAGTTGGAG
CTGGTG**G**CGTAGGCAAGAGTGCCTTGACGATACAGCTAATTCAGAATCATTTTGTGGACG
AATATGATCCAACAATAGAGGTAAATCTTGTTTTAATATGCATATTACTGGTGCAGGACC
ATTCTTTGATACACGATAAAGGTTTCAAGCTT
(SEQ ID NO:14)

CATTATTTTTATTATAAGGCCTGCTGAAAATGACTGAATATAAACTTGTGGTAGTTGGAG
CTGGTG**A**CGTAGGCAAGAGTGCCTTGACGATACAGCTAATTCAGAATCATTTTGTGGACG
AATATGATCCAACAATAGAGGTAAATCTTGTTTTAATATGCATATTACTGGTGCAGGACC
ATTCTTTGATACACGATAAAGGTTTCAAGCTT
(SEQ ID NO:15)

### 5-4. Primers and BNA Clamp

The same primers and BNA clamp as those used in Test 2 were used.

### 5-5. BNA Clamp PCR Reaction Mixture

Reaction mixtures 1 and 2 having compositions as shown in the following table were prepared.

[Table 28]

| Reaction mixture 1 (polymer +, BNA clamp +) | |
|---|---|
| Component | Content |
| PCB Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0.2μL |
| 250 ng/μl salmon sperm DNA | 0.2μL |
| Template DNA*2 | 2.0μL |
| Polymer aqueous solution | 2.0μL |
| Nuclease-free water (manufactured by Thermo Fisher Scientific, Inc.) | 4.0μL |
| *1 contains Taq polymerase (TaKaRa Ex Taq HS), dNTP Mixture, $Mg^{2+}$, and a fluorescent dye (TB Green).<br>*2 contains 50 copies of KRAS Mutant plasmid DNA and ($5\times10^5$-50) copies of KRAS WT plasmid DNA in 2.0 μL (Mutant ratio: 0.01%). | |

[Table 29]

| Reaction mixture 2 (polymer -, BNA clamp +) | |
|---|---|
| Component | Content |
| PCB Buffer TB Green Premix Dimer Eraser*1 (manufactured by Takara Bio, Inc.) | 10.0μL |
| 10 μM Forward primer (manufactured by Riken Genesis Co., Ltd.) | 0.6μL |
| 10 μM Reverse primer (manufactured by Riken Genesis Co., Ltd.) | 0.6μL |
| ROX dye (manufactured by Takara Bio, Inc.) | 0.4μL |
| 10 μM BNA clamp (manufactured by Riken Genesis Co., Ltd.) | 0.2μL |
| 250 ng/μl salmon sperm DNA | 0.2μL |
| Template DNA*2 | 2.0μL |
| Polymer aqueous solution | 0μL |
| Nuclease-free water (manufactured by Thermo Fisher Scientific, Inc.) | 6.0μL*2 |
| *1 contains Taq polymerase (TaKaRa Ex Taq HS), dNTP Mixture, $Mg^{2+}$, and a fluorescent dye (TB Green).<br>*2 contains 50 copies of KRAS Mutant plasmid DNA and ($5\times10^5$-50) copies of KRAS WT plasmid DNA in 2.0 μL (Mutant ratio: 0.01%). | |

5-6. Real-Time PCR System and PCR Reaction

PCR reactions were carried out under the same conditions as those in Test 2, using the same PReal-Time PCR system as that in Test 2.

5-7. Determination of δCt (WT) and δCt (Mutant) with or without Addition of Polymer, and Calculation of δδCt with or without Addition of Polymer

The same procedure as that used in Test 3 was carried out.

5-8. Sequence Analysis of PCR Products

The PCR products obtained by the PCR reaction were entrusted to Fasmac Co., Ltd. to perform sequence analysis by a direct sequencing method (FIG. 6).

5-9. Results

5-9-1. Study on Sensitivity Enhancement in BNA Clamp PCR

The test results are shown in the following table and FIG. 5.

[Table 30]

| Polymer | δδCt evaluation |
|---|---|
| Example 1 (copolymer of diallylamine hydrochloride/maleic acid 1 : 1) | B |
| Example 2 (copolymer of diallylmethylamine/maleic acid 1 : 1) | B |
| Example 4 (copolymer of dimethylaminopropyl methacrylamide hydrochloride/acrylamide/acrylic acid 1 : 1 : 2) | B |
| Example 6 (copolymer of diallylamine hydrochloride/acrylamide/acrylic acid 1 : 1 : 2) | B |
| A: δδCt is 3.20 or more, B: δδCt is 1.00 or more and less than 3.20 | |

As a result of the present test, it was difficult to detect a mutant gene at a Mutant ratio of 0.01% in the case without addition of polymer, but when the polymer of Example 1, 2, 4, or 6 was added, a mutant gene could be reliably detected even at a Mutant ratio of 0.01%.

5-9-1. Sequence Analysis of PCR Products

As shown in FIG. 6, at a Mutant ratio of 0.01%, the mutation can be detected even in the case without addition of polymer, but as understood from the peaks of bases surrounded by a broken line, it is understood that on the whole, the PCR products obtained when the polymers are added have a significantly larger mutant content, and sensitivity enhancement is clearly achieved compared to the case without addition of polymer.

## Claims

1. A method for detecting mutation in a target base sequence of a nucleic acid in a sample relative to a standard base sequence, comprising the steps of:

   subjecting the nucleic acid in the sample to a nucleic acid amplification reaction as a template with
   a clamp nucleic acid having a base sequence complementary to the standard base sequence and containing at least one artificial nucleotide residue, and
   an amphoteric copolymer that comprises a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in a range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

   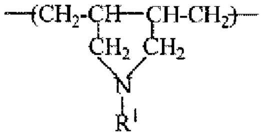
   (I-a)

   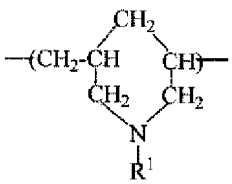
   (I-b)

   wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

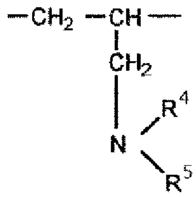

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

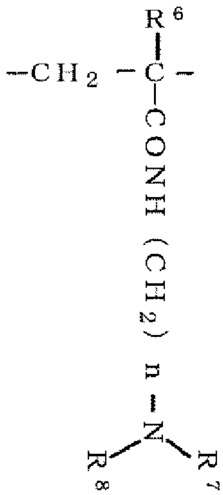

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and
at least some of the anionic constituent units (2) are a constituent unit (2-1), (2-2) or (2-4) represented by general formula (II-a), (II-b) or (II-d):

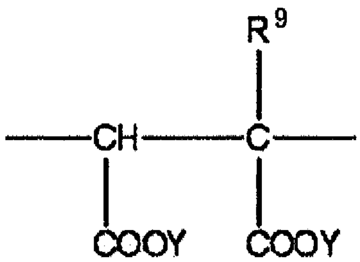

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

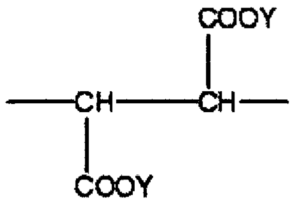

(II-b)

wherein Y represents a hydrogen, Na, K, $NH_4$, 1/2Ca, 1/2Mg, 1/2Fe, 1/3Al, or 1/3Fe independently for each carboxy group to be bonded, or

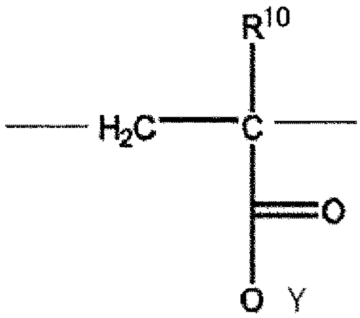

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded; and
determining the presence of the mutation based on the total amount of nucleic acid amplification products obtained by the nucleic acid amplification reaction, the number of amplification cycles of the nucleic acid amplification reaction required for the total amount of nucleic acid amplification products to reach a threshold value, the amount of nucleic acids with the mutation among nucleic acid amplification products, or the number of amplification cycles of the nucleic acid amplification reaction required for the amount of nucleic acids with the mutation among nucleic acid amplification products to reach a threshold value.

**2.** The method according to claim 1, wherein

(i) the cationic constituent unit (1) is selected from the group consisting of the constituent unit (1-1), and the constituent unit (1-4); or
(ii) the anionic constituent unit (2) is selected from the group consisting of the constituent unit (2-1), and the constituent unit (2-4); or
(iii) the amphoteric copolymer further comprises a nonionic constituent unit (3) derived from a methacrylamide-based monomer, or an acrylamide-based monomer; or
(iv) in the amphoteric copolymer, at least some of the cationic constituent units (1) are constituent units (1-1) having a structure represented by general formula (I-a) or general formula (I-b), or a structure of an acid addition salt thereof:

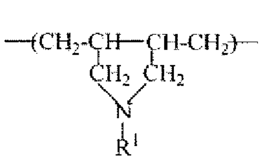

(I-a)

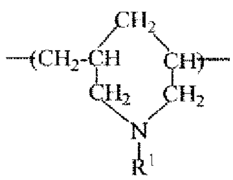

(I-b)

wherein $R^1$ represents a hydrogen atom, an alkyl group having 1 to 10 carbon atoms that may have a hydroxyl

group, a cycloalkyl group having 5 to 10 carbon atoms, or an aralkyl group having 7 to 10 carbon atoms, and at least some of the anionic constituent units (2) are constituent units (2-1) each represented by general formula (II-a):

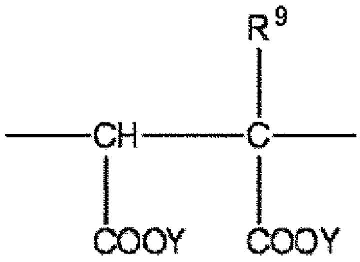

(II-a)

wherein $R^9$ is hydrogen or a methyl group, and Y's each independently is hydrogen, Na, K, $NH_4$, 1/2Ca, 1/2Mg, 1/2Fe, 1/3Al, or 1/3Fe for every carboxy group bonded thereto.

**3.** The method according to claim 1, wherein

(i) the artificial nucleotide is a BNA; or
(ii) the nucleic acid amplification is performed by real-time PCR; or
(iii) the nucleic acid sample is a genomic DNA.

**4.** The method according to any one of claims 1 to 3, wherein

a nucleic acid with the standard base sequence is further subjected to the nucleic acid amplification reaction as a template, and
when the number of amplification cycles of the nucleic acid amplification reaction required for the total amount of nucleic acid amplification products in the case of using the nucleic acid in the sample as a template to reach a threshold value, is smaller than the number of amplification cycles of the nucleic acid amplification reaction required for the total amount of nucleic acid amplification products in the case of using the nucleic acid with the standard base sequence as a template to reach a threshold value, it is determined that the mutation is present in the target base sequence of the nucleic acid in the sample.

**5.** A kit for:

(i) detecting a mutation in a target base sequence of a nucleic acid in a sample relative to a standard base sequence, comprising a clamp nucleic acid having a base sequence complementary to the standard base sequence and containing at least one artificial nucleotide residue; or
(ii) suppressing amplification of a nucleic acid having a target base sequence in a nucleic acid amplification reaction, comprising a clamp nucleic acid having a base sequence complementary to the target base sequence and containing at least one artificial nucleotide residue;

wherein the kit comprises

an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

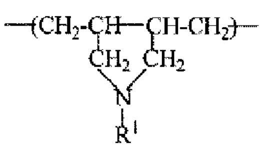

(I-a)

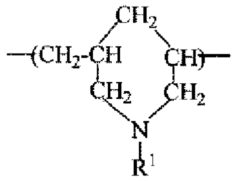

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

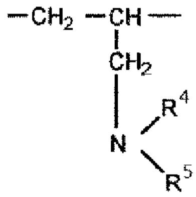

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

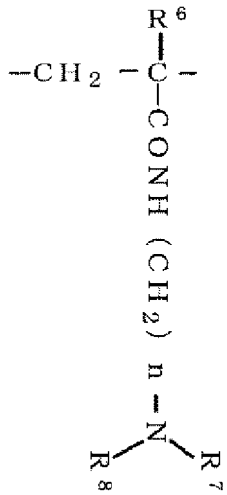

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and

at least some of the anionic constituent units (2) are a constituent unit (2-1), (2-2) or (2-4) represented by general formula (II-a), (II-b) or (II-d):

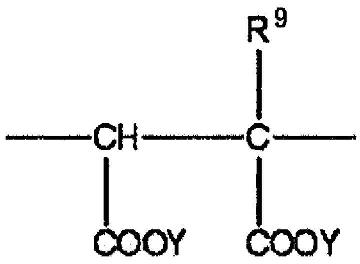

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

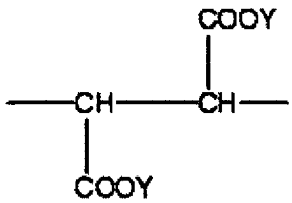

(II-b)

wherein Y represents a hydrogen, Na, K, $NH_4$, 1/2Ca, 1/2Mg, 1/2Fe, 1/3Al, or 1/3Fe independently for each carboxy group to be bonded, or

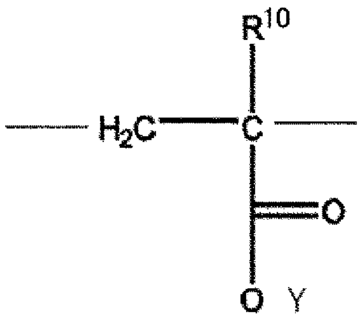

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

**6.** The kit according to claim 5, wherein

(i) the cationic constituent unit (1) is selected from the group consisting of the constituent unit (1-1), and the constituent unit (1-4); or
(ii) the anionic constituent unit (2) is selected from the group consisting of the constituent unit (2-1), and the constituent unit (2-4); or
(iii) the amphoteric copolymer further comprises a nonionic constituent unit (3) derived from a methacrylamide-based monomer, or an acrylamide-based monomer; or
(iv) in the amphoteric copolymer, at least some of the cationic constituent units (1) are a constituent unit (1-1) having a structure represented by general formula (I-a) or general formula (I-b), or a structure of an acid addition salt thereof:

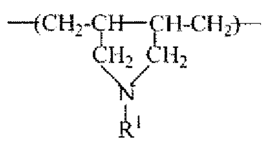 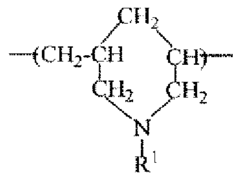

(I-a) (I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and at least some of the anionic constituent units (2) are constituent units (2-1) each represented by general formula (II-a):

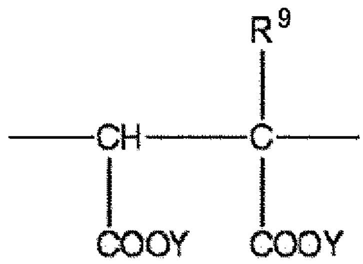

(II-a)

wherein $R^9$ is hydrogen or a methyl group, and Y's each independently is hydrogen, Na, K, $NH_4$, 1/2Ca, 1/2Mg, 1/2Fe, 1/3Al, or 1/3Fe for every carboxy group bonded thereto.

7. The kit according to claim 5 or 6, wherein the artificial nucleotide is a BNA.

8. A method for suppressing amplification of a nucleic acid in a sample having a predetermined target base sequence in a nucleic acid amplification reaction, comprising:

subjecting the nucleic acid in the sample to a nucleic acid amplification reaction as a template with
a clamp nucleic acid having a base sequence complementary to the target base sequence and containing at least one artificial nucleic acid residue, and
an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in a range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

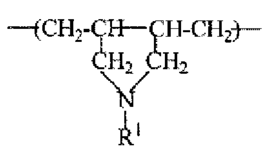 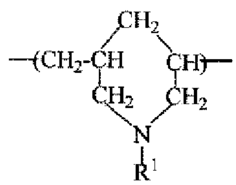

(I-a) (I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

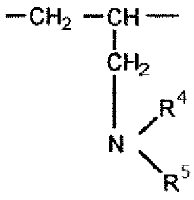

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

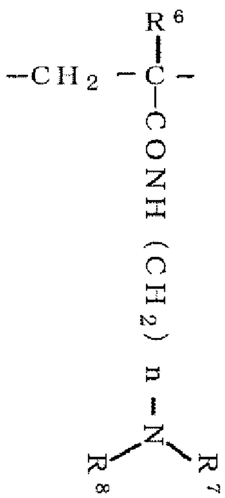

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and at least some of the anionic constituent units (2) are a constituent unit (2-1), (2-2) or (2-4) represented by general formula (II-a), (II-b) or (II-d):

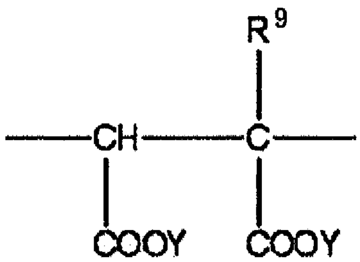

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

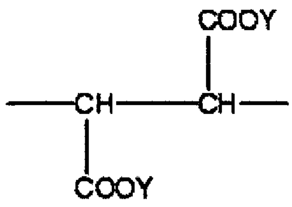

(II-b)

wherein Y represents a hydrogen, Na, K, $NH_4$, 1/2Ca, 1/2Mg, 1/2Fe, 1/3Al, or 1/3Fe independently for each carboxy group to be bonded, or

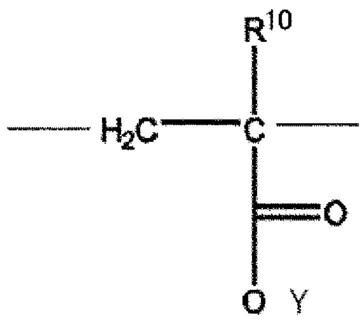

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

**9.** The method according to claim 8, wherein

(i) the cationic constituent unit (1) is selected from the group consisting of the constituent unit (1-1), and the constituent unit (1-4); or
(ii) the anionic constituent unit (2) is selected from the group consisting of the constituent unit (2-1), and the constituent unit (2-4); or
(iii) the amphoteric copolymer further comprises a nonionic constituent unit (3) derived from a methacrylamide-based monomer, or an acrylamide-based monomer; or
(iv) in the amphoteric copolymer, at least some of the cationic constituent units (1) are constituent units (1-1) having a structure represented by general formula (I-a) or general formula (I-b), or a structure of an acid addition salt thereof:

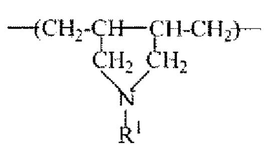

(I-a)

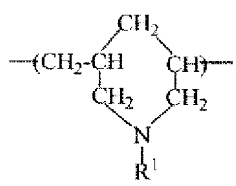

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and at least some of the anionic constituent units (2) are a constituent unit (2-1) represented by general formula (II-a):

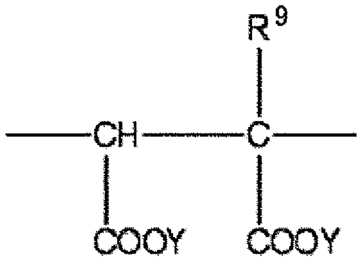

(II-a)

wherein $R^9$ is hydrogen or a methyl group, and Y's each independently is hydrogen, Na, K, $NH_4$, 1/2Ca, 1/2Mg, 1/2Fe, 1/3Al, or 1/3Fe for every carboxy group bonded thereto.

**10.** The method according to claim 8 or 9, wherein the artificial nucleotide is a BNA.

**11.** Use of an agent for enhancing an effect of inhibiting nucleic acid amplification of a nucleic acid having a target base sequence by a clamp nucleic acid that has a base sequence complementary to the target base sequence and contains at least one artificial nucleotide residue, the agent comprising:

an amphoteric copolymer comprising a cationic constituent unit (1) with an amino group in its structure and an anionic constituent unit (2), wherein a molar ratio of the cationic constituent unit (1) to the anionic constituent unit (2) (cationic constituent unit (1)/anionic constituent unit (2)) is in the range of 0.13 to 1.62, and wherein at least some of the cationic constituent units (1) are a constituent unit (1-1), (1-3) or (1-4) having a structure represented by general formula (I-a), (I-b), (I-e) or (I-f), or a structure of an acid addition salt thereof:

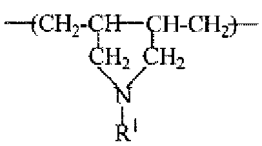

(I-a)

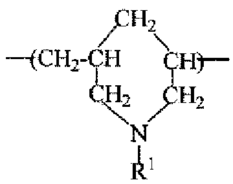

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

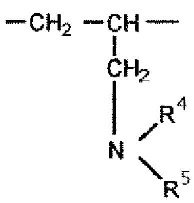

(1-e)

wherein $R^4$ and $R^5$ independently represent a hydrogen atom, an alkyl group having 1 to 12 carbon atoms that may have a hydroxyl group, an aralkyl group having 7 to 12 carbon atoms, or a cycloalkyl group having 5 to 6 carbon atoms, or

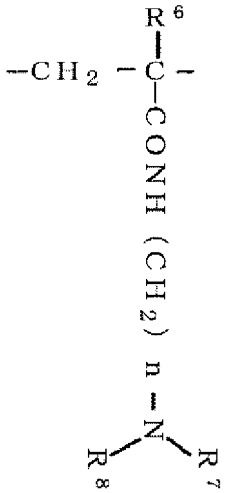

(1-f)

wherein $R^6$ represents a hydrogen atom or a methyl group, $R^7$ and $R^8$ each independently represent a hydrogen atom or an alkyl group having 1 to 4 carbon atoms, and n is an integer of 2 to 4, and
at least some of the anionic constituent units (2) are a constituent unit (2-1), (2-2) or (2-4) represented by general formula (II-a), (II-b) or (II-d):

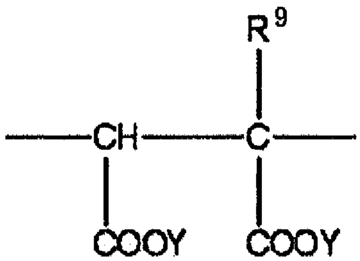

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto,

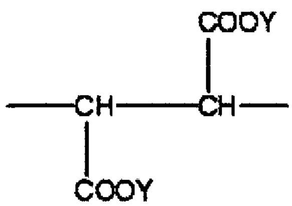

(II-b)

wherein Y represents a hydrogen, Na, K, $NH_4$, 1/2Ca, 1/2Mg, 1/2Fe, 1/3Al, or 1/3Fe independently for each carboxy group to be bonded, or

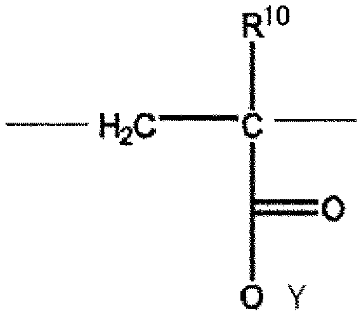

(II-d)

wherein $R^{10}$ is a hydrogen or a methyl group, and Y is a hydrogen, Na, K, $NH_4$, 1/2Ca, 1/2Mg, 1/2Fe, 1/3Al, or 1/3Fe independently for each carboxy group to be bonded.

**12.** The use according to claim 11, wherein

(i) the cationic constituent unit (1) is selected from the group consisting of the constituent unit (1-1), and the constituent unit (1-4); or
(ii) the anionic constituent unit (2) is selected from the group consisting of the constituent unit (2-1), and the constituent unit (2-4); or
(iii) the amphoteric copolymer further comprises a nonionic constituent unit (3) derived from a methacrylamide-based monomer, or an acrylamide-based monomer; or
(iv) in the amphoteric copolymer, at least some of the cationic constituent units (1)) are a constituent unit (1-1) having a structure represented by general formula (I-a) or general formula (I-b), or a structure of an acid addition salt thereof:

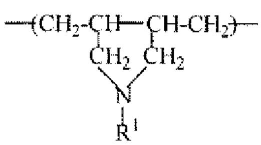

(I-a)

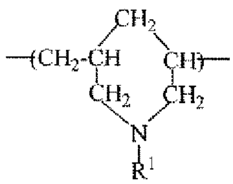

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms, and at least some of the anionic constituent units (2) are a constituent unit (2-1) represented by general formula (II-a):

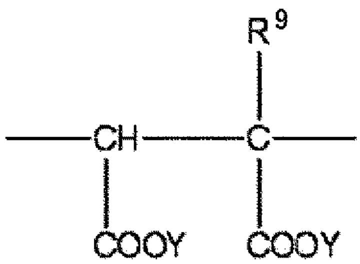

(II-a)

wherein $R^9$ is hydrogen or a methyl group, and Y's each independently are hydrogen, Na, K, $NH_4$, 1/2Ca, 1/2Mg, 1/2Fe, 1/3Al, or 1/3Fe for every carboxy group bonded thereto.

## Patentansprüche

**1.** Verfahren zum Detektieren von Mutation in einer Ziel-Basensequenz einer Nukleinsäure in einer Probe relativ zu einer Standard-Basensequenz, umfassend die Schritte:

Unterziehen der Nukleinsäure in der Probe einer Nukleinsäureamplifikationsreaktion als Matrize mit einer Nukleinsäureklemme mit einer Basensequenz, die komplementär zur Standard-Basensequenz ist und wenigstens einen artifiziellen Nukleotidrest enthält, und ein amphoteres Copolymer, welches eine kationische konstituierende Einheit (1) mit einer Aminogruppe in ihrer Struktur und eine anionische konstituierende Einheit (2) umfasst, wobei ein molares Verhältnis der kationischen konstituierenden Einheit (1) zu der anionischen konstituierenden Einheit (2) (kationische konstituierende Einheit (1)/anionische konstituierende Einheit (2)) in einem Bereich von 0,13 bis 1,62 liegt und wobei wenigstens einige der kationischen konstituierenden Einheiten (1) konstituierende Einheiten (1-1), (1-3) oder (1-4) sind mit einer Struktur, dargestellt durch die allgemeine Formel (I-a), (I-b), (I-e) oder (I-f), oder eine Struktur eines Säureadditionssalzes davon:

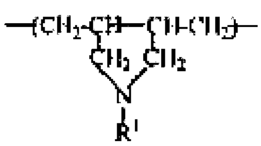

(I-a)

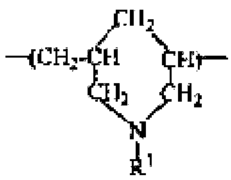

(I-b)

,

wobei $R^1$ ein Wasserstoffatom, eine Alkylgruppe mit 1 bis 10 Kohlenstoffatomen, die eine Hydroxylgruppe aufweisen kann, eine Cycloalkylgruppe mit 5 bis 10 Kohlenstoffatomen oder eine Aralkylgruppe mit 7 bis 10 Kohlenstoffatomen darstellt,

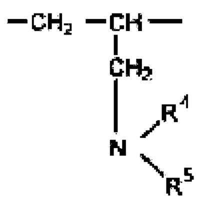

(1-e)

,

wobei $R^4$ und $R^5$ unabhängig ein Wasserstoffatom, eine Alkylgruppe mit 1 bis 12 Kohlenstoffatomen, die eine Hydroxylgruppe aufweisen kann, eine Aralkylgruppe mit 7 bis 12 Kohlenstoffatomen oder eine Cycloalkylgruppe mit 5 bis 6 Kohlenstoffatomen darstellen, oder

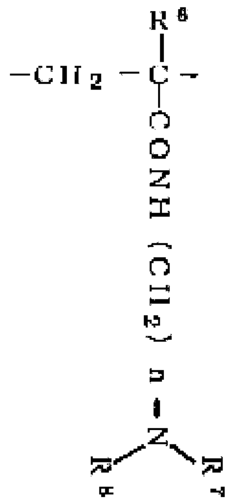

(I-f)

wobei $R^6$ ein Wasserstoffatom oder eine Methylgruppe darstellt, $R^7$ und $R^8$ jeweils unabhängig ein Wasserstoffatom oder eine Alkylgruppe mit 1 bis 4 Kohlenstoffatomen darstellen und n eine ganze Zahl von 2 bis 4 ist, und wenigstens einige der anionischen konstituierenden Einheiten (2) eine konstituierende Einheit (2-1), (2-2) oder (2-4) sind, dargestellt durch die allgemeine Formel (II-a), (II-b) oder (II-d):

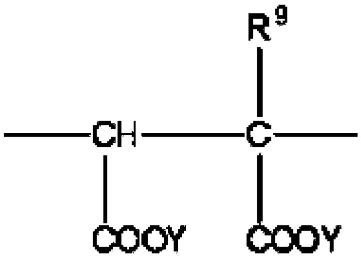

(II-a) ,

wobei $R^9$ Wasserstoff oder eine Methylgruppe ist und Y jeweils unabhängig Wasserstoff, Na, K, $NH_4$, 1/2Ca, 1/2Mg, 1/2Fe, 1/3Al oder 1/3Fe für jede daran gebundene Carboxygruppe sind,

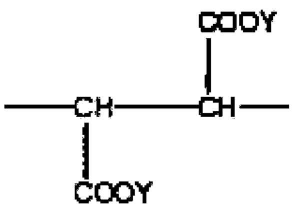

(II-b) ,

wobei Y einen Wasserstoff, Na, K, $NH_4$, 1/2Ca, 1/2Mg, 1/2Fe, 1/3Al oder 1/3Fe unabhängig für jede zu bindende Carboxygruppe darstellt, oder

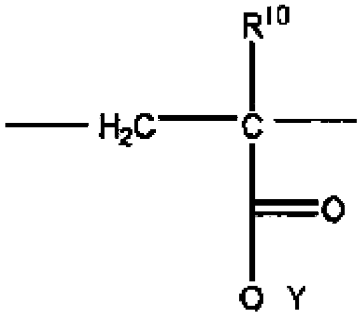

(II-d)

wobei $R^{10}$ ein Wasserstoff oder eine Methylgruppe ist und Y ein Wasserstoff, Na, K, $NH_4$, 1/2Ca, 1/2Mg, 1/2Fe, 1/3Al oder 1/3Fe unabhängig für jede zu bindende Carboxygruppe ist; und
Bestimmen des Vorliegens der Mutation auf Grundlage der Gesamtmenge an Nukleinsäureamplifikationsprodukten, erhalten durch die Nukleinsäureamplifikationsreaktion, der Anzahl an Amplifizierungszyklen der Nukleinsäureamplifikationsreaktion, die erforderlich sind, damit die Gesamtmenge an Nukleinsäureamplifikationsprodukten einen Schwellwert erreicht, der Menge an Nukleinsäuren mit der Mutation unter den Nukleinsäureamplifikationsprodukten oder der Anzahl an Amplifizierungszyklen der Nukleinsäureamplifikationsreaktion, die erforderlich sind, damit die Menge an Nukleinsäuren mit der Mutation unter den Nukleinsäureamplifikationsprodukten einen Schwellwert erreicht.

**2.** Verfahren gemäß Anspruch 1, wobei

(i) die kationische konstituierende Einheit (1) aus der Gruppe ausgewählt ist, bestehend aus der konstituierenden Einheit (1-1) und der konstituierenden Einheit (1-4); oder
(ii) die anionische konstituierende Einheit (2) aus der Gruppe ausgewählt ist, bestehend aus der konstituierenden Einheit (2-1) und der konstituierenden Einheit (2-4); oder
(iii) das amphotere Copolymer ferner eine nicht-ionische konstituierende Einheit (3) umfasst, abgeleitet von einem Methacrylamid-basierenden Monomer oder einem Acrylamid-basierenden Monomer; oder
(iv) in dem amphoteren Copolymer wenigstens einige der kationischen konstituierenden Einheiten (1) konstituierende Einheiten (1-1) sind mit einer Struktur, dargestellt durch die allgemeine Formel (I-a) oder die allgemeine Formel (I-b), oder eine Struktur eines Säureadditionssalzes davon:

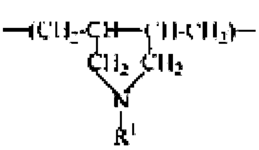

(I-a)

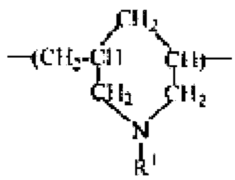

(I-b)

,

wobei $R^1$ ein Wasserstoffatom, eine Alkylgruppe mit 1 bis 10 Kohlenstoffatomen, die eine Hydroxylgruppe aufweisen kann, eine Cycloalkylgruppe mit 5 bis 10 Kohlenstoffatomen oder eine Aralkylgruppe mit 7 bis 10 Kohlenstoffatomen darstellt, und
wenigstens einige der anionischen konstituierenden Einheiten (2) konstituierende Einheiten (2-1) sind, jeweils dargestellt durch die allgemeine Formel (II-a):

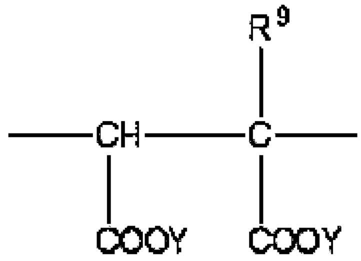

(II-a)

,

wobei $R^9$ Wasserstoff oder eine Methylgruppe ist und Y jeweils unabhängig Wasserstoff, Na, K, $NH_4$, 1/2Ca, 1/2Mg, 1/2Fe, 1/3Al oder 1/3Fe für jede daran gebundene Carboxygruppe ist.

**3.** Verfahren gemäß Anspruch 1, wobei

(i) das artifizielle Nukleotid eine BNA ist; oder
(ii) die Nukleinsäureamplifikation durch Echtzeit-PCR durchgeführt wird; oder
(iii) die Nukleinsäureprobe eine genomische DNA ist.

**4.** Verfahren gemäß irgendeinem der Ansprüche 1 bis 3, wobei

eine Nukleinsäure mit der Standard-Basensequenz ferner der Nukleinsäureamplifikationsreaktion als Matrize unterworfen wird und
wenn die Anzahl an Amplifizierungszyklen der Nukleinsäureamplifikationsreaktion, die erforderlich sind, damit die Gesamtmenge an Nukleinsäureamplifikationsprodukten, falls die Nukleinsäure in der Probe als Matrize verwendet wird, einen Schwellwert erreicht, kleiner ist als die Anzahl an Amplifizierungszyklen der Nukleinsäureamplifikationsreaktion, die erforderlich sind, damit die Gesamtmenge an Nukleinsäureamplifikationsprodukten, falls die Nukleinsäure mit der Standard-Basensequenz als Matrize verwendet wird, einen Schwellwert erreicht, bestimmt wird, dass die Mutation in der Ziel-Basensequenz der Nukleinsäure in der Probe vorhanden ist.

**5.** Kit:

(i) zum Detektieren einer Mutation in einer Ziel-Basensequenz einer Nukleinsäure in einer Probe relativ zu einer Standard-Basensequenz, umfassend eine Nukleinsäureklemme mit einer Basensequenz, die komplementär zur Standard-Basensequenz ist und wenigstens einen artifiziellen Nukleotidrest enthält; oder
(ii) zum Unterdrücken der Amplifikation einer Nukleinsäure mit einer Ziel-Basensequenz in einer Nukleinsäureamplifikationsreaktion, umfassend eine Nukleinsäureklemme mit einer Basensequenz, die komplementär zur Ziel-Basensequenz ist und wenigstens einen artifiziellen Nukleotidrest enthält;

wobei das Kit umfasst:

ein amphoteres Copolymer, umfassend eine kationische konstituierende Einheit (1) mit einer Aminogruppe in ihrer Struktur und eine anionische konstituierende Einheit (2), wobei ein molares Verhältnis der kationischen konstituierenden Einheit (1) zu der anionischen konstituierenden Einheit (2) (kationische konstituierende Einheit (1)/anionische konstituierende Einheit (2)) im Bereich von 0,13 bis 1,62 liegt, und wobei wenigstens einige der kationischen konstituierenden Einheiten (1) konstituierende Einheiten (1-1), (1-3) oder (1-4) sind mit einer Struktur, dargestellt durch die allgemeine Formel (I-a), (I-b), (I-e) oder (I-f), oder eine Struktur eines Säureadditionssalzes davon:

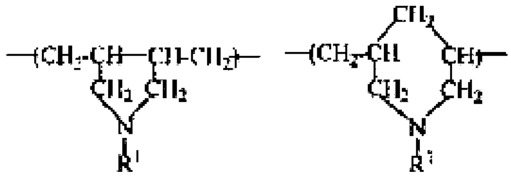

(I-a) (I-b)

,

wobei $R^1$ ein Wasserstoffatom, eine Alkylgruppe mit 1 bis 10 Kohlenstoffatomen, die eine Hydroxylgruppe aufweisen kann, eine Cycloalkylgruppe mit 5 bis 10 Kohlenstoffatomen oder eine Aralkylgruppe mit 7 bis 10 Kohlenstoffatomen darstellt,

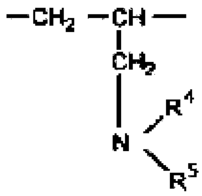

(1-e)

,

wobei $R^4$ und $R^5$ unabhängig ein Wasserstoffatom, eine Alkylgruppe mit 1 bis 12 Kohlenstoffatomen, die eine Hydroxylgruppe aufweisen kann, eine Aralkylgruppe mit 7 bis 12 Kohlenstoffatomen oder eine Cycloalkylgruppe mit 5 bis 6 Kohlenstoffatomen darstellen, oder

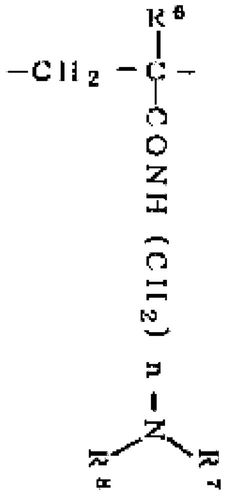

(1-f)

,

wobei $R^6$ ein Wasserstoffatom oder eine Methylgruppe darstellt, $R^7$ und $R^8$ jeweils unabhängig ein Wasserstoffatom oder eine Alkylgruppe mit 1 bis 4 Kohlenstoffatomen darstellen und n eine ganze Zahl von 2 bis 4 ist, und wenigstens einige der anionischen konstituierenden Einheiten (2) eine konstituierende Einheit (2-1), (2-2) oder (2-4) sind, dargestellt durch die allgemeine Formel (II-a), (II-b) oder (II-d):

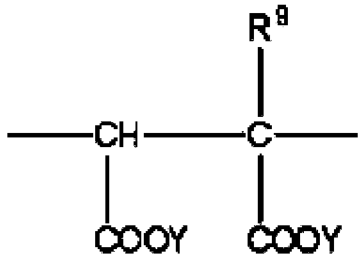

(II-a)

,

wobei $R^9$ Wasserstoff oder eine Methylgruppe ist und Y jeweils unabhängig Wasserstoff, Na, K, $NH_4$, 1/2Ca, 1/2Mg, 1/2Fe, 1/3Al oder 1/3Fe für jede daran gebundene Carboxygruppe sind,

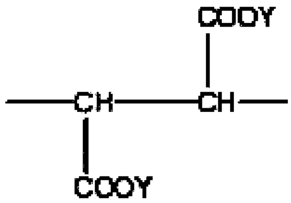

(II-b)

,

wobei Y einen Wasserstoff, Na, K, $NH_4$, 1/2Ca, 1/2Mg, 1/2Fe, 1/3Al oder 1/3Fe unabhängig für jede zu bindende Carboxygruppe darstellt, oder

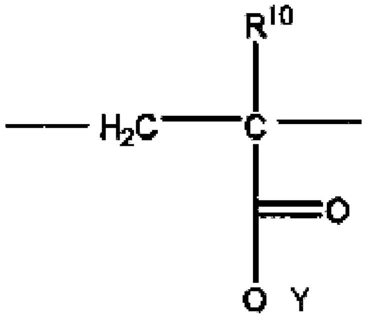

(II-d)

,

wobei $R^{10}$ ein Wasserstoff oder eine Methylgruppe ist und Y ein Wasserstoff, Na, K, $NH_4$, 1/2Ca, 1/2Mg, 1/2Fe, 1/3Al oder 1/3Fe unabhängig für jede zu bindende Carboxygruppe ist.

**6.** Kit gemäß Anspruch 5, wobei

(i) die kationische konstituierende Einheit (1) aus der Gruppe ausgewählt ist, bestehend aus der konstituierenden Einheit (1-1) und der konstituierenden Einheit (1-4); oder
(ii) die anionische konstituierende Einheit(2) aus der Gruppe ausgewählt ist, bestehend aus der konstituierenden Einheit (2-1) und der konstituierenden Einheit (2-4); oder
(iii) das amphotere Copolymer ferner eine nicht-ionische konstituierende Einheit (3) umfasst, abgeleitet von einem Methacrylamid-basierenden Monomer oder einem Acrylamid-basierenden Monomer; oder
(iv) in dem amphoteren Copolymer wenigstens einige der kationischen konstituierenden Einheiten (1) konstituierende Einheiten (1-1) sind mit einer Struktur, dargestellt durch die allgemeine Formel (I-a) oder die allgemeine Formel (I-b) oder eine Struktur eines Säureadditionssalzes davon:

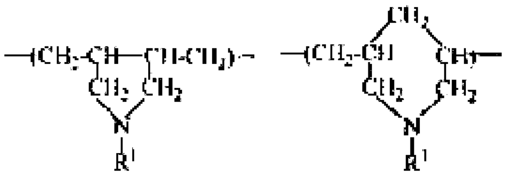

(I-a) (I-b) ,

wobei $R^1$ ein Wasserstoffatom, eine Alkylgruppe mit 1 bis 10 Kohlenstoffatomen, die eine Hydroxylgruppe aufweisen kann, eine Cycloalkylgruppe mit 5 bis 10 Kohlenstoffatomen oder eine Aralkylgruppe mit 7 bis 10 Kohlenstoffatomen darstellt, und

wenigstens einige der anionischen konstituierenden Einheiten (2) konstituierende Einheiten (2-1) sind, jeweils dargestellt durch die allgemeine Formel (II-a):

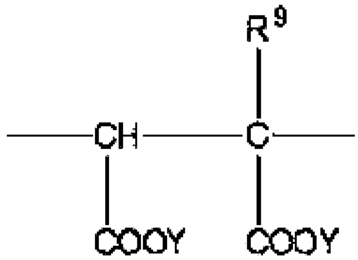

(II-a) ,

wobei $R^9$ Wasserstoff oder eine Methylgruppe ist und Y jeweils unabhängig Wasserstoff, Na, K, $NH_4$, 1/2Ca, 1/2Mg, 1/2Fe, 1/3Al oder 1/3Fe für jede daran gebundene Carboxygruppe ist.

**7.** Kit gemäß Anspruch 5 oder 6, wobei das artifizielle Nukleotid eine BNA ist.

**8.** Verfahren zum Unterdrücken der Amplifikation einer Nukleinsäure in einer Probe mit einer vorbestimmten Ziel-Basensequenz in einer Nukleinsäureamplifikationsreaktion, umfassend:

Unterziehen der Nukleinsäure in der Probe einer Nukleinsäureamplifikationsreaktion als Matrize mit

einer Nukleinsäureklemme mit einer Basensequenz, die komplementär zu der Ziel-Basensequenz ist und wenigstens einen artifiziellen Nukleinsäurerest enthält, und

einem amphoteren Copolymer, umfassend eine kationische konstituierende Einheit (1) mit einer Aminogruppe in ihrer Struktur und eine anionische konstituierende Einheit (2), wobei ein molares Verhältnis der kationischen konstituierenden Einheit (1) zu der anionischen konstituierenden Einheit (2) (kationische konstituierende Einheit (1)/anionische konstituierende Einheit (2)) in einem Bereich von 0,13 bis 1,62 liegt und wobei wenigstens einige der kationischen konstituierenden Einheiten (1) konstituierende Einheiten (1-1), (1-3) oder (1-4) sind mit einer Struktur, dargestellt durch die allgemeine Formel (I-a), (I-b), (I-e) oder (I-f) oder eine Struktur eines Säure-additionssalzes davon:

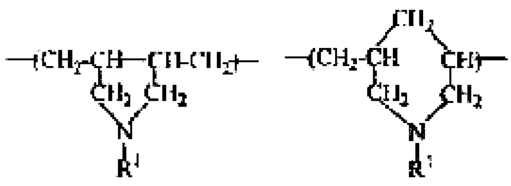

(I-a) (I-b) ,

wobei $R^1$ ein Wasserstoffatom, eine Alkylgruppe mit 1 bis 10 Kohlenstoffatomen, die eine Hydroxylgruppe aufweisen kann, eine Cycloalkylgruppe mit 5 bis 10 Kohlenstoffatomen oder eine Aralkylgruppe mit 7 bis 10 Kohlenstoffatomen darstellt,

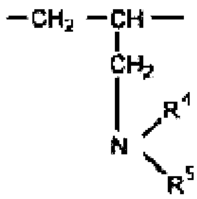

(1-e)

,

wobei $R^4$ und $R^5$ unabhängig ein Wasserstoffatom, eine Alkylgruppe mit 1 bis 12 Kohlenstoffatomen, die eine Hydroxylgruppe aufweisen kann, eine Aralkylgruppe mit 7 bis 12 Kohlenstoffatomen oder eine Cycloalkylgruppe mit 5 bis 6 Kohlenstoffatomen darstellen, oder

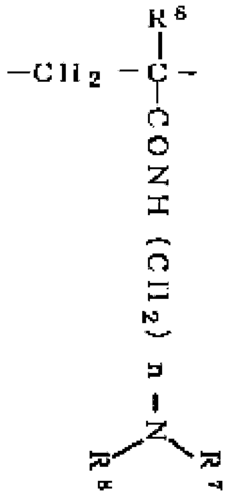

(1-f)

,

wobei $R^6$ ein Wasserstoffatom oder eine Methylgruppe darstellt, $R^7$ und $R^8$ jeweils unabhängig ein Wasserstoffatom oder eine Alkylgruppe mit 1 bis 4 Kohlenstoffatomen darstellen und n eine ganze Zahl von 2 bis 4 ist, und wenigstens einige der anionischen konstituierenden Einheiten (2) eine konstituierende Einheit (2-1), (2-2) oder (2-4) sind, dargestellt durch die allgemeine Formel (II-a), (II-b) oder (II-d):

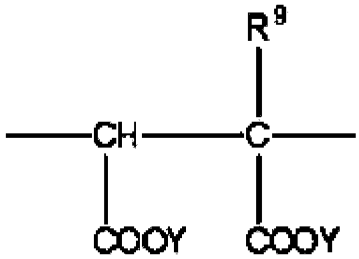

(II-a)

,

wobei $R^9$ Wasserstoff oder eine Methylgruppe ist und Y jeweils unabhängig Wasserstoff, Na, K, $NH_4$, 1/2Ca, 1/2Mg, 1/2Fe, 1/3Al oder 1/3Fe für jede daran gebundene Carboxygruppe sind,

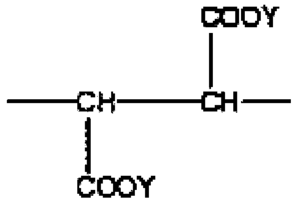

(II-b)

,

wobei Y einen Wasserstoff, Na, K, $NH_4$, 1/2Ca, 1/2Mg, 1/2Fe, 1/3Al oder 1/3Fe unabhängig für jede zu bindende Carboxygruppe darstellt, oder

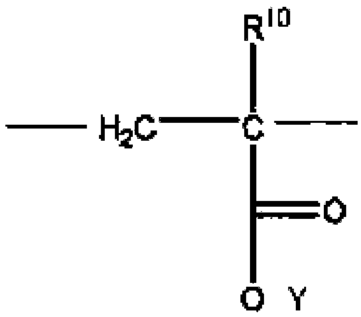

(II-d) ,

wobei $R^{10}$ ein Wasserstoff oder eine Methylgruppe ist und Y ein Wasserstoff, Na, K, $NH_4$, 1/2Ca, 1/2Mg, 1/2Fe, 1/3Al oder 1/3Fe unabhängig für jede zu bindende Carboxygruppe ist.

**9.** Verfahren gemäß Anspruch 8, wobei

(i) die kationische konstituierende Einheit (1) aus der Gruppe ausgewählt ist, bestehend aus der konstituierenden Einheit (1-1) und der konstituierenden Einheit (1-4); oder
(ii) die anionische konstituierende Einheit(2) aus der Gruppe ausgewählt ist, bestehend aus der konstituierenden Einheit (2-1) und der konstituierenden Einheit (2-4); oder
(iii) das amphotere Copolymer ferner eine nicht-ionische konstituierende Einheit (3) umfasst, abgeleitet von einem Methacrylamid-basierenden Monomer oder einem Acrylamid-basierenden Monomer; oder
(iv) in dem amphoteren Copolymer wenigstens einige der kationischen konstituierenden Einheiten (1) konstituierende Einheiten (1-1) sind mit einer Struktur, dargestellt durch die allgemeine Formel (I-a) oder die allgemeine Formel (I-b) oder eine Struktur eines Säureadditionssalzes davon:

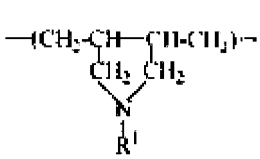

(I-a)

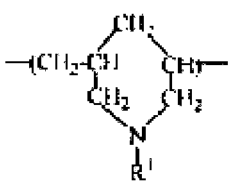

(I-b)

,

wobei $R^1$ ein Wasserstoffatom, eine Alkylgruppe mit 1 bis 10 Kohlenstoffatomen, die eine Hydroxylgruppe aufweisen kann, eine Cycloalkylgruppe mit 5 bis 10 Kohlenstoffatomen oder eine Aralkylgruppe mit 7 bis 10 Kohlenstoffatomen darstellt, und
wenigstens einige der anionischen konstituierenden Einheiten (2) eine konstituierende Einheit (2-1) sind, dargestellt durch die allgemeine Formel (IIa):

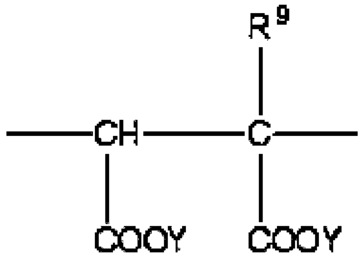

(II-a)

,

wobei $R^9$ Wasserstoff oder eine Methylgruppe ist und Y jeweils unabhängig Wasserstoff, Na, K, $NH_4$, 1/2Ca, 1/2Mg, 1/2Fe, 1/3Al oder 1/3Fe für jede daran gebundene Carboxygruppe ist.

**10.** Verfahren gemäß Anspruch 8 oder 9, wobei das artifizielle Nukleotid eine BNA ist.

**11.** Verwendung eines Agens zum Verstärken einer Wirkung der Inhibition einer Nukleinsäureamplifikation einer Nukleinsäure mit einer Ziel-Basensequenz durch eine Nukleinsäureklemme, die eine Basensequenz hat, die komplementär zur Ziel-Basensequenz ist, und wenigstens einen artifiziellen Nukleotidrest enthält, wobei das Agens umfasst:

ein amphoteres Copolymer, umfassend eine kationische konstituierende Einheit (1) mit einer Aminogruppe in ihrer Struktur und eine anionische konstituierende Einheit (2), wobei ein molares Verhältnis der kationischen konstituierenden Einheit (1) zu der anionischen konstituierenden Einheit (2) (kationische konstituierende Einheit (1)/anionische konstituierende Einheit (2)) im Bereich von 0,13 bis 1,62 liegt und wobei wenigstens einige der kationischen konstituierenden Einheiten (1) konstituierende Einheiten (1-1), (1-3) oder (1-4) sind mit einer Struktur, dargestellt durch die allgemeine Formel (I-a), (I-b), (I-e) oder (I-f) oder eine Struktur eines Säureadditionssalzes davon:

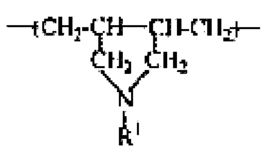

(I-a)

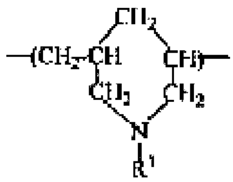

(I-b)

,

wobei $R^1$ ein Wasserstoffatom, eine Alkylgruppe mit 1 bis 10 Kohlenstoffatomen, die eine Hydroxylgruppe aufweisen kann, eine Cycloalkylgruppe mit 5 bis 10 Kohlenstoffatomen oder eine Aralkylgruppe mit 7 bis 10 Kohlenstoffatomen darstellt,

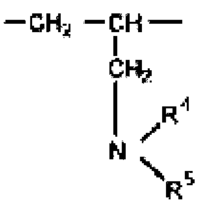

(1-e)

,

wobei $R^4$ und $R^5$ unabhängig ein Wasserstoffatom, eine Alkylgruppe mit 1 bis 12 Kohlenstoffatomen, die eine Hydroxylgruppe aufweisen kann, eine Aralkylgruppe mit 7 bis 12 Kohlenstoffatomen oder eine Cycloalkylgruppe mit 5 bis 6 Kohlenstoffatomen darstellen, oder

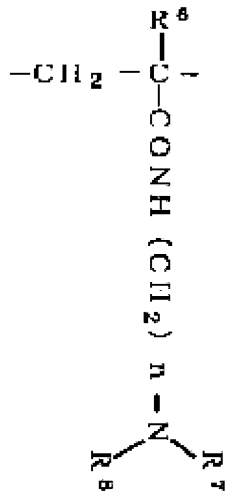

(I-f)

wobei $R^6$ ein Wasserstoffatom oder eine Methylgruppe darstellt, $R^7$ und $R^8$ jeweils unabhängig ein Wasserstoffatom oder eine Alkylgruppe mit 1 bis 4 Kohlenstoffatomen darstellen und n eine ganze Zahl von 2 bis 4 ist, und wenigstens einige der anionischen konstituierenden Einheiten (2) eine konstituierende Einheit (2-1), (2-2) oder (2-4) sind, dargestellt durch die allgemeine Formel (II-a), (II-b) oder (II-d):

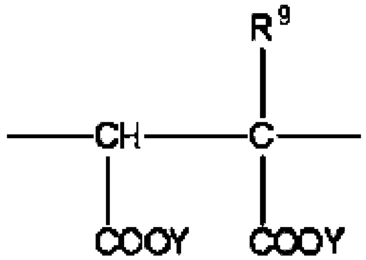

(II-a)

,

wobei $R^9$ Wasserstoff oder eine Methylgruppe ist und Y jeweils unabhängig Wasserstoff, Na, K, $NH_4$, 1/2Ca, 1/2Mg, 1/2Fe, 1/3Al oder 1/3Fe für jede daran gebundene Carboxygruppe sind,

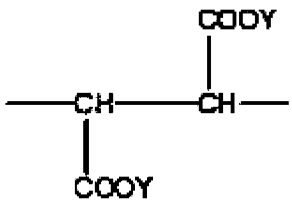

(II-b)

wobei Y einen Wasserstoff, Na, K, $NH_4$, 1/2Ca, 1/2Mg, 1/2Fe, 1/3Al oder 1/3Fe unabhängig für jede zu bindende Carboxygruppe darstellt, oder

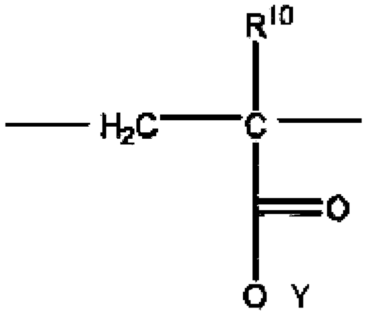

(II-d)

,

wobei $R^{10}$ ein Wasserstoff oder eine Methylgruppe ist und Y ein Wasserstoff, Na, K, $NH_4$, 1/2Ca, 1/2Mg, 1/2Fe, 1/3Al oder 1/3Fe unabhängig für jede zu bindende Carboxygruppe ist.

**12.** Verwendung gemäß Anspruch 11, wobei

(i) die kationische konstituierende Einheit (1) aus der Gruppe ausgewählt ist, bestehend aus der konstituierenden Einheit (1-1) und der konstituierenden Einheit (1-4); oder
(ii) die anionische konstituierende Einheit (2) aus der Gruppe ausgewählt ist, bestehend aus der konstituierenden Einheit (2-1) und der konstituierenden Einheit (2-4); oder
(iii) das amphotere Copolymer ferner eine nicht-ionische konstituierende Einheit (3) umfasst, abgeleitet von einem Methacrylamid-basierenden Monomer oder einem Acrylamid-basierenden Monomer; oder
(iv) in dem amphoteren Copolymer wenigstens einige der kationischen konstituierenden Einheiten (1) konstituierende Einheiten (1-1) sind mit einer Struktur, dargestellt durch die allgemeine Formel (I-a) oder die allgemeine Formel (I-b) oder eine Struktur eines Säureadditionssalzes davon:

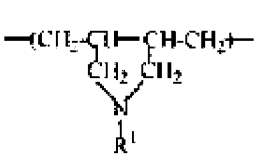

(I-a)

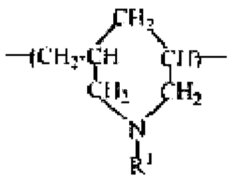

(I-b)

wobei $R^1$ ein Wasserstoffatom, eine Alkylgruppe mit 1 bis 10 Kohlenstoffatomen, die eine Hydroxylgruppe aufweisen kann, eine Cycloalkylgruppe mit 5 bis 10 Kohlenstoffatomen oder eine Aralkylgruppe mit 7 bis 10 Kohlenstoffatomen darstellt, und
wenigstens einige der anionischen konstituierenden Einheiten (2) eine konstituierende Einheit (2-1) sind, dargestellt durch die allgemeine Formel (IIa):

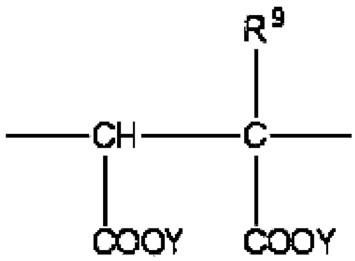

(II-a)

,

wobei $R^9$ Wasserstoff oder eine Methylgruppe ist und Y jeweils unabhängig Wasserstoff, Na, K, $NH_4$, 1/2Ca, 1/2Mg, 1/2Fe, 1/3Al oder 1/3Fe für jede daran gebundene Carboxygruppe sind.

## Revendications

**1.** Procédé destiné à détecter une mutation dans une séquence de bases objet d'un acide nucléique dans un échantillon par rapport à une séquence de bases standard, comprenant les étapes consistant à :

soumettre l'acide nucléique dans l'échantillon à une réaction d'amplification d'acide nucléique en tant que matrice avec

un acide nucléique clamp présentant une séquence de bases complémentaire de la séquence de bases standard et contenant au moins un résidu nucléotidique artificiel, et

un copolymère amphotère qui comprend une unité constitutive cationique (1) avec un groupe amino dans sa structure et une unité constitutive anionique (2), dans lequel un rapport molaire de l'unité constitutive cationique (1) à l'unité constitutive anionique (2) (unité constitutive cationique (1)/unité constitutive anionique (2)) est compris dans une plage allant de 0,13 à 1,62, et dans lequel au moins certaines des unités constitutives cationiques (1) sont une unité constitutive (1-1), (1-3) ou (1-4) présentant une structure représentée par la formule générale (I-a), (I-b), (I-e) ou (I-f), ou une structure d'un sel d'addition d'acide de celle-ci :

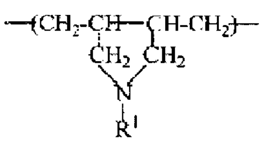

(I-a)

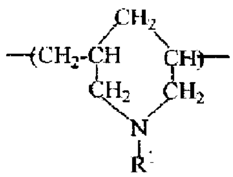

(I-b)

dans lequel $R^1$ représente un atome d'hydrogène, un groupe alkyle présentant 1 à 10 atomes de carbone pouvant présenter un groupe hydroxyle, un groupe cycloalkyle présentant 5 à 10 atomes de carbone, ou un groupe aralkyle présentant 7 à 10 atomes de carbone,

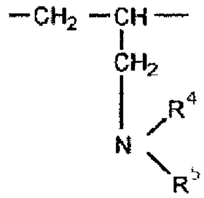

(1-e)

dans lequel $R^4$ et $R^5$ représentent indépendamment un atome d'hydrogène, un groupe alkyle présentant 1 à 12 atomes de carbone pouvant présenter un groupe hydroxyle, un groupe aralkyle présentant 7 à 12 atomes de carbone, ou un groupe cycloalkyle présentant 5 à 6 atomes de carbone, ou

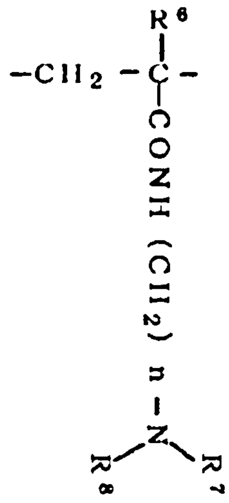

(1-f)

dans lequel $R^6$ représente un atome d'hydrogène ou un groupe méthyle, $R^7$ et $R^8$ représentent chacun indépendamment un atome d'hydrogène ou un groupe alkyle présentant 1 à 4 atomes de carbone, et n est un nombre entier de 2 à 4, et

au moins certaines des unités constitutives anioniques (2) sont une unité constitutive (2-1), (2-2) ou (2-4) représentée par la formule générale (II-a), (II-b) ou (II-d) :

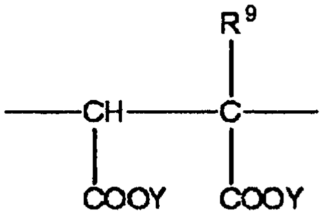

(II-a)

dans lequel $R^9$ est l'hydrogène ou un groupe méthyle, et les Y représentent chacun indépendamment l'hydrogène, Na, K, $NH_4$, 1/2Ca, 1/2Mg, 1/2Fe, 1/3Al, ou 1/3Fe pour chaque groupe carboxy lié à celui-ci,

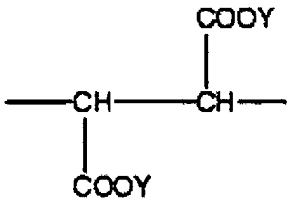

(II-b)

dans lequel Y représente l'hydrogène, Na, K, $NH_4$, 1/2Ca, 1/2Mg, 1/2Fe, 1/3Al, ou 1/3Fe indépendamment pour chaque groupe carboxy devant être lié, ou

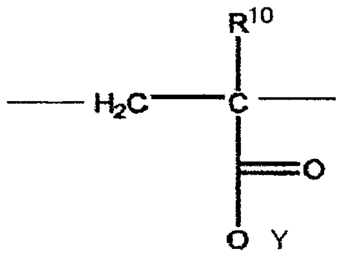

(II-d)

dans lequel $R^{10}$ est un hydrogène ou un groupe méthyle, et Y est l'hydrogène, Na, K, $NH_4$, 1/2Ca, 1/2Mg, 1/2Fe, 1/3Al, ou 1/3Fe indépendamment pour chaque groupe carboxy devant être lié
et
déterminer la présence de la mutation sur la base de la quantité totale de produits d'amplification d'acide nucléique obtenus par la réaction d'amplification d'acide nucléique, du nombre de cycles d'amplification de la réaction d'amplification d'acide nucléique requis pour que la quantité totale de produits d'amplification d'acide nucléique atteigne une valeur seuil, de la quantité d'acides nucléiques avec la mutation parmi les produits d'amplification d'acide nucléique, ou du nombre de cycles d'amplification de la réaction d'amplification d'acide nucléique requis pour que la quantité d'acides nucléiques avec la mutation parmi les produits d'amplification d'acide nucléique atteigne une valeur seuil.

**2.** Procédé selon la revendication 1, dans lequel

(i) l'unité constitutive cationique (1) est sélectionnée dans le groupe consistant en l'unité constitutive (1-1) et l'unité constitutive (1-4) ; ou
(ii) l'unité constitutive anionique (2) est sélectionnée dans le groupe consistant en l'unité constitutive (2-1) et l'unité constitutive (2-4) ; ou
(iii) le copolymère amphotère comprend en outre une unité constitutive non ionique (3) dérivée d'un monomère à base de méthacrylamide, ou d'un monomère à base d'acrylamide ; ou
(iv) dans le copolymère amphotère, au moins certaines des unités constitutives cationiques (1) sont des unités constitutives (1-1) présentant une structure représentée par la formule générale (I-a) ou la formule générale (I-b), ou une structure d'un sel d'addition d'acide de celles-ci :

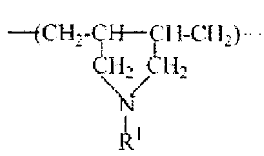

(I-a)

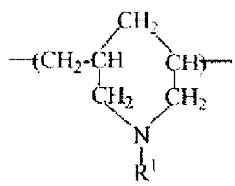

(I-b)

dans lequel $R^1$ représente un atome d'hydrogène, un groupe alkyle présentant 1 à 10 atomes de carbone pouvant présenter un groupe hydroxyle, un groupe cycloalkyle présentant 5 à 10 atomes de carbone, ou un groupe aralkyle présentant 7 à 10 atomes de carbone, et
au moins certaines des unités constitutives anioniques (2) sont des unités constitutives (2-1) chacune représentée par la formule générale (II-a) :

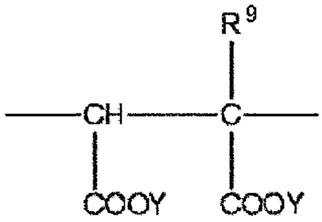

(II-a)

dans lequel $R^9$ est l'hydrogène ou un groupe méthyle, et les Y représentent chacun indépendamment l'hydrogène, Na, K, $NH_4$, 1/2Ca, 1/2Mg, 1/2Fe, 1/3Al, ou 1/3Fe pour chaque groupe carboxy lié à celui-ci.

**3.** Procédé selon la revendication 1, dans lequel

(i) le nucléotide artificiel est un BNA ; ou
(ii) l'amplification d'acide nucléique est effectuée par PCR en temps réel ; ou
(iii) l'échantillon d'acide nucléique est un ADN génomique.

**4.** Procédé selon l'une quelconque des revendications 1 à 3, dans lequel

un acide nucléique avec la séquence de bases standard est soumis en outre à la réaction d'amplification d'acide nucléique en tant que matrice, et
lorsque le nombre de cycles d'amplification de la réaction d'amplification d'acide nucléique requis pour que la quantité totale de produits d'amplification d'acide nucléique dans le cas de l'utilisation de l'acide nucléique dans l'échantillon comme matrice atteigne une valeur seuil, est inférieur au nombre de cycles d'amplification de la réaction d'amplification d'acide nucléique requis pour que la quantité totale de produits d'amplification d'acide nucléique dans le cas de l'utilisation de l'acide nucléique avec la séquence de bases standard comme matrice atteigne une valeur seuil, il est déterminé que la mutation est présente dans la séquence de bases objet de l'acide nucléique dans l'échantillon.

**5.** Kit pour :

(i) détecter une mutation dans une séquence de bases objet d'un acide nucléique dans un échantillon par rapport à une séquence de bases standard, comprenant un acide nucléique clamp présentant une séquence de bases complémentaire de la séquence de bases standard et contenant au moins un résidu nucléotidique artificiel ; ou
(ii) supprimer l'amplification d'un acide nucléique présentant une séquence de bases objet dans une réaction d'amplification d'acide nucléique, comprenant un acide nucléique clamp présentant une séquence de bases complémentaire de la séquence de bases objet et contenant au moins un résidu nucléotidique artificiel ;

dans lequel le kit comprend

un copolymère amphotère comprenant une unité constitutive cationique (1) avec un groupe amino dans sa structure et une unité constitutive anionique (2), dans lequel un rapport molaire de l'unité constitutive cationique (1) à l'unité constitutive anionique (2) (unité constitutive cationique (1)/unité constitutive anionique (2)) est compris dans la plage allant de 0,13 à 1,62, et dans lequel au moins certaines des unités constitutives cationiques (1) sont une unité constitutive (1-1), (1-3) ou (1-4) présentant une structure représentée par la formule générale (I-a), (I-b), (I-e) ou (I-f), ou une structure d'un sel d'addition d'acide de celle-ci :

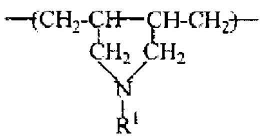

(I-a)

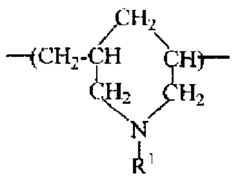

(I-b)

dans lequel $R^1$ représente un atome d'hydrogène, un groupe alkyle présentant 1 à 10 atomes de carbone pouvant présenter un groupe hydroxyle, un groupe cycloalkyle présentant 5 à 10 atomes de carbone, ou un groupe aralkyle présentant 7 à 10 atomes de carbone.

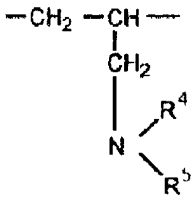

(1-e)

dans lequel $R^4$ et $R^5$ représentent indépendamment un atome d'hydrogène, un groupe alkyle présentant 1 à 12 atomes de carbone pouvant présenter un groupe hydroxyle, un groupe aralkyle présentant 7 à 12 atomes de carbone, ou un groupe cycloalkyle présentant 5 à 6 atomes de carbone, ou

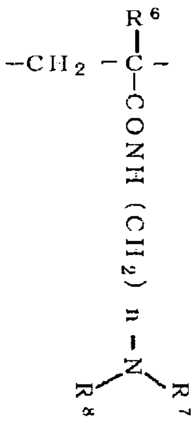

(1-f)

dans lequel $R^6$ représente un atome d'hydrogène ou un groupe méthyle, $R^7$ et $R^8$ représentent chacun indépendamment un atome d'hydrogène ou un groupe alkyle présentant 1 à 4 atomes de carbone, et n est un nombre entier de 2 à 4, et

au moins certaines des unités constitutives anioniques (2) sont une unité constitutive (2-1), (2-2) ou (2-4) représentée par la formule générale (II-a), (II-b) ou (II-d) :

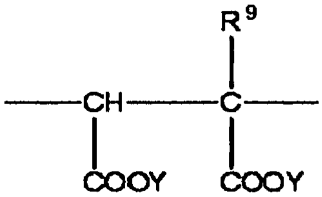

(II-a)

dans lequel $R^9$ est l'hydrogène ou un groupe méthyle, et les Y représentent chacun indépendamment l'hydrogène, Na, K, $NH_4$, 1/2Ca, 1/2Mg, 1/2Fe, 1/3Al, ou 1/3Fe pour chaque groupe carboxy lié à celui-ci

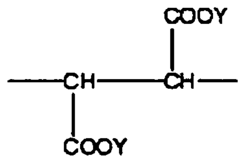

(II-b)

dans lequel Y représente l'hydrogène, Na, K, $NH_4$, 1/2Ca, 1/2Mg, 1/2Fe, 1/3Al, ou 1/3Fe indépendamment pour chaque groupe carboxy devant être lié, ou

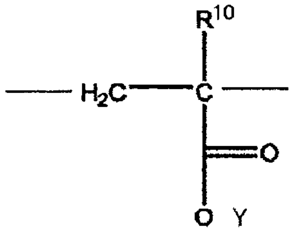

(II-d)

dans lequel $R^{10}$ est un hydrogène ou un groupe méthyle, et Y est l'hydrogène, Na, K, $NH_4$, 1/2Ca, 1/2Mg, 1/2Fe, 1/3Al, ou 1/3Fe indépendamment pour chaque groupe carboxy devant être lié.

**6.** Kit selon la revendication 5, dans lequel

(i) l'unité constitutive cationique (1) est sélectionnée dans le groupe consistant en l'unité constitutive (1-1) et l'unité constitutive (1-4) ; ou
(ii) l'unité constitutive anionique (2) est sélectionnée dans le groupe consistant en l'unité constitutive (2-1), et l'unité constitutive (2-4) ; ou
(iii) le copolymère amphotère comprend en outre une unité constitutive non ionique (3) dérivée d'un monomère à base de méthacrylamide, ou d'un monomère à base d'acrylamide ; ou
(iv) dans le copolymère amphotère, au moins certaines des unités constitutives cationiques (1) sont une unité constitutive (1-1) présentant une structure représentée par la formule générale (I-a) ou la formule générale (I-b), ou une structure d'un sel d'addition d'acide de celle-ci :

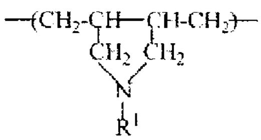

(I-a)

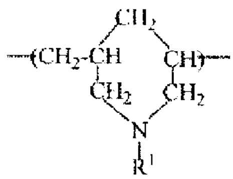

(I-b)

dans lequel $R^1$ représente un atome d'hydrogène, un groupe alkyle présentant 1 à 10 atomes de carbone pouvant présenter un groupe hydroxyle, un groupe cycloalkyle présentant 5 à 10 atomes de carbone, ou un groupe aralkyle présentant 7 à 10 atomes de carbone, et
au moins certaines des unités constitutives anioniques (2) sont des unités constitutives (2-1) chacune représentée par la formule générale (II-a) :

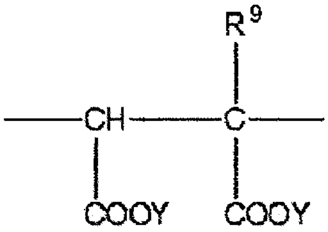

(II-a)

dans lequel $R^9$ est l'hydrogène ou un groupe méthyle, et les Y représentent chacun indépendamment l'hydrogène, Na, K, $NH_4$, 1/2Ca, 1/2Mg, 1/2Fe, 1/3Al, ou 1/3Fe pour chaque groupe carboxy lié à celui-ci.

7. Kit selon la revendication 5 ou la revendication 6, dans lequel le nucléotide artificiel est un BNA.

8. Procédé destiné à supprimer l'amplification d'un acide nucléique dans un échantillon présentant une séquence de bases objet prédéterminée dans une réaction d'amplification d'acide nucléique, comprenant :

soumettre l'acide nucléique dans l'échantillon à une réaction d'amplification d'acide nucléique en tant que matrice avec
un acide nucléique clamp présentant une séquence de bases complémentaire de la séquence de bases objet et contenant au moins un résidu d'acide nucléique artificiel, et
un copolymère amphotère comprenant une unité constitutive cationique (1) avec un groupe amino dans sa structure et une unité constitutive anionique (2), dans lequel un rapport molaire de l'unité constitutive cationique (1) à l'unité constitutive anionique (2) (unité constitutive cationique (1)/unité constitutive anionique (2)) est compris dans une plage allant de 0,13 à 1,62, et dans lequel au moins certaines des unités constitutives cationiques (1) sont une unité constitutive (1-1), (1-3) ou (1-4) présentant une structure représentée par la formule générale (I-a), (I-b), (I-e) ou (I-f), ou une structure d'un sel d'addition d'acide de celle-ci :

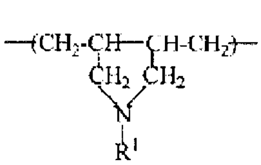

(I-a)

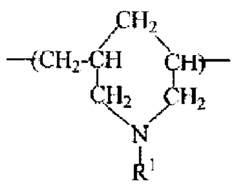

(I-b)

dans lequel $R^1$ représente un atome d'hydrogène, un groupe alkyle présentant 1 à 10 atomes de carbone pouvant présenter un groupe hydroxyle, un groupe cycloalkyle présentant 5 à 10 atomes de carbone, ou un groupe aralkyle présentant 7 à 10 atomes de carbone,

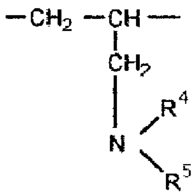

(1-e)

dans lequel $R^4$ et $R^5$ représentent indépendamment un atome d'hydrogène, un groupe alkyle présentant 1 à 12 atomes de carbone pouvant présenter un groupe hydroxyle, un groupe aralkyle présentant 7 à 12 atomes de carbone, ou un groupe cycloalkyle présentant 5 à 6 atomes de carbone, ou

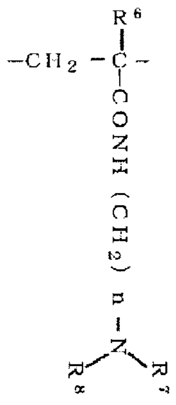

(1-f)

dans lequel $R^6$ représente un atome d'hydrogène ou un groupe méthyle, $R^7$ et $R^8$ représentent chacun indépendamment un atome d'hydrogène ou un groupe alkyle présentant 1 à 4 atomes de carbone, et n est un nombre entier de 2 à 4, et

au moins certaines des unités constitutives anioniques (2) sont une unité constitutive (2-1), (2-2) ou (2-4) représentée par la formule générale (II-a), (II-b) ou (II-d) :

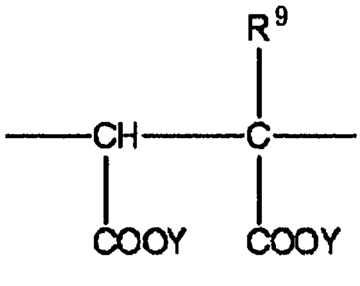

(II-a)

dans lequel $R^9$ est l'hydrogène ou un groupe méthyle, et les Y représentent chacun indépendamment l'hydrogène, Na, K, $NH_4$, 1/2Ca, 1/2Mg, 1/2Fe, 1/3Al, ou 1/3Fe pour chaque groupe carboxy lié à celui-ci,

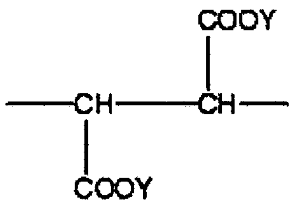

(II-b)

dans lequel Y représente l'hydrogène, Na, K, $NH_4$, 1/2Ca, 1/2Mg, 1/2Fe, 1/3Al, ou 1/3Fe indépendamment pour chaque groupe carboxy devant être lié, ou

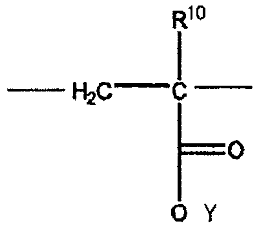

(II-d)

dans lequel $R^{10}$ est un hydrogène ou un groupe méthyle, et Y est l'hydrogène, Na, K, $NH_4$, 1/2Ca, 1/2Mg, 1/2Fe, 1/3Al, ou 1/3Fe indépendamment pour chaque groupe carboxy devant être lié.

**9.** Procédé selon la revendication 8, dans lequel

(i) l'unité constitutive cationique (1) est sélectionnée dans le groupe consistant en l'unité constitutive (1-1) et l'unité constitutive (1-4) ; ou
(ii) l'unité constitutive anionique (2) est sélectionnée dans le groupe consistant en l'unité constitutive (2-1) et l'unité constitutive (2-4) ; ou
(iii) le copolymère amphotère comprend en outre une unité constitutive non ionique (3) dérivée d'un monomère à base de méthacrylamide, ou d'un monomère à base d'acrylamide ; ou
(iv) dans le copolymère amphotère, au moins certaines des unités constitutives cationiques (1) sont des unités constitutives (1-1) présentant une structure représentée par la formule générale (I-a) ou la formule générale (I-b), ou une structure d'un sel d'addition d'acide de celles-ci :

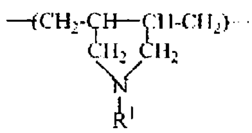

(I-a)

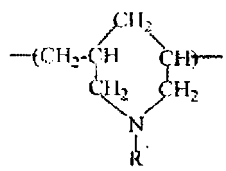

(I-b)

dans lequel $R^1$ représente un atome d'hydrogène, un groupe alkyle présentant 1 à 10 atomes de carbone pouvant présenter un groupe hydroxyle, un groupe cycloalkyle présentant 5 à 10 atomes de carbone, ou un groupe aralkyle présentant 7 à 10 atomes de carbone, et
au moins certaines des unités constitutives anioniques (2) sont une unité constitutive (2-1) représentée par la formule générale (II-a) :

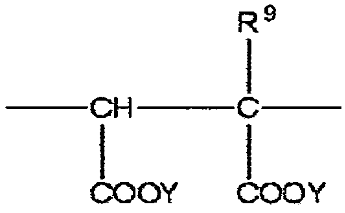

(II-a)

dans lequel $R^9$ est l'hydrogène ou un groupe méthyle, et les Y représentent chacun indépendamment l'hydrogène, Na, K, $NH_4$, 1/2Ca, 1/2Mg, 1/2Fe, 1/3Al, ou 1/3Fe pour chaque groupe carboxy lié à celui-ci;

**10.** Procédé selon la revendication 8 ou 9, dans lequel le nucléotide artificiel est un BNA.

**11.** Utilisation d'un agent pour renforcer un effet d'inhibition de l'amplification d'acide nucléique d'un acide nucléique présentant une séquence de bases objet par un acide nucléique clamp qui présente une séquence de bases complémentaire de la séquence de bases objet et contient au moins un résidu nucléotidique artificiel, l'agent

comprenant :

un copolymère amphotère comprenant une unité constitutive cationique (1) avec un groupe amino dans sa structure et une unité constitutive anionique (2), dans laquelle un rapport molaire de l'unité constitutive cationique (1) à l'unité constitutive anionique (2) (unité constitutive cationique (1)/unité constitutive anionique (2)) est dans la plage comprise de 0,13 à 1,62, et dans laquelle au moins certaines des unités constitutives cationiques (1) sont une unité constitutive (1-1), (1-3) ou (1-4) présentant une structure représentée par la formule générale (I-a), (I-b), (I-e) ou (I-f), ou une structure d'un sel d'addition d'acide de celle-ci :

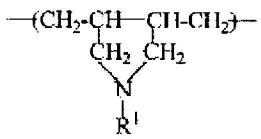

(I-a)

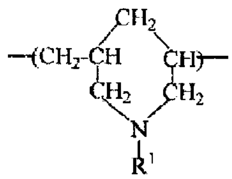

(I-b)

dans laquelle $R^1$ représente un atome d'hydrogène, un groupe alkyle présentant 1 à 10 atomes de carbone pouvant présenter un groupe hydroxyle, un groupe cycloalkyle présentant 5 à 10 atomes de carbone, ou un groupe aralkyle présentant 7 à 10 atomes de carbone,

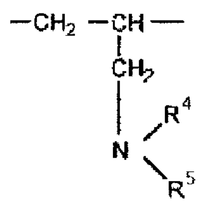

(1-e)

dans laquelle $R^4$ et $R^5$ représentent indépendamment un atome d'hydrogène, un groupe alkyle présentant 1 à 12 atomes de carbone pouvant présenter un groupe hydroxyle, un groupe aralkyle présentant 7 à 12 atomes de carbone, ou un groupe cycloalkyle présentant 5 à 6 atomes de carbone, ou

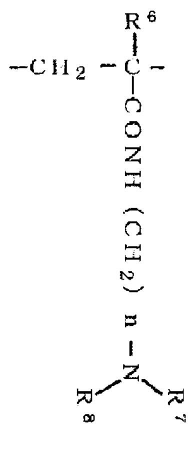

(1-f)

dans laquelle $R^6$ représente un atome d'hydrogène ou un groupe méthyle, $R^7$ et $R^8$ représentent chacun indépendamment un atome d'hydrogène ou un groupe alkyle présentant 1 à 4 atomes de carbone, et n est un nombre entier de 2 à 4, et

au moins certaines des unités constitutives anioniques (2) sont une unité constitutive (2-1), (2-2) ou (2-4) représentée par la formule générale (II-a), (II-b) ou (II-d) :

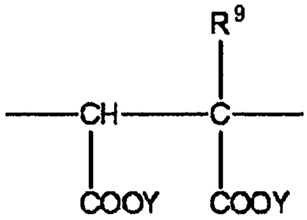

(II-a)

dans laquelle $R^9$ est l'hydrogène ou un groupe méthyle, et les Y représentent chacun indépendamment l'hydrogène, Na, K, $NH_4$, 1/2Ca, 1/2Mg, 1/2Fe, 1/3Al, ou 1/3Fe pour chaque groupe carboxy lié à celui-ci,

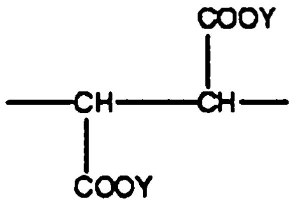

(II-b)

dans laquelle Y représente l'hydrogène, Na, K, $NH_4$, 1/2Ca, 1/2Mg, 1/2Fe, 1/3Al, ou 1/3Fe indépendamment pour chaque groupe carboxy devant être lié, ou

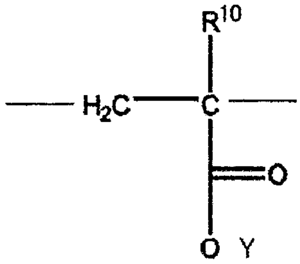

(II-d)

dans laquelle $R^{10}$ est un hydrogène ou un groupe méthyle, et Y est l'hydrogène, Na, K, $NH_4$, 1/2Ca, 1/2Mg, 1/2Fe, 1/3Al, ou 1/3Fe indépendamment pour chaque groupe carboxy devant être lié.

**12.** Utilisation selon la revendication 11, dans laquelle

(i) l'unité constitutive cationique (1) est sélectionnée dans le groupe consistant en l'unité constitutive (1-1) et l'unité constitutive (1-4) ; ou
(ii) l'unité constitutive anionique (2) est sélectionnée dans le groupe consistant en l'unité constitutive (2-1) et l'unité constitutive (2-4) ; ou
(iii) le copolymère amphotère comprend en outre une unité constitutive non ionique (3) dérivée d'un monomère à base de méthacrylamide, ou d'un monomère à base d'acrylamide ; ou
(iv) dans le copolymère amphotère, au moins certaines des unités constitutives cationiques (1)) sont une unité constitutive (1-1) présentant une structure représentée par la formule générale (I-a) ou la formule générale (I-b), ou une structure d'un sel d'addition d'acide de celle-ci :

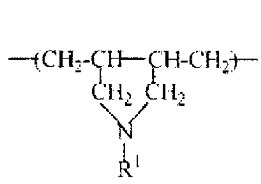

(I-a)

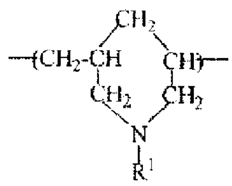

(I-b)

dans laquelle $R^1$ représente un atome d'hydrogène, un groupe alkyle présentant 1 à 10 atomes de carbone pouvant présenter un groupe hydroxyle, un groupe cycloalkyle présentant 5 à 10 atomes de carbone, ou un groupe aralkyle présentant 7 à 10 atomes de carbone, et

au moins certaines des unités constitutives anioniques (2) sont une unité constitutive (2-1) représentée par la formule générale (II-a) :

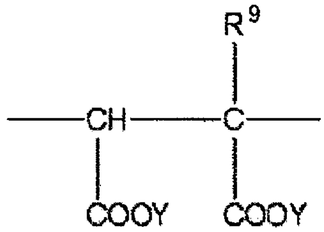

(II-a)

dans laquelle $R^9$ est l'hydrogène ou un groupe méthyle, et les Y représentent chacun indépendamment l'hydrogène, Na, K, $NH_4$, 1/2Ca, 1/2Mg, 1/2Fe, 1/3Al, ou 1/3Fe pour chaque groupe carboxy lié à celui-ci.

# FIG. 1

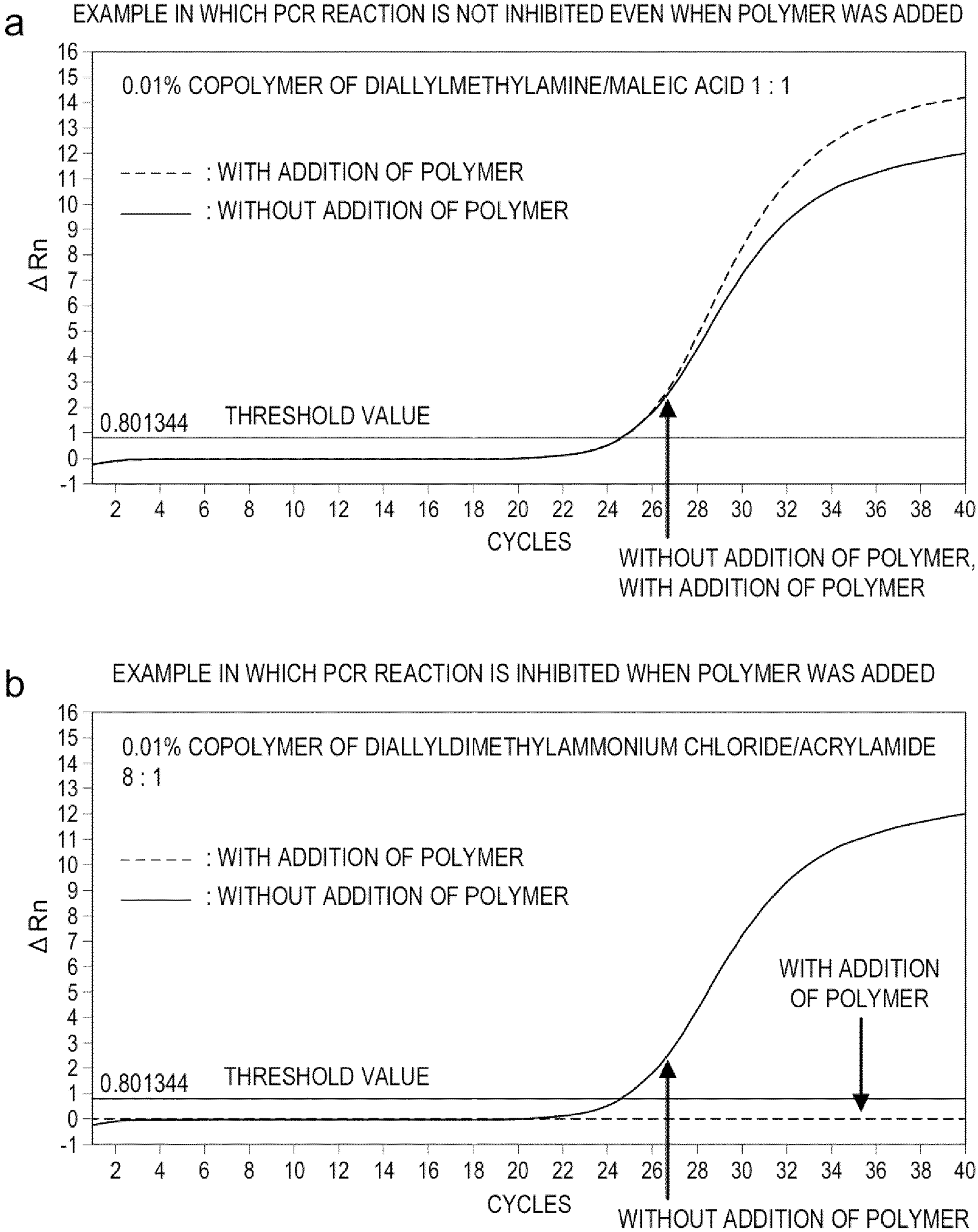
a
EXAMPLE IN WHICH PCR REACTION IS NOT INHIBITED EVEN WHEN POLYMER WAS ADDED
0.01% COPOLYMER OF DIALLYLMETHYLAMINE/MALEIC ACID 1 : 1
: WITH ADDITION OF POLYMER
: WITHOUT ADDITION OF POLYMER
Δ Rn
0.801344
THRESHOLD VALUE
CYCLES
WITHOUT ADDITION OF POLYMER,
WITH ADDITION OF POLYMER
b
EXAMPLE IN WHICH PCR REACTION IS INHIBITED WHEN POLYMER WAS ADDED
0.01% COPOLYMER OF DIALLYLDIMETHYLAMMONIUM CHLORIDE/ACRYLAMIDE
8 : 1
: WITH ADDITION OF POLYMER
: WITHOUT ADDITION OF POLYMER
Δ Rn
WITH ADDITION
OF POLYMER
0.801344
THRESHOLD VALUE
CYCLES
WITHOUT ADDITION OF POLYMER

# FIG. 2-1

0.1% COPOLYMER OF DIMETHYLAMINOPROPYL METHACRYLAMIDE HYDROCHLORIDE/ACRYLAMIDE/ACRYLIC ACID 1 : 1 : 2

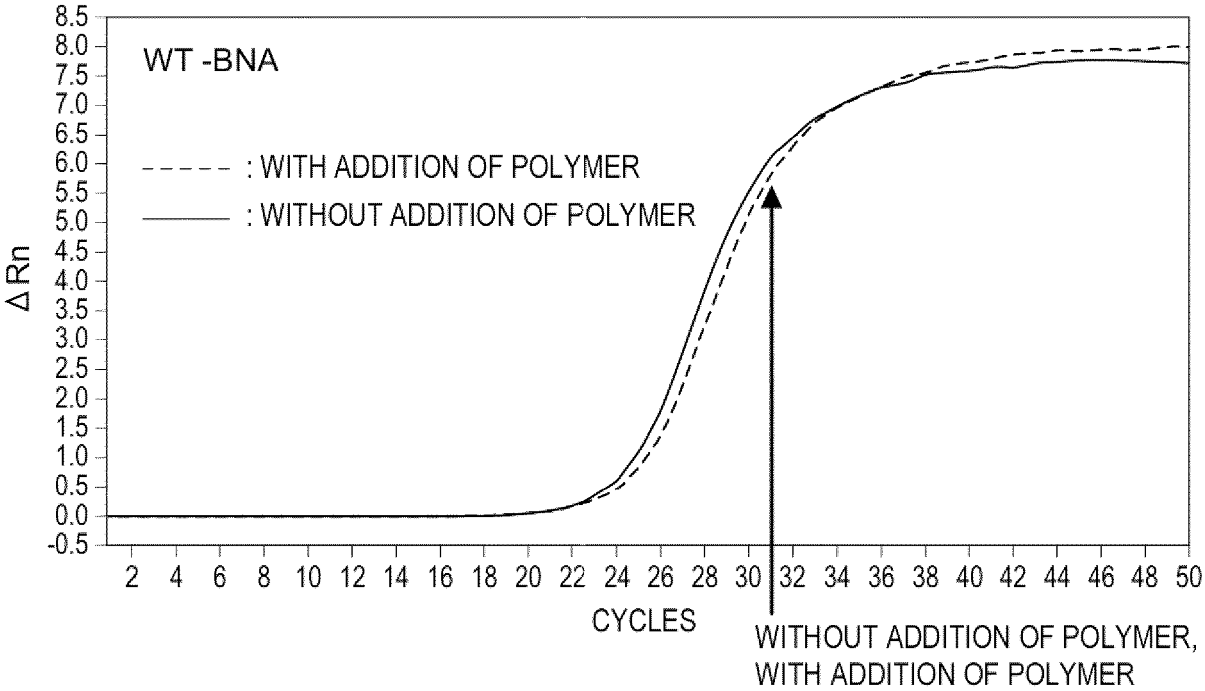


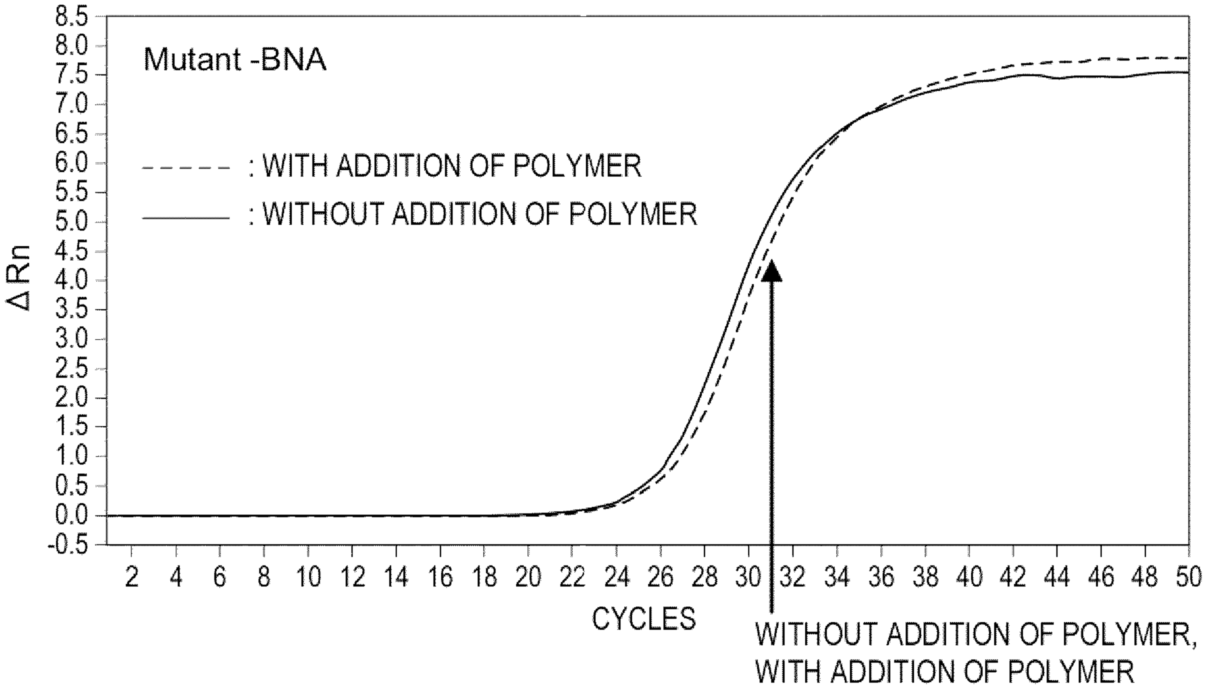

FIG. 2-2

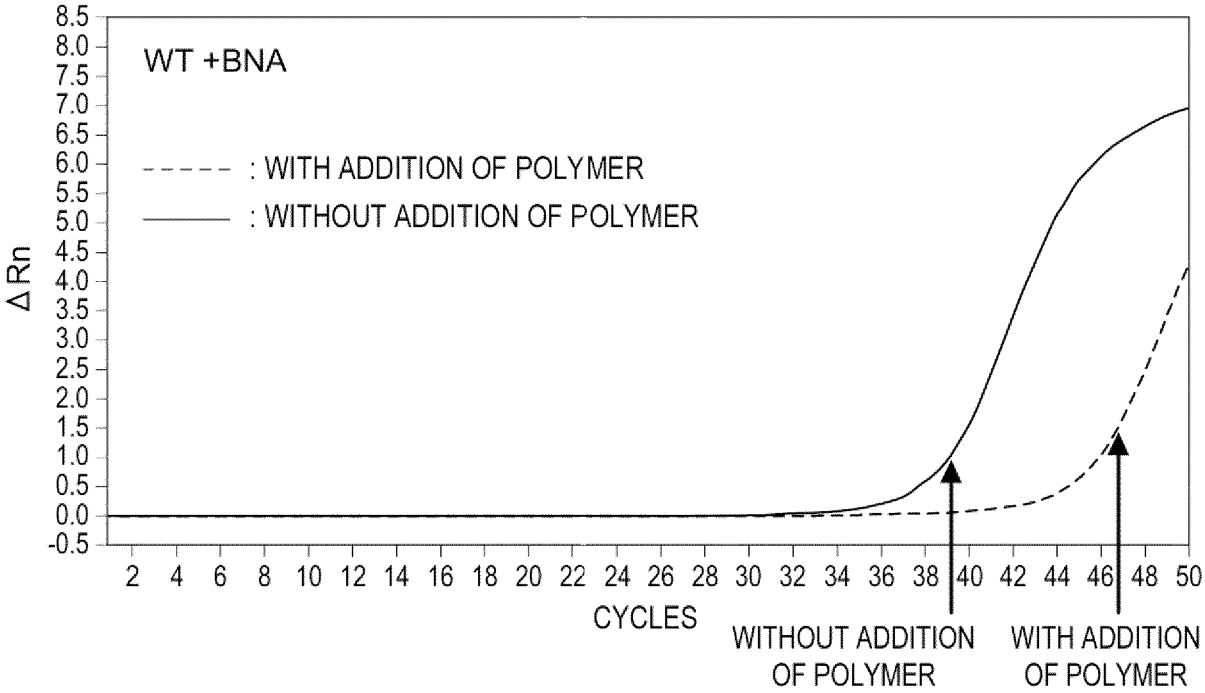
WT +BNA
- - - - - : WITH ADDITION OF POLYMER
——— : WITHOUT ADDITION OF POLYMER
Δ Rn
8.5
8.0
7.5
7.0
6.5
6.0
5.5
5.0
4.5
4.0
3.5
3.0
2.5
2.0
1.5
1.0
0.5
0.0
-0.5
2 4 6 8 10 12 14 16 18 20 22 24 26 28 30 32 34 36 38 40 42 44 46 48 50
CYCLES
WITHOUT ADDITION OF POLYMER
WITH ADDITION OF POLYMER

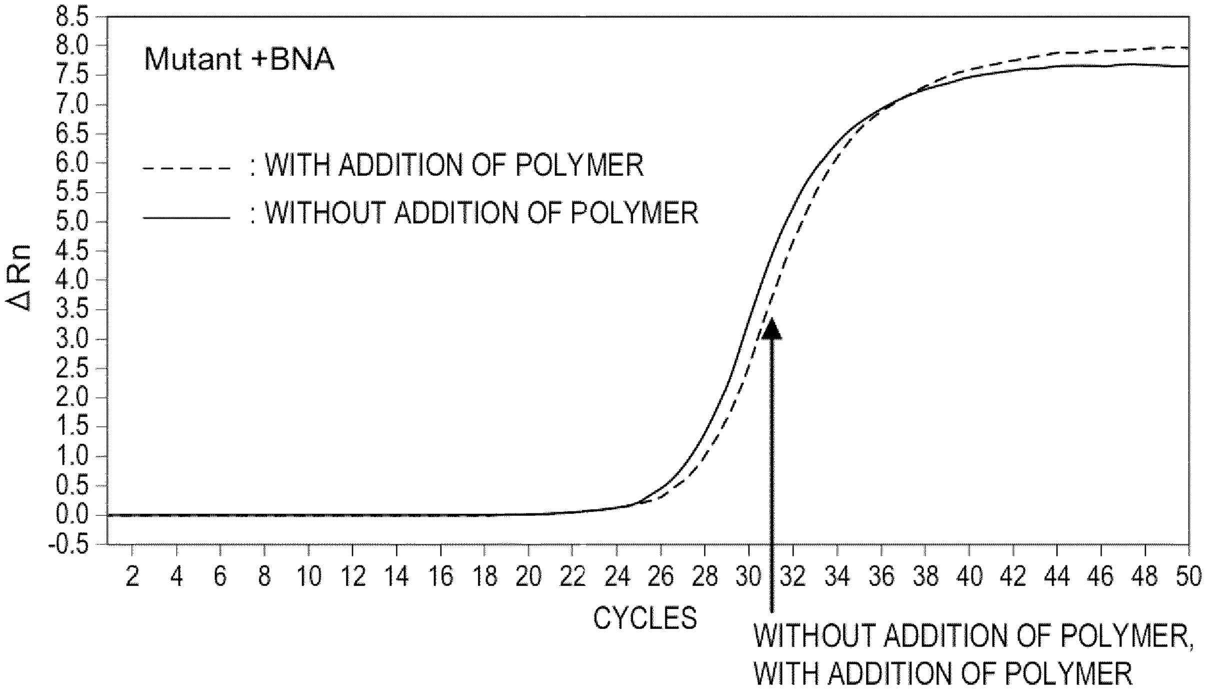
Mutant +BNA
- - - - - : WITH ADDITION OF POLYMER
——— : WITHOUT ADDITION OF POLYMER
Δ Rn
8.5
8.0
7.5
7.0
6.5
6.0
5.5
5.0
4.5
4.0
3.5
3.0
2.5
2.0
1.5
1.0
0.5
0.0
-0.5
2 4 6 8 10 12 14 16 18 20 22 24 26 28 30 32 34 36 38 40 42 44 46 48 50
CYCLES
WITHOUT ADDITION OF POLYMER, WITH ADDITION OF POLYMER

FIG. 3-1

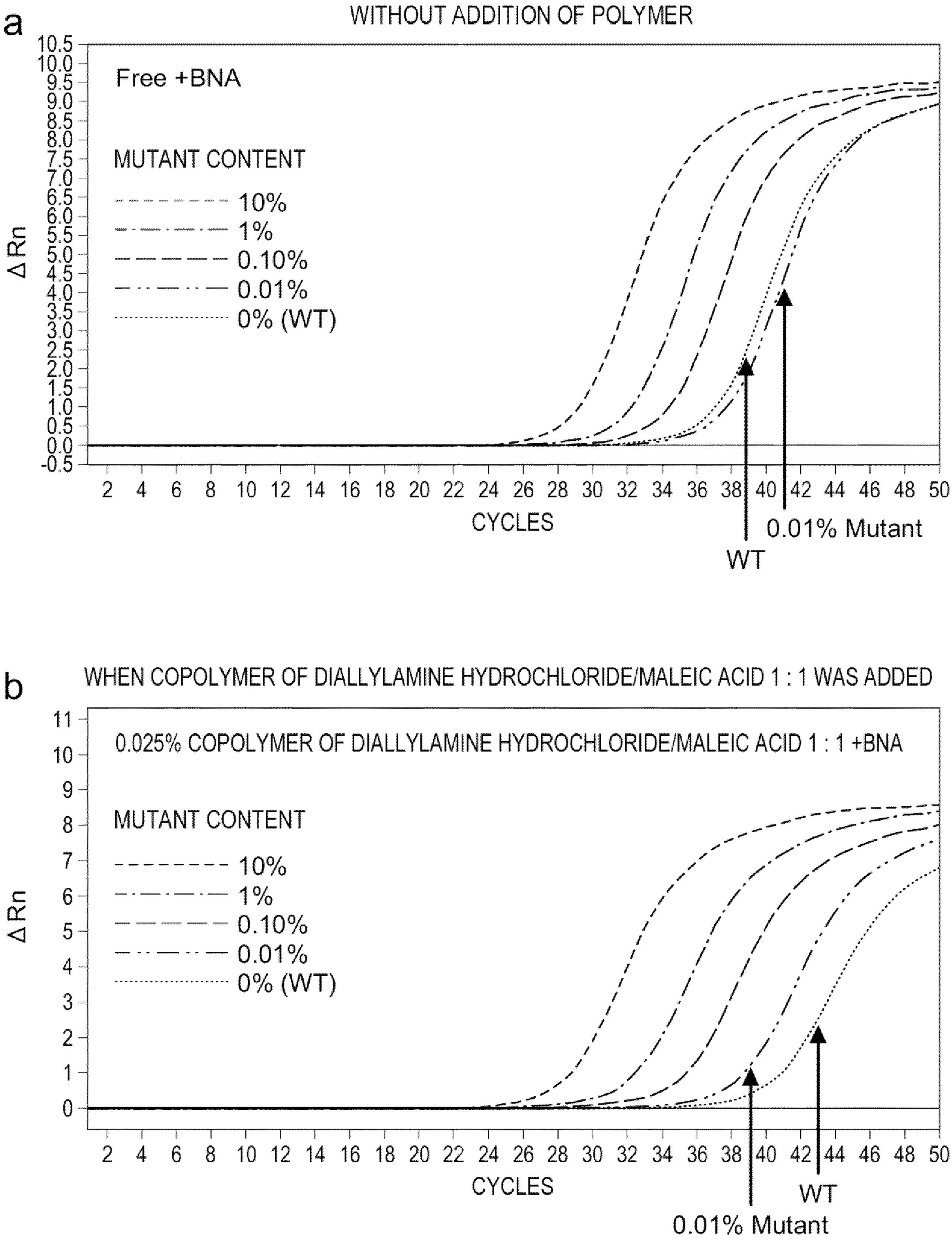
a
WITHOUT ADDITION OF POLYMER
Free +BNA
MUTANT CONTENT
10%
1%
0.10%
0.01%
0% (WT)
Δ Rn
CYCLES
WT
0.01% Mutant
b
WHEN COPOLYMER OF DIALLYLAMINE HYDROCHLORIDE/MALEIC ACID 1 : 1 WAS ADDED
0.025% COPOLYMER OF DIALLYLAMINE HYDROCHLORIDE/MALEIC ACID 1 : 1 +BNA
MUTANT CONTENT
10%
1%
0.10%
0.01%
0% (WT)
Δ Rn
CYCLES
WT
0.01% Mutant

# FIG. 3-2

c

WHEN COPOLYMER OF DIALLYLMETHYLAMINE/MALEIC ACID 1 : 1 WAS ADDED

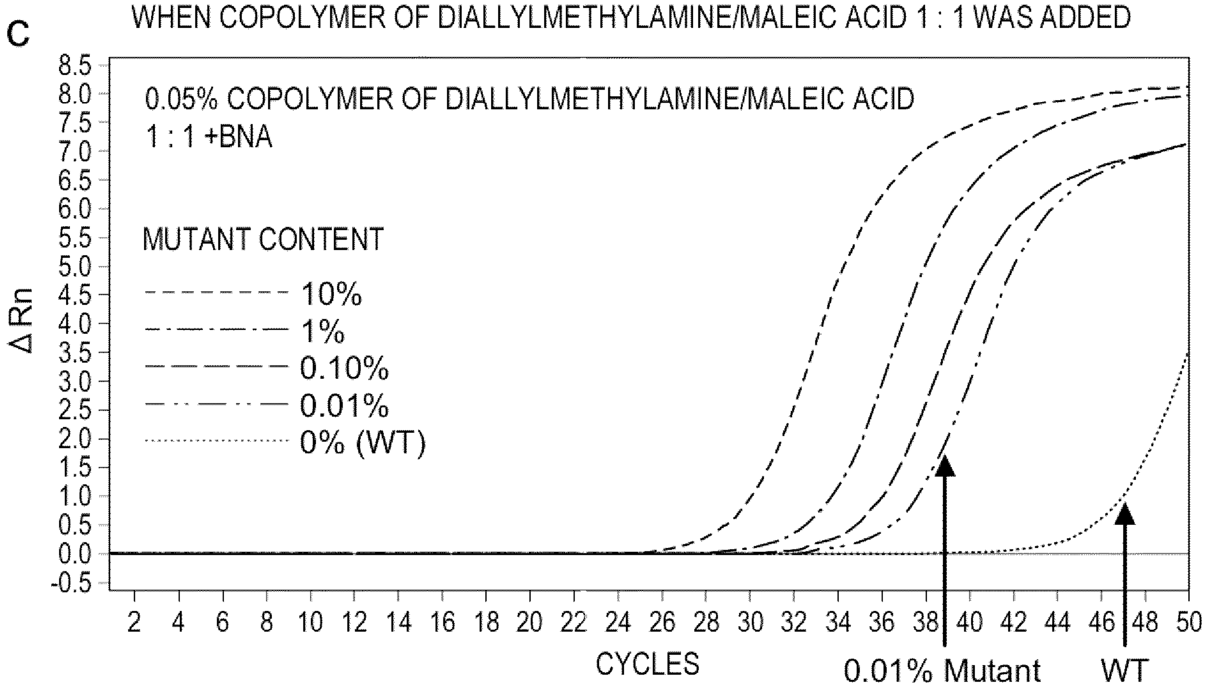


d

WHEN COPOLYMER OF DIMETHYLAMINOPROPYL METHACRYLAMIDE HYDROCHLORIDE/ACRYLAMIDE/ACRYLIC ACID 1 : 1 : 2 WAS ADDED

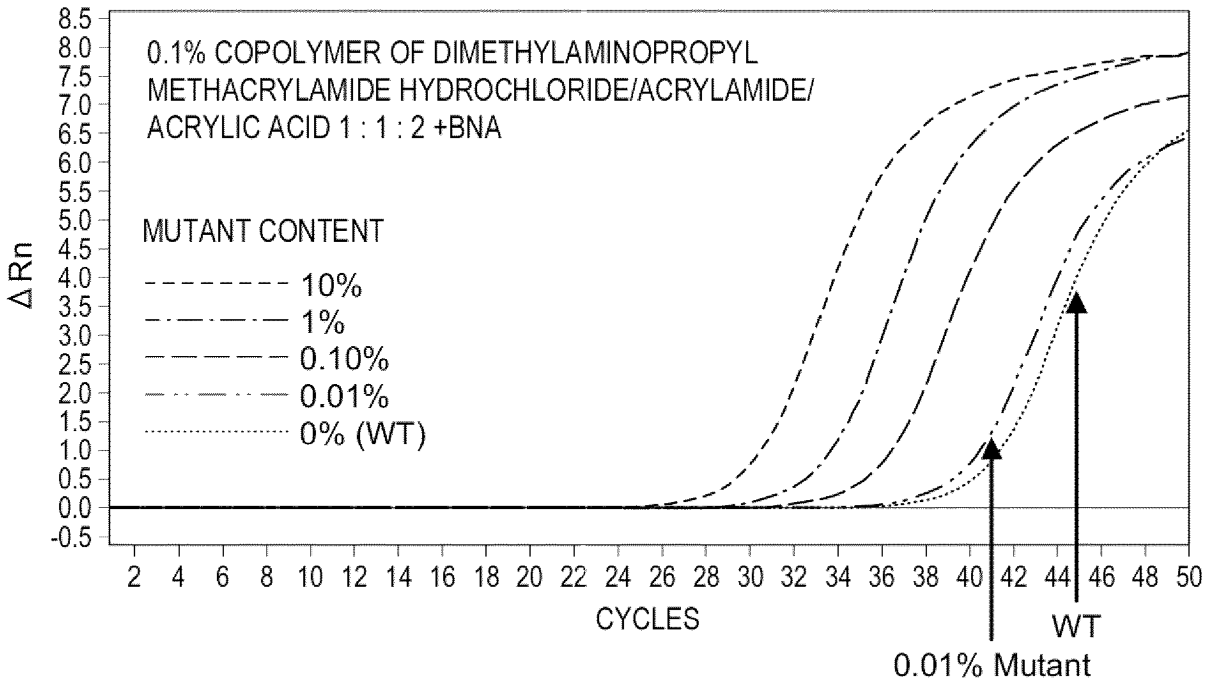

# FIG. 3-3

e

WHEN COPOLYMER OF DIALLYLAMINE HYDROCHLORIDE/ACRYLAMIDE/ ACRYLIC ACID 1 : 1 : 2 WAS ADDED

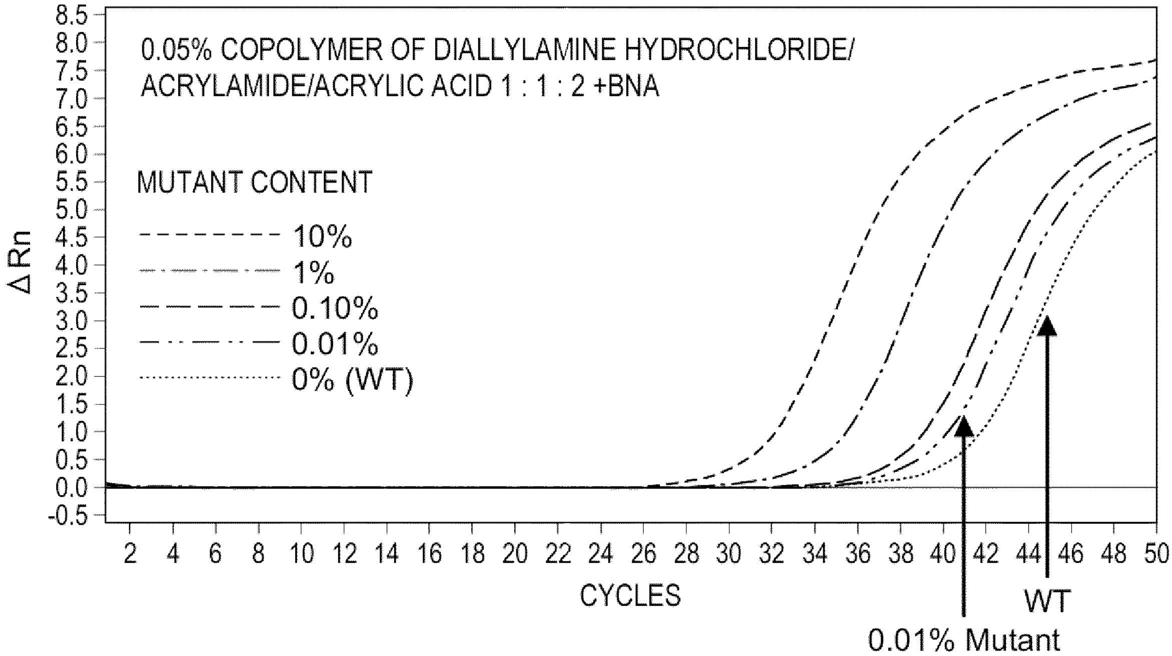


# FIG. 4

<PLASMID CONSTRUCT>

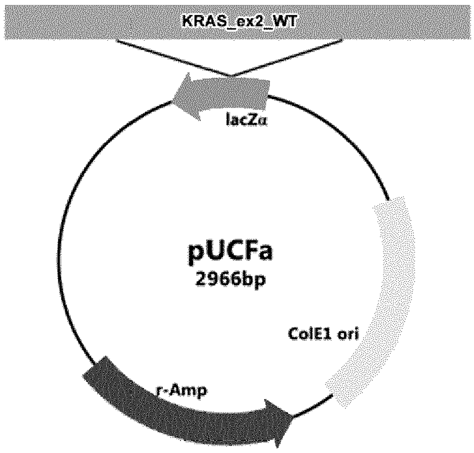


INSERTED SEQUENCE: 212 bp (KRAS WT/Mutant SEQUENCE + Hind III RECOGNITION SEQUENCE
FULL LENGTH : 3178 bp

1. FRAGMENTATION WITH RESTRICTIVE ENZYME Hind III
2. DEPHOSPHATASE REACTION OF BOTH ENDS *IN ORDER TO PREVENT SELF-LIGATION
3. DNA PURIFICATION (USING Promega Wizard SV PCR and Gel Clean-up System)

PRODUCTION OF plasmid DNA

FIG. 5-1

a

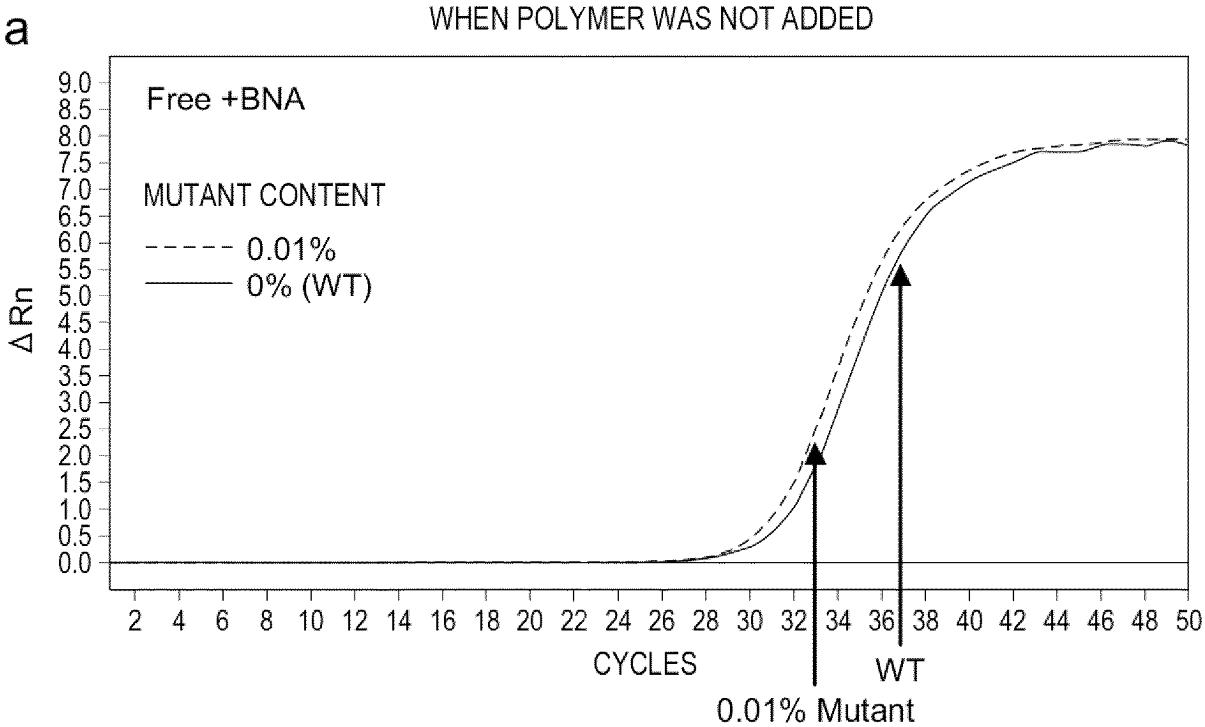
WHEN POLYMER WAS NOT ADDED
Free +BNA
MUTANT CONTENT
0.01%
0% (WT)
Δ Rn
9.0 8.5 8.0 7.5 7.0 6.5 6.0 5.5 5.0 4.5 4.0 3.5 3.0 2.5 2.0 1.5 1.0 0.5 0.0
2 4 6 8 10 12 14 16 18 20 22 24 26 28 30 32 34 36 38 40 42 44 46 48 50
CYCLES
WT
0.01% Mutant

b

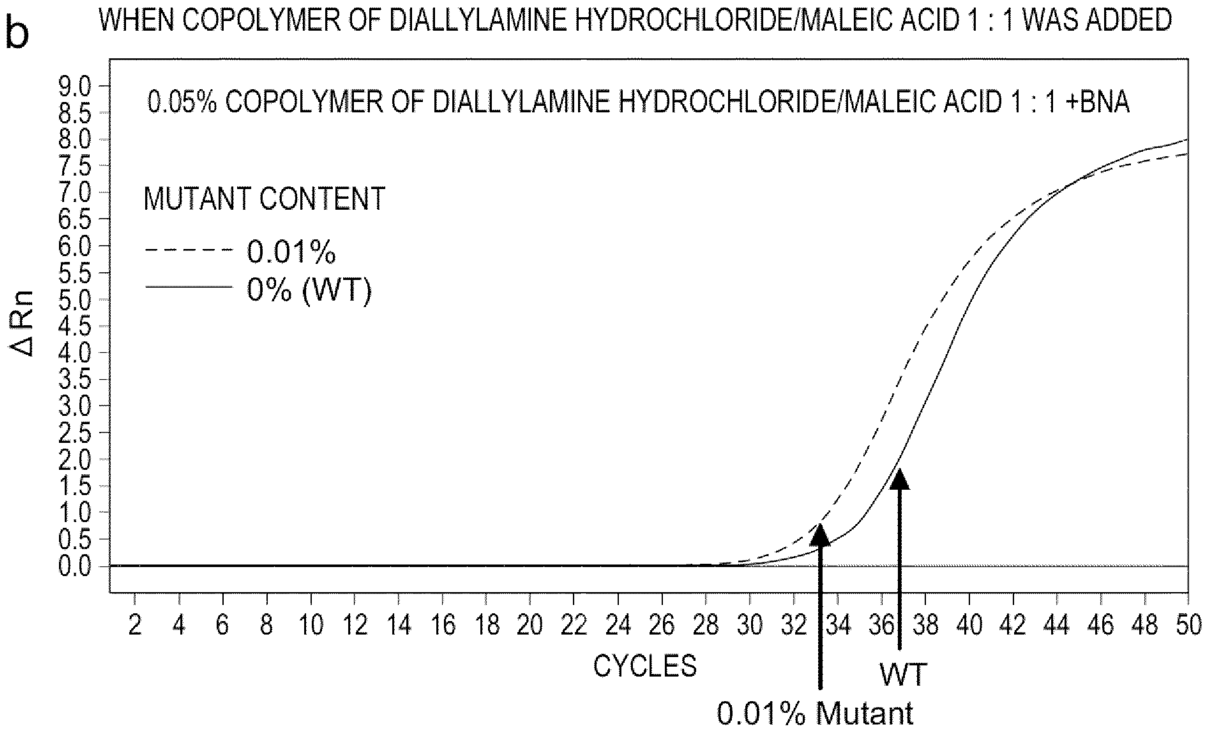
WHEN COPOLYMER OF DIALLYLAMINE HYDROCHLORIDE/MALEIC ACID 1 : 1 WAS ADDED
0.05% COPOLYMER OF DIALLYLAMINE HYDROCHLORIDE/MALEIC ACID 1 : 1 +BNA
MUTANT CONTENT
0.01%
0% (WT)
Δ Rn
9.0 8.5 8.0 7.5 7.0 6.5 6.0 5.5 5.0 4.5 4.0 3.5 3.0 2.5 2.0 1.5 1.0 0.5 0.0
2 4 6 8 10 12 14 16 18 20 22 24 26 28 30 32 34 36 38 40 42 44 46 48 50
CYCLES
WT
0.01% Mutant

FIG. 5-2

c

WHEN COPOLYMER OF DIALLYLMETHYLAMINE/MALEIC ACID 1 : 1 WAS ADDED

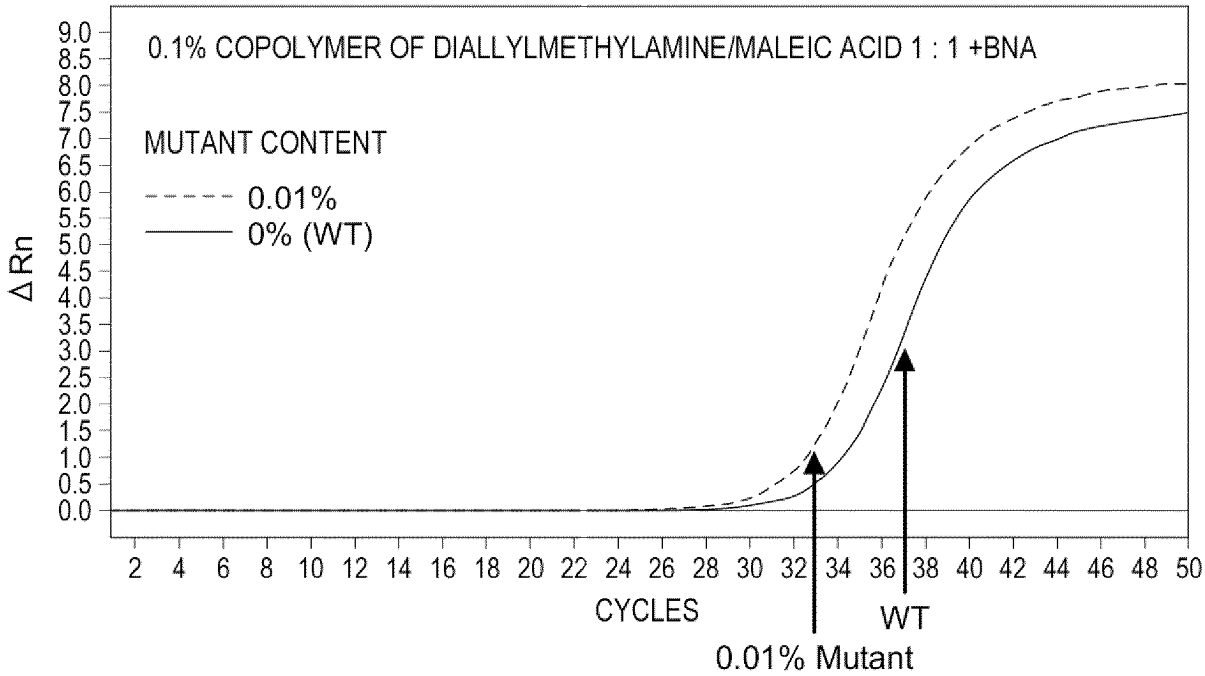


d

WHEN COPOLYMER OF DIMETHYLAMINOPROPYL METHACRYLAMIDE HYDROCHLORIDE/ACRYLAMIDE/ACRYLIC ACID 1 : 1 : 2 WAS ADDED

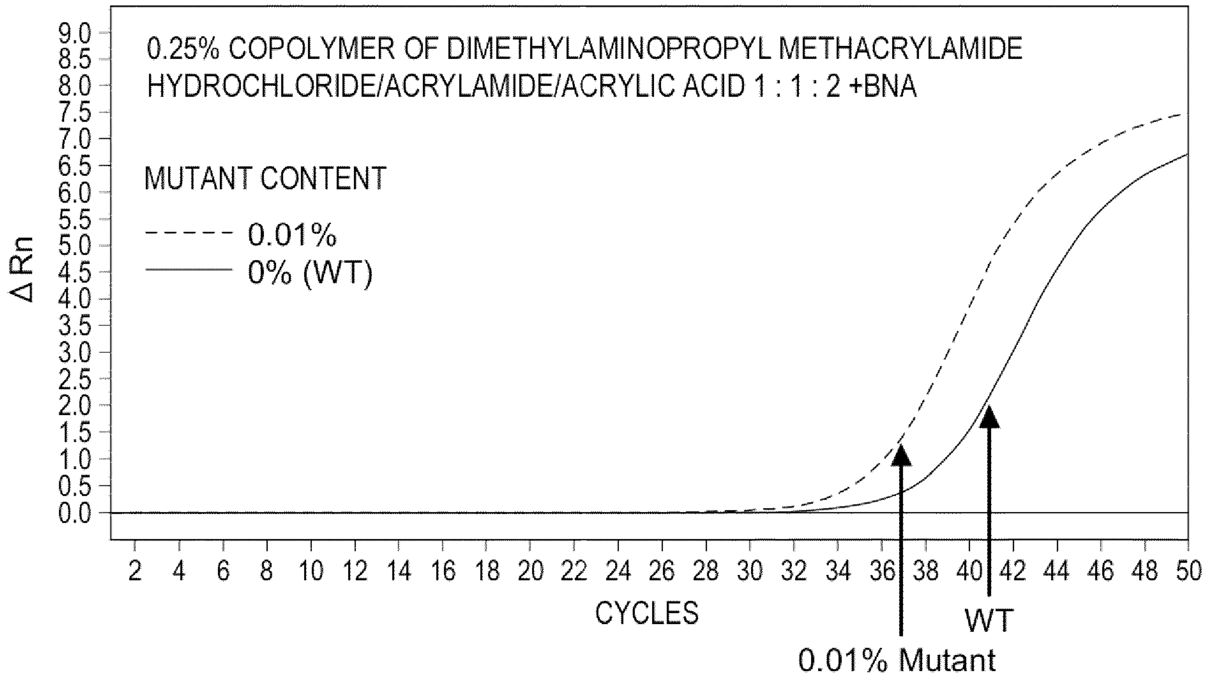

# FIG. 5-3

e WHEN COPOLYMER OF DIALLYLAMINE HYDROCHLORIDE/ACRYLAMIDE/ACRYLIC ACID 1 : 1 : 2 WAS ADDED

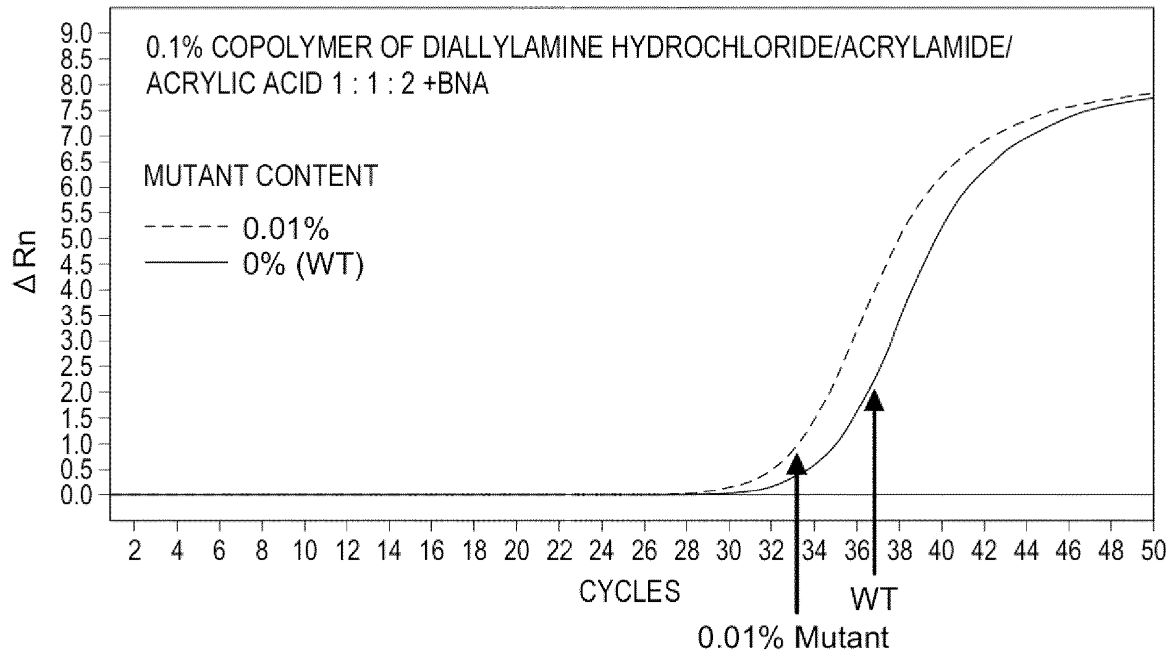


# FIG. 6

SEQUENCING RESULTS AT Mutant RATIO OF 0.01%

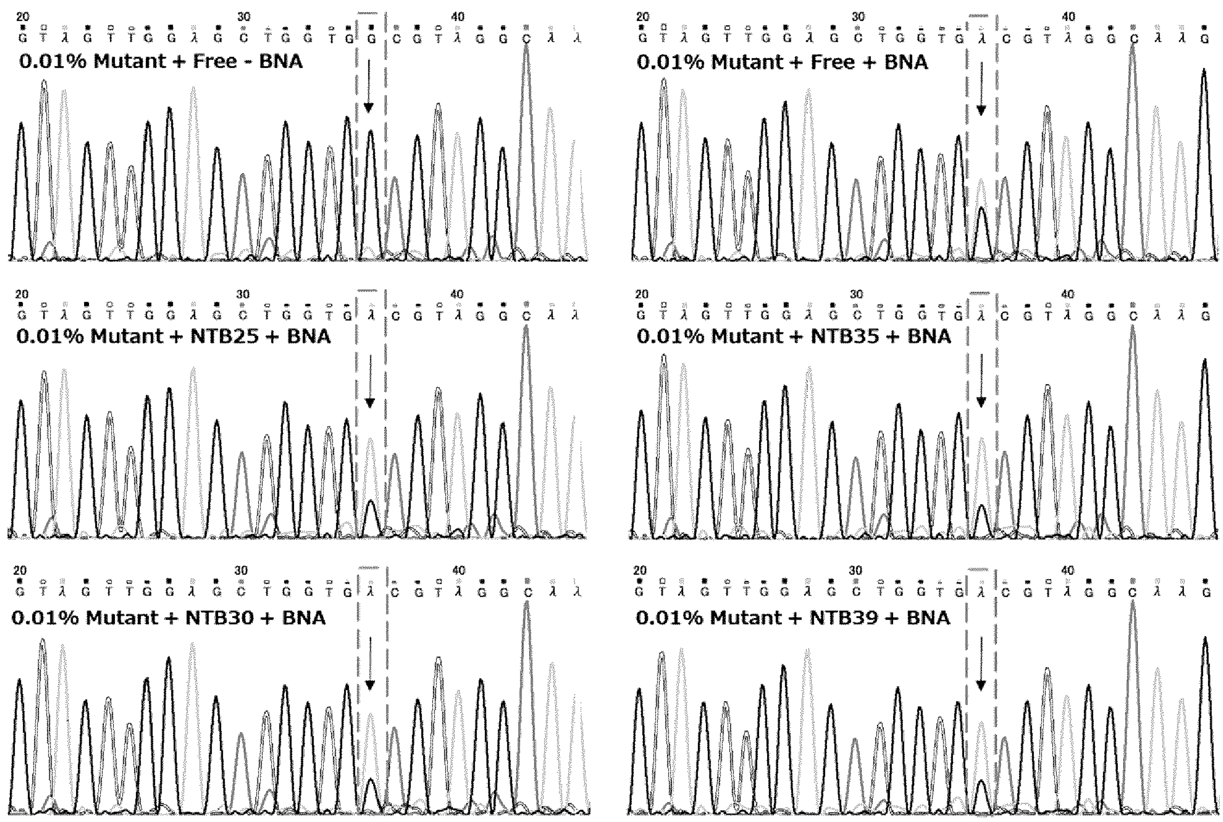

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6242336 B
- JP 5813263 B
- JP 4731324 B
- JP 4383178 B
- JP 5030998 B
- JP 4151751 B
- JP 2001089496 A
- WO 2003068795 A
- WO 2005021570 A
- JP 3756313 B
- WO 2016167320 A
- WO 2014051076 A


### Non-patent literature cited in the description


- **THOMAS J**. *The NEW ENGLAND JURNAL of MEDICINE*, 2004, vol. 350, 21
- **NAGAI K**. *Cancer Research*, 2005, vol. 65, 7276-7282
- **NISHINO K. et al.** *Guide for EGFR gene mutation testing in lung cancer patients*, 2019
- **LUMING Z. et al.** *BioTechniques*, 2011, vol. 50, 311-318
- **NAGAKUBO Y. et al.** *BMC Medical Genomics*, 2019, vol. 12, 162
- **MURINA F.F. et al.** *Molecules*, 2020, vol. 25 (4), 786